# EUROPEAN PATENT SPECIFICATION

(11) **EP 1 761 634 B1**
(45) Date of publication and mention of the grant of the patent: **30.12.2009**
(21) Application number: 05789221.8
(22) Date of filing: 16.06.2005
(51) Int. Cl.: C12N 15/82

(54) **CELL NUMBER POLYNUCLEOTIDES AND POLYPEPTIDES AND METHODS OF USE THEREOF**
POLYNUCLEOTIDE UND POLYPEPTIDE, DIE FÜR DIE ZELLANZAHL VERANTWORTLICH SIND UND METHODEN ZU DEREN VERWENDUNG
POLYNUCLEOTIDES ET POLYPEPTIDES REGULANT LE NOMBRE DE CELLULES ET LEURS METHODES D'UTILISATION

(30) Priority: 28.06.2004 US 583340 P
(43) Date of publication of application: 14.03.2007
(73) Proprietor: Pioneer-Hi-Bred International, Inc., Johnston, IA 50131-1014 (US)
(72) Inventor: GUO, Mei, West Des Moines, Iowa 50265 (US); SIMMONS, Carl R., Des Moines, Iowa 50310 (US); HERSHEY, Howard P., Cumming, Iowa 50061 (US)
(74) Representative: Bentham, Andrew
(86) International application number: PCT/US2005/021232
(87) International publication number: WO 2006/012024

(56) References cited:
- WO-A-03/008540
- US-A1- 2003 024 013
- US-A1- 2003 135 888
- US-A1- 2004 034 888
- DATABASE UniProt [Online] 5 July 2004 (2004-07-05), "Putative PGPS/D12." XP002373725 retrieved from EBI accession no. UNIPROT:Q6Z6F9 Database accession no. Q6Z6F9
- DATABASE UniProt [Online] 16 August 2004 (2004-08-16), "Putative ORFX." XP002387907 retrieved from EBI accession no. UNIPROT:Q6EP48 Database accession no. Q6EP48
- DATABASE UniProt [Online] 16 August 2004 (2004-08-16), "Putative ORFX." XP002387908 retrieved from EBI accession no. UNIPROT:Q6EP46 Database accession no. Q6EP46
- YAN LIULING ET AL: "The wheat VRN2 gene is a flowering repressor down-regulated by vernalization." SCIENCE (WASHINGTON D C), vol. 303, no. 5664, 12 March 2004 (2004-03-12), pages 1640-1644, XP002384646 ISSN: 0036-8075 & DATABASE UniProt [Online] 5 July 2004 (2004-07-05), "Hypothetical protein. PWTTGIFGCT DDPESCRTGL FCPCVLFGRN VEALREDIPW TTPCVCHAIF VEGGITLAIL" retrieved from EBI accession no. UNIPROT:Q6RYF1 Database accession no. Q6RYF1 & DATABASE UniProt [Online] 5 July 2004 (2004-07-05), "Hypothetical protein. PWSTGIFGCT DDPESCRTGL FCPCVLFGRN VEALREDIPW TTPCVCHAIF VEGGITLAIL" retrieved from EBI accession no. UNIPROT:Q6PX45 Database accession no. Q6PX45
- DATABASE UniProt [Online] 1 October 2003 (2003-10-01), "Expressed protein." XP002387909 retrieved from EBI accession no. UNIPROT:Q7XC69 Database accession no. Q7XC69
- DATABASE UniProt [Online] 1 October 2002 (2002-10-01), "Hypothetical protein At2g45010 (Hypothetical protein) (At2g45010 protein). NWTTGIFGCA EDPESCRTGL FCPCVLFGRN IEAVREEIPW TQPCVCHAVC VEGGMALAAV" XP002387910 retrieved from EBI accession no. UNIPROT:Q8L3T0 Database accession no. Q8L3T0
- DATABASE UniProt [Online] 1 June 2003 (2003-06-01), "Hypothetical protein OSJNBb0027B08.16 (Hypothetical protein OSJNBa0078D06.11)." XP002387911 retrieved from EBI accession no. UNIPROT:Q850Y9 Database accession no. Q850Y9

## Description

### FIELD OF THE INVENTION

The invention relates generally to the field of molecular biology.

### BACKGROUND OF THE INVENTION

The domestication of many plants has correlated with dramatic increases in yield. Most phenotypic variation occurring in natural populations is continuous and is effected by multiple gene influences. The identification of specific genes responsible for the dramatic differences in yield, in domesticated plants, has become an important focus of agricultural research.

One group of genes effecting yield are the cell number regulator genes, or CNR genes. One quantitative trait locus (QTL), *fw2.2*, containing the specific gene family, was found to be associated with decreases of up to 30% in fruit size in tomatoes. The suggestion is that *fw2.2* may be a negative regulator of cell division. (Frary, et al., fw2.2: A Quantitative Trait Locus Key to the Evolution of Tomato Fruit Size, Science, Vol. 289, 85-88, 2000) The plants producing the fruit did not differ significantly in the total fruit weight, or harvest index (fruit yield divided by plant weight). Alterations in the fruit size, as imparted by the *fw2.2* alleles appeared to be due to changes in regulation rather than in the sequence and structure of the encoded protein. The cause of the QTL effect in the tomato plants was a single gene controlling carpel cell number that is expressed early in the floral development Further research indicated that the primary effect of *fw2.2* is in controlling ovary and fruit size, and that other associated phenotypic effects are secondary. (Nesbitt, et al., fw2.2 Directly Affects the Size of Developing Tomato Fruit, with Secondary Effects on Fruit Number and Photosynthate Distribution, Plant Physiology, Vol. 127, 575-583, 2001). The *fw2.2* genes were found to change plant morphology by modifying sink-source relationships at the whole plant level, causing alteration of inflorescence number; fruit number, and fruit and flower abortion rates. The genes have the potential to alter the competition for photosynthate among fruit, thereby having a significant impact on the size of fruit at maturity.

Heterosis is an important mechanism whereby many crop plants are enhanced in yield and performance. It is characterized by an increase in plant growth and vigor that gives rise to enhanced yield. This heterotic gain usually occurs in hybrid offspring of select parent lines that may otherwise be middling in yield performance. These parent lines are often inbred and relatively genetically homogenous lines. While heterosis is used in several crop plant production systems, its application to maize is most widely known. It is used in the majority of maize production systems in the developed world. It is the underpinning to the hybrid seed corn industry.

The full mechanism of heterosis is still being investigated. While not intending to be bound by any one theory, proposed heterosis mechanisms are listed here. There is a hypothesis which envisions a complementation of multiple gene alleles, whether by protein function, by gene expression or otherwise. However other non-exclusive theories envision a smaller set of key genes, especially those whose alteration in function might result in changes in plant vigor.

### SUMMARY OF THE INVENTION

Genes that regulate cell number in plants have the potential to be involved in heterosis. Cell-number regulating genes could be judiciously manipulated to achieve a heterotic phenotype. Heterotic plants are usually more robust and of larger stature. Few studies have investigated the source of this greater size. One key observation made by maize biologist T. A. Kiesselbach in 1922 revealed that in maize heterosis is primarily due to increases in *cell number* not cell size. (See T.A Kiesselbach (1922). Corn Investigations. Bulletin of the Agricultural Experiment Station of Nebraska. Research Bulletin No. 20. The University of Nebraska. Lincoln, Nebraska U.S.A. Histological Effects of Inbreeding, pages 96-102.) Specifically Klesselbach states, "These data suggest that 10.6 per cent of the increased size due to crossing results from an increase in cell size and 89.4 per cent of it from increased numbers".

The tomato gene Fw2.2 can be defined as a negative regulator of cell number in tomato carpels. When the Fw2.2 gene expression is diminished, larger tomato fruit are produced. The fruit have been analyzed microscopically and show the larger fruit to be due to increases in cell number not cell size. It is consistent with the effect of heterosis, thus maize orthologues to the tomato gene Fw2.2 are desirable.

The present invention is have identified maize genes encoding proteins related to the gene family containing tomato Fw2.2 gene. Methods relating to the ZmCNR1 gene could be used to produce the heterotic phenotype. Such a phenotype would exhibit diminishment of the negative regulation of cell number leading to a plant, or part(s) of a plant, that are larger (larger cell number), and more vigorous, ultimately leading to enhanced crop plant yield.

Methods relating to the CNR (cell number regulator) gene ZmCNR01, may be useful for enhancing crop yield.

The CNR genes are negative regulators of cell number, and their decreased expression causes increased cell number, enhanced vigor, and heterotic phenotype. If the genes were to be downregulated (by knockout, mutation, homologous recombination, antisense, microRNAs, or otherwise), then increased cell numbers would result. Provided that this downregulation does not deleteriously affect normal balance in development, a larger-sized, more vigorous plant may result. The downregulation may be partial or complete, increasing cell number without imbalance in development, or the knockout or diminished expression in particular tissues (such as endosperm) could result in larger organs, by virtue of extending their period of cell division. These approaches could be used in addition to conventional heterosis, giving an added yield enhancement.

The CNR01 gene shows expression in diverse tissues, and may control cell number throughout the plant. General heterosis tends to make the whole plant more vigorous, not simply one or a set of tissues. Accordingly, the above strategies requiring promoters to diminish the gene expression may be tried using promoters with a broad developmental expression pattern. These may be among the so-called constitutive promoters, one example being the maize ubiquitin promoter. Promoters could also be used to focus expression in one or several tissues (if desired enhanced cell number is deemed to be sufficient within a limited spectrum of the plant development). Such enhanced tissues of interest include for example, roots (enhance root development by diminishing gene or gene expression there), embryos (larger embryos, including effecting higher oil content of the whole seed), seedling (seedling vigor, enhanced mesocotyl size and extension to emerge more successfully from the soil), silks (enhanced silk emergence, including during droughted conditions, or to nick or synchronize with pollen donation), stalks (to increase the girth leading to greater stalk strength), and other plant tissues of interest.

CNR gene function has a relationship to auxin and cadmium in growing tissue. While not being bound by any particular theory, a possible molecular/physiological mode of action of the CNR-type genes affecting plant cell number is presented. This hypothesis is not represented in the public literature and reconciles information that would otherwise appear confusing or contradictory.

The connections between CNR genes, growth, auxin action and cadmium, lead to the following theorized mode of action.

The CNR genes affect cell number through auxin action, a well known plant hormone that affects plant cell growth, in some instances through cell number. Cadmium in a general metal binding capacity, also has a role in auxin action, where a substitution by cadmium occurs where there would otherwise be a zinc ion that is involved in auxin binding or action. The CNR gene expression may increase as tissues mature in order to bind to auxin and check the continued growth of the tissue. In this way the CNR genes may bind auxin to help to stop its growth-promoting action.

One reason that CNR genes have not been found to be auxin binding proteins to date may be in part because they are membrane bound (and thus less likely to be isolated). Another reason may be because their expression may be higher in maturing tissues, an area where modes of auxin action have been investigated to a lesser degree. Plants over-expressing CNR genes may be auxin resistant and/or cadmium resistant. The auxin resistance and cadmium resistance may be in conflict as cadmium replaces zinc and undermines the auxin-binding activity. The auxin resistance could mean resistance to auxin-related herbicides. The cadmium resistance may be efficacious in certain settings for resistance of plants to toxic metals. Reductions in CNR expression or activity would result in auxin sensitivity and/or cadmium sensitivity. The former may result in increased sensitivity to auxin-related growth, which is the mode of action of auxin-related herbicides. When CNR genes affect auxin action, they could affect both cell number and cell size, as auxin does. So, while CNR genes affecting cell size is the result of the Fw2.2 gene, and is expected to be the general effect of modulating these genes' expression, one would not rule out a possible accompanying effect on cell size.

The *fw2* tomato gene family has at least 12 members related to it in maize, designated as *ZmCNR1* - *12* (see Table 1). Methods relating to the ZmCNR1 gene may be used to control cell number in the maize plants. Potential uses include controlling the size of whole plants, or specific organs within the crop plant. Methods relating to the ZmCNR1 gene may also be used to control seed and fruit size. Proper control of the gene can result in whole organs being enhanced in size, or reduced or eliminated altogether. Either outcome would be agronomically advantageous. While the gene may generally function as a negative regulator of cell number and thus organ size in its normal wild type function, it is recognized that this function could be altered by judicious manipulation of the level or timing of the expression of the gene, or by altering or disrupting the coding region of the gene. In that instance, the function of the gene product, a protein, is altered. Potential outcomes include but are not limited to: increased leaf size, increased root size, increased ear size, increased seed size which could include increased endosperm size, alteration in the relative size of embryos and endosperm which in turn would effect changes in the relative levels of protein, oil and starch in the seeds, and elimination of tassels, or at least functional pollen bearing tassels.

RT-PCR data shows that *ZmCNR02* expressed highly in ovules prior to fertilization, but declined thereafter. This is consistent with the gene being involved in negative cell number regulation. This observation suggests ways to further exploit this and related genes for crop improvement. Methods related to the CNR1 gene may be used in the area of fertilization independent seed production. The phenomenon of negative cell number regulation (by diminishing CNR expression and/or function) in the ovules could result in seed or seed-like formation in the absence of fertilization. Such seeds may be viable to germinate in the next generation, or they could be useful for seed production for food, fuel, and the other normal consumption roles of seeds. There are a number of possible novel uses and virtues for fertilization independent seed formation that could affect the agriculture industry. Haploids are increasingly being used for parent line production in hybrid crop (maize) production. A potential parent plant can be identified as a haploid, and then doubled to a diploid to achieve a homozygous parent line without the need for inbreeding.

The present invention provides methods for controlling CNR1, and for reducing the activity of this protein in order to express a modified nonfunctional version of the protein. This may disrupt function of the intact natural versions of the genes by blocking or competing for sites of action. The promoter of the CNR gene could be used to direct expression to ovules. Applications include novel means for regulating the CNR or related genes in the ovule as mentioned above, and in other areas where control of gene expression in the ovule is needed. The CNR genes are expressed in many tissues besides the ovule and that expression is increased where tissues are maturing or mature.

One application for CNR1 includes altering the formation of tissue by controlling cell division. For example, controlling functional tassel formation is an important aspect of hybrid maize production. The CNR gene can be operably linked to a tassel-, anther- or tapetum-specific promoter, thereby controlling the development of those tissues, leading to an alteration of male sterility.

**TABLE 1**

| SEQUENCE ID NUMBER | IDENTITY |
|---|---|
| SEQ ID NO: 1 | ZmCNR 1 polynucleotide |
| SEQ ID NO: 2 | ZmCNR 1 polypeptide |
| SEQ ID NO: 3 | ZmCNR 2 polynucleotide |
| SEQ ID NO: 4 | ZmCNR 2 polypeptide |
| SEQ ID NO: 5 | ZmCNR 3 polynucleotide |
| SEQ ID NO: 6 | ZmCNR 3 polypeptide |
| SEQ ID NO: 7 | ZmCNR 4 polynucleotide |
| SEQ ID NO: 8 | ZmCNR 4 polypeptide |
| SEQ ID NO: 9 | ZmCNR 5 polynucleotide |
| SEQ ID NO: 10 | ZmCNR 5 polypeptide |
| SEQ ID NO: 11 | ZmCNR 6 polynucleotide |
| SEQ ID NO: 12 | ZmCNR 6 polypeptide |
| SEQ ID NO: 13 | ZmCNR 7 polynucleotide |
| SEQ ID NO: 14 | ZmCNR 7 polypeptide |
| SEQ ID NO: 15 | ZmCNR 8 polynucleotide |
| SEQ ID NO: 16 | ZmCNR 8 polypeptide |
| SEQ ID NO: 17 | ZmCNR 9 polynucleotide |
| SEQ ID NO: 18 | ZmCNR 9 polypeptide |
| SEQ ID NO: 19 | ZmCNR 10 polynucleotide |
| SEQ ID NO: 20 | ZmCNR 10 polypeptide |
| SEQ ID NO: 21 | ZmCNR 11 polynucleotide |
| SEQ ID NO: 22 | ZmCNR 11 polypeptide |
| SEQ ID NO: 23 | ZmCNR 12 polynucleotide |
| SEQ ID NO: 24 | ZmCNR 12 polypeptide |
| SEQ ID NO: 25 | ZmCNR 2 polypeptide full length with ORF identification |
| SEQ ID NO: 26 | ZmCNR 2 open reading frame |
| SEQ ID NO: 27 | ZmCNR 2 polypeptide translation of SEQ ID NO: 26 |
| SEQ ID NO: 28 | ZmCNR 1 Promoter |
| SEQ ID NO: 29 | ZmCNR 2 Promoter |
| SEQ ID NO: 30 | ZmCNR 4 Promoter |
| SEQ ID NO: 31 | ZmCNR 6 Promoter |
| SEQ ID NO: 32 | ZmCNR 7 Promoter |
| SEQ ID NO: 33 | ZmCNR 9 Promoter |
| SEQ ID NO: 34 | ZmCNR 11 Promoter |
| SEQ ID NO: 35 | ZmCNR 12 Promoter |
| SEQ ID NO: 36 | ZmCNR 1 MPSS preferred Tag |
| SEQ ID NO: 37 | ZmCNR 2 MPSS preferred Tag |
| SEQ ID NO: 38 | ZmCNR 3 MPSS preferred Tag |
| SEQ ID NO: 39 | ZmCNR 5 MPSS preferred Tag |
| SEQ ID NO: 40 | ZmCNR 6 MPSS preferred Tag |
| SEQ ID NO: 41 | ZmCNR 7 and ZmCNR 9 MPSS preferred Tag |
| SEQ ID NO: 42 | ZmCNR 8 MPSS preferred Tag |
| SEQ ID NO: 43 | ZmCNR 10 MPSS preferred Tag |
| SEQ ID NO: 44 | ORF translation of homologue to Lycopersicon esculentum *fw2.2* |
| SEQ ID NO: 45 | Lycopersicon esculentum *fw2.2* |

The invention provides a method of increasing the cell number regulatory activity in a plant, comprising:
(a) introducing into a plant cell a recombinant expression cassette, said cassette comprising a polynucleotide selected from:
   (i) a polynucleotide of SEQ ID NO: 1 or a polynucleotide having at least 90% sequence identity, as determined by the GAP algorithm under default parameters, to the full length sequence of a polynucleotide of SEQ ID NO: 1; wherein the polynucleotide encodes a polypeptide that functions as a modifier of cell number, and
   (ii) a polynucleotide encoding a polypeptide of SEQ ID NO: 2 or a polypeptide having at least 90% sequence identity, as determined by the GAP algorithm under default parameters, to the full length sequence of a polypeptide of SEQ ID NO: 2, wherein the polypeptide functions as a modifier of cell number;
   operably linked, in sense or anti-sense orientation, to a promoter; and
(b) regenerating a plant from said plant cell;
wherein the cell number regulatory activity in said plant is increased and the plant has decreased root growth, decreased seed size, decreased seed weight, decreased embryo size, or decreased tassel production.
The invention further provides a method of reducing or eliminating the activity of a cell number regulatory (CNR) polypeptide in a plant, wherein said CNR polypeptide is encoded by a polynucleotide of SEQ ID NO: 1 or a a polynucleotide having at least 90% sequence identity, as determined by the GAP algorithm under default parameters, to the full length sequence of a polynucleotide of SEQ ID NO: 1; said method comprising:
(a) transformation of a plant cell with an expression cassette that expresses a polynucleotide that inhibits the expression of said CNR polypeptide, or
(b) gene disruption of said polynucleotide encoding said CNR polypeptide, and
and regeneration of a plant in which the activity of said CNR polypeptide is reduced or eliminated; said plant having enhanced root growth, increased seed size, increased seed weight, increased embryo size, increased leaf size, increased seedling vigor, enhanced silk emergence, or increased ear size.

The level or activity of the polypeptide could also be reduced or eliminated in specific tissues, causing increased cell number in said tissues. Reducing the level and/or activity of the CNR polypeptide increases the number of cells produced in the associated tissue.

Methods for regulating gene expression in a plant are also provided. In specific embodiments, the DNA construct is stably integrated into the genome of the plant. The method comprises introducing into a plant a nucleotide sequence of interest operably linked to a promoter.

### BRIEF DESCRIPTION OF THE FIGURES

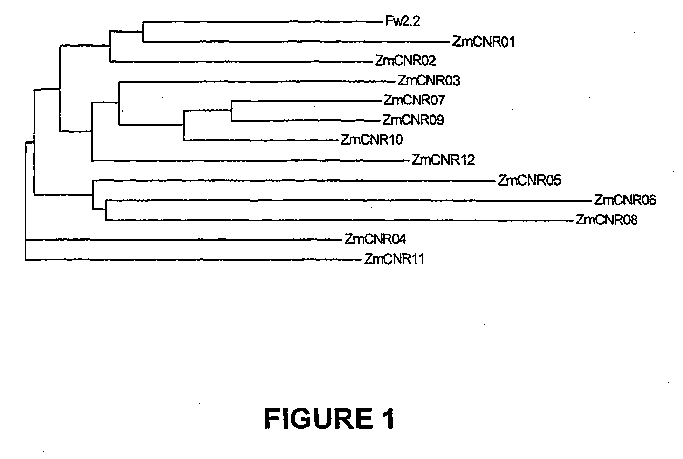

Figure 1. Clustal Dendogram of Tomato *Fw2-2* (SEQ ID NO: 45) with 12 Maize Gene Translations (SEQ ID NOS: 2, 4, 6, 8, 10, 12, 14, 16, 18, 20, 22, and 24).

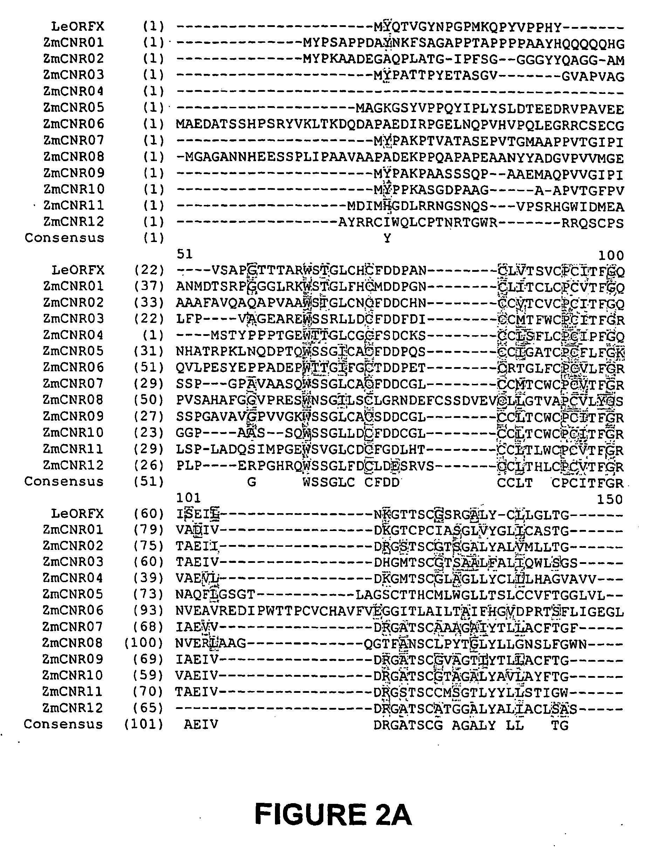

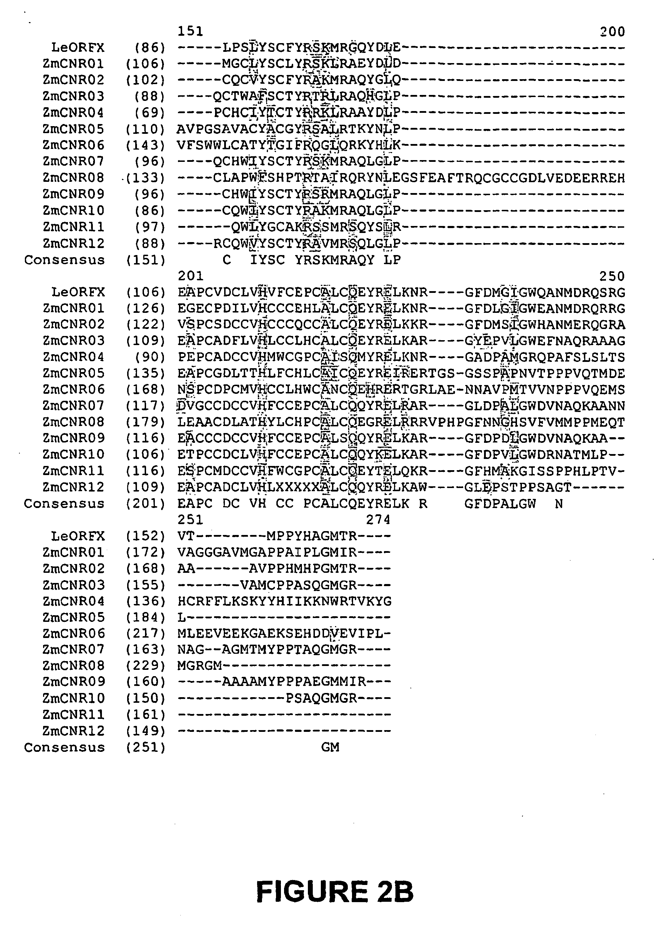

Figure 2. Alignment of Tomato *Fw2-2* (SEQ ID NO: 45) with 12 Maize Gene Translations (SEQ ID NOS: 2, 4, 6, 8, 10, 12, 14, 16,18, 20, 22, and 24).

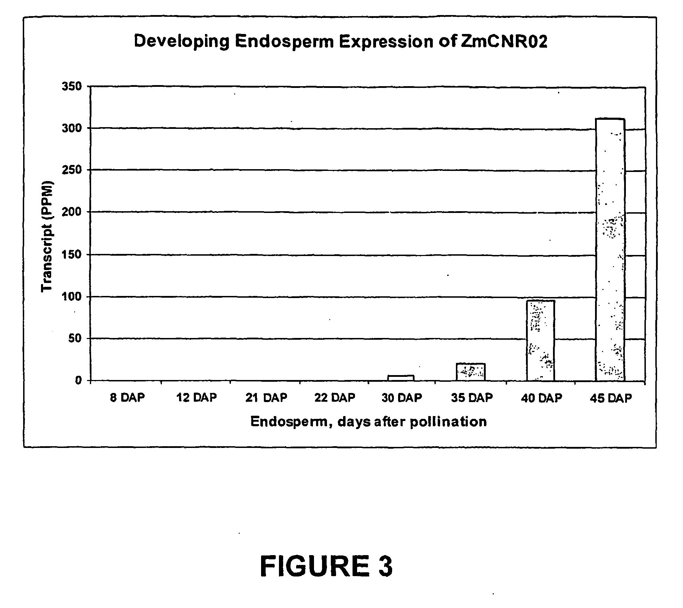

Figure 3. Endosperm Development. The pattern of *ZmCNR02* gene expression as revealed by MPSS data reveals that the gene expression is very low in the early stages of endosperm development (in early days after pollination - DAP), but that as the endosperm matures (higher DAP), the expression of CNR02 increases. Thus this pattern of expression in endosperm is consistent with a role of CNR02 in negatively regulating cell number.

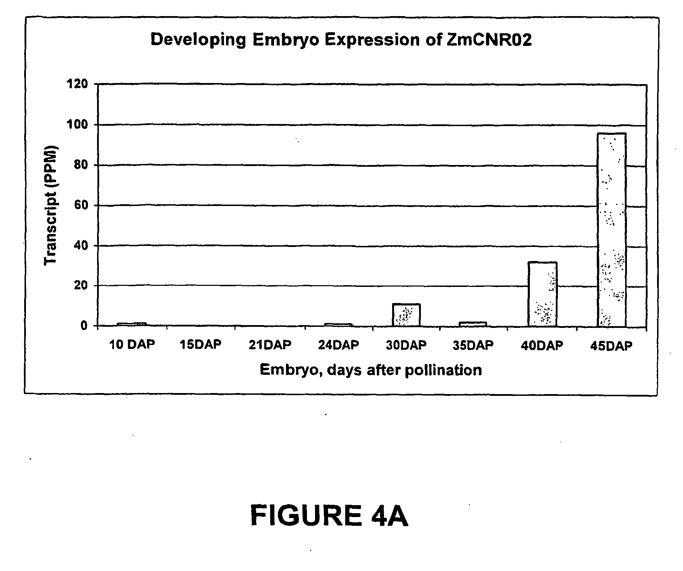

Figure 4A. Embryo Development. The seed embryo development is scored in terms of days after pollination (DAP). The pattern of *ZmCNR02* expression rises towards the end of embryo development after 30 DAP, with the highest expression at 45 DAP. This corresponds to the period of completion of cell number growth, this pattern of expression is consistent with a role for *ZmCNR02* as a negative cell number regulator.

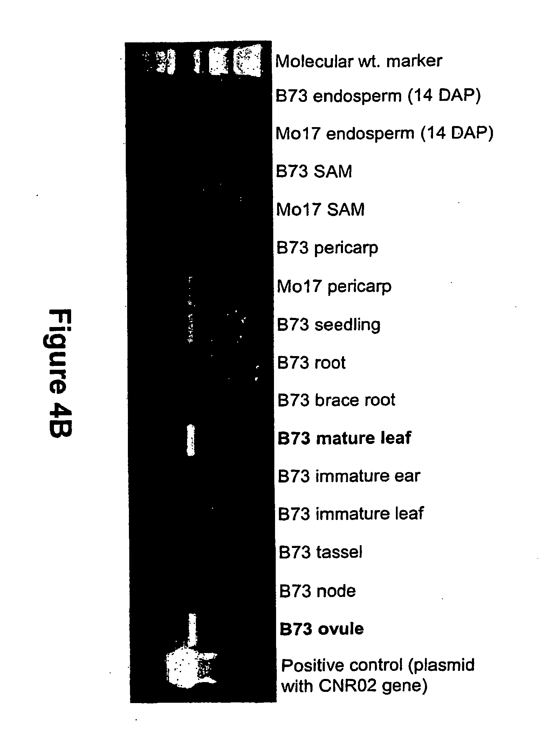

Figure 4B. RT-PCT analysis of *ZmCNR02* expression in different maize tissues. Thirty-five cycles of RT-PCR was performed with different maize tissues, including endosperm (14 DAP), shoot apical meristem, pericarp, seedling, root, brace root, mature and immature leaf, immature ear, immature tassel, node, and ovule. Consistent with the Lynx MPSS profiling data, the expression of this gene is detected mostly in the tissue where there is little growth activity, such as mature leaf. Interestingly, a very high expression is detected in the ovule tissue. The ovule (pre-fertilization) has no cell division activity and is at a rest stage. *ZmCNR02* is expressed at a very high level in the ovule, comparable to the level in the mature leaf tissue. However, immediately after fertilization when active cell division begins, the *ZmCNR02* expression is dramatically reduced to a minimal level (demonstrated by the early embryo and endosperm development illustrated in Figures 3 and 4A).

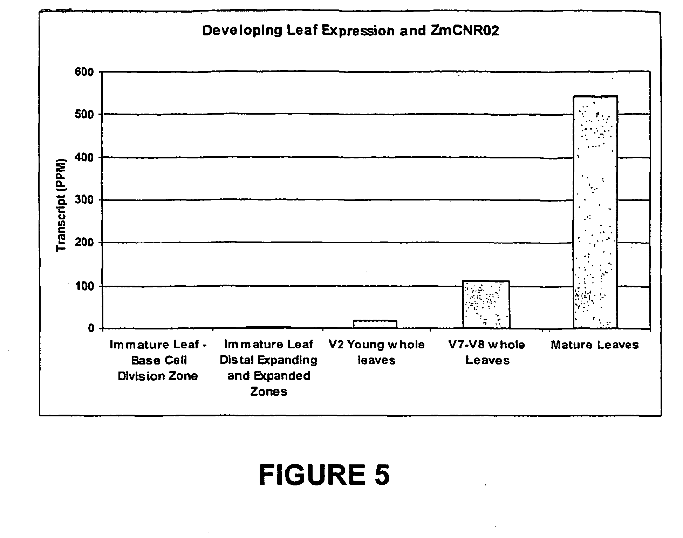

Figure 5. Leaf Development. Several samples were assayed in relation to developing maize leaves. The basal region of immature leaves, the region of most active cell division, showed no *ZmCNR02* expression. The distal expanding and expanded portions of the same immature leaves showed a small but noticeable *ZmCNR02* expression. A series of whole leaves from young plants (V2) to middle stage leaves (V7-V8) to mature leaves, showed progressively higher *ZmCNR02* expression. This expression pattern is consistent with *ZmCNR02* being related to negative control of cell number; its expression is highest in leaf stages that are undergoing little cell division.

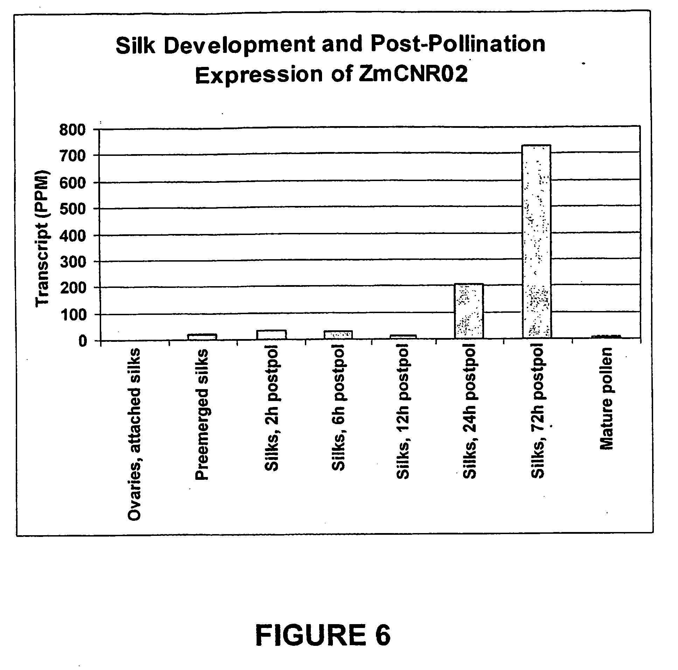

Figure 6. Carpels, Silk Development, and Pollen. The silks, ovary walls and pericarp are analogous to the dicot flower carpel. *ZmCNR02* expression is detected in the latter two. The *ZmCNR02* expression is in the maize 'carpels' by virtue of the silk and pericarp expression. The pericarp samples assayed are fairly late in development and are compromised by remaining endosperm tissue. The silk tissues are fairly easy to gather and assay for gene expression. In the young growing silks (those still attached to the ovaries) the expression of *ZmCNR02* expression is not detected. Then moving through a series of pre-emergent to post emergent silks, and thence through a post pollination series, the expression of *ZmCNR02* increases. For comparison the pollen sample is offered indicating that the increase of *ZmCNR02* expression is not derived from the pollen landing on the silks. As silks mature, and especially after they are pollinated, the cell division slows and stops. The pattern of *ZmCNR02* expression in silks (a carpel tissue) is consistent with a negative cell number regulator.

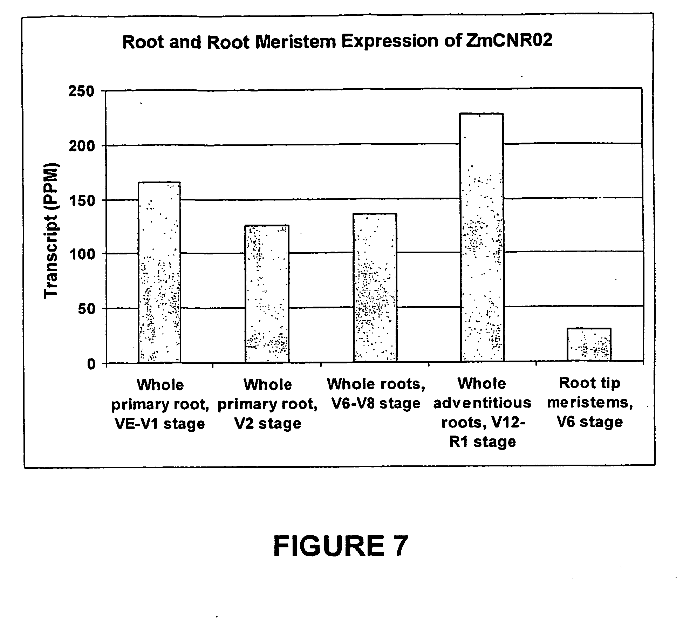

Figure 7. Root and Root Meristems. A comparison of whole roots (with meristems) to root tips (meristem enriched), shows that *ZmCNR02* expression is higher in whole roots than root tips. The *ZmCNR02* expression having higher expression in areas of the root not actively dividing, and the expression pattern in roots is consistent with as a negative regulator of cell number (division).

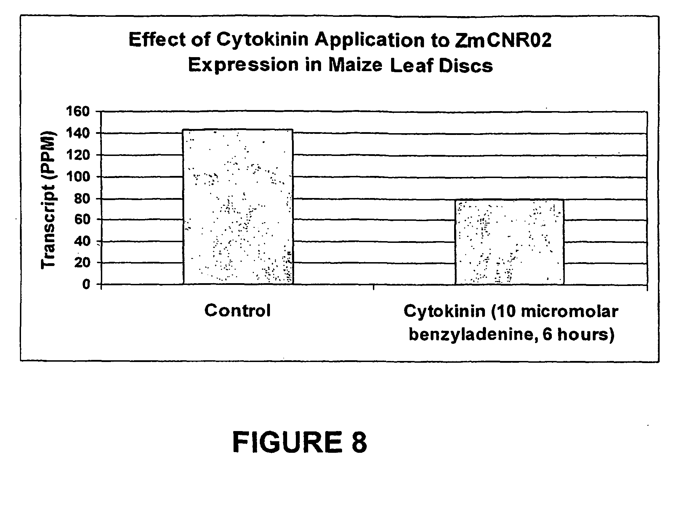

Figure 8. Cytokinin Treatment. Data from an experiment showing that the *ZmCNR02* genes' expression, as revealed by MPSS transcript assay, decreases in excised maize leaf discs, when 10 micromolar benzyladenine is added for 6 hours. This result offers additional evidence that the expression of *ZmCNR02* is consistent with a role in negatively regulating cell number. The addition of a plant hormone that is known to induce cell number (cell division) results in the decline in expression of *ZmCNR02,* as expected per the hypothesis that this gene negatively regulates cell number.

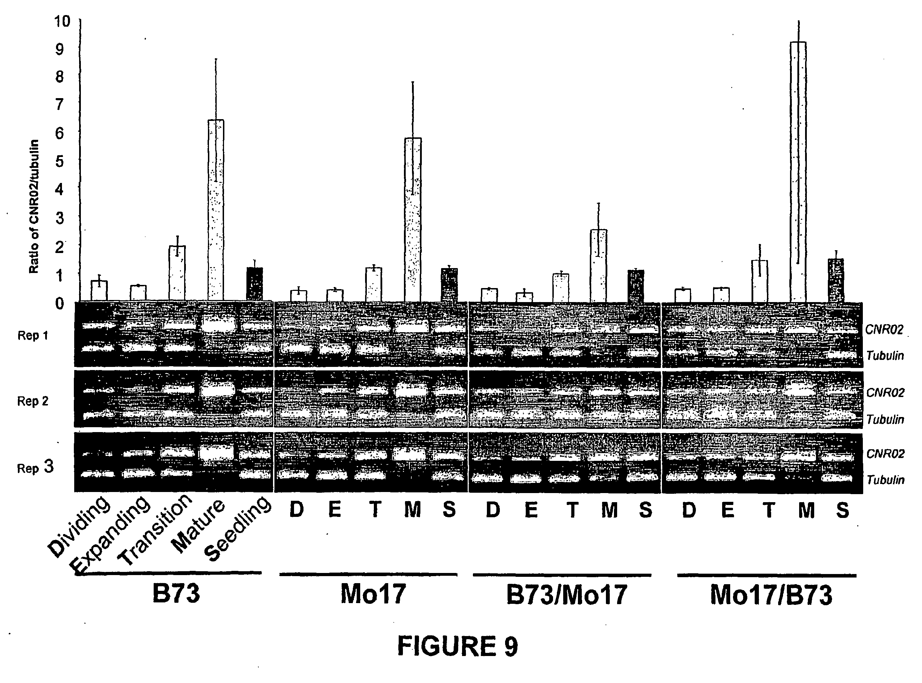

Figure 9. *ZmCNR02* Expression Negative Correlation with Growth: RT-PCR analysis of leaf sections of different growth activity in four genotypes. Leaf sections of different growth activity are collected from seedlings at V3 stage. The RT-PCR analysis of *ZmCNR02* is multiplexes with tubulin as a control. RT-PCR analysis confirmed the negative correlation of *ZmCNR02* expression with growth and activity in different expression platform from MPSS profiling. The negatively correlative relationship with growth is consistently seen in all four different genotypes tested, indicating the general role in growth of this gene regardless of the genetic backgrounds.

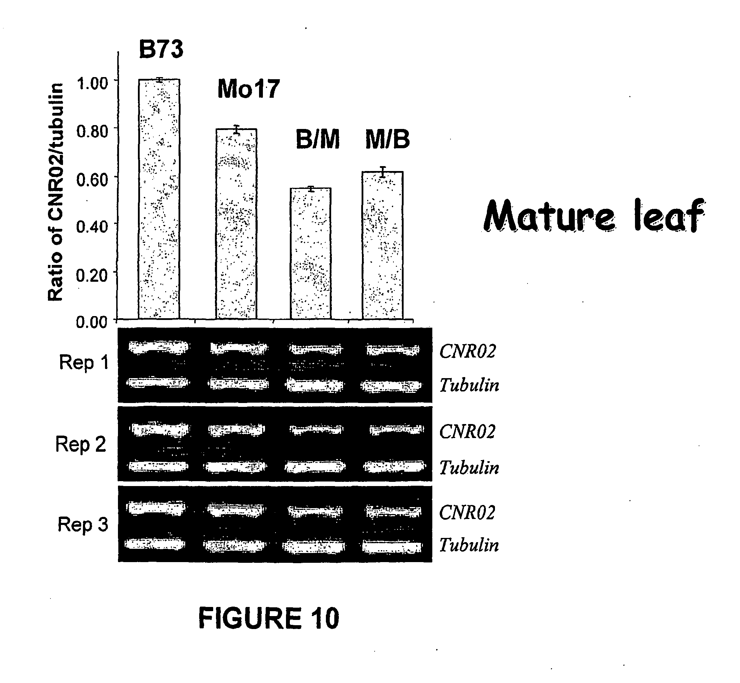

Figure 10. *ZmCNR02* Expression in Mature Leaf of inbred parents and their reciprocal hybrids. This is a RT-PCR assay with the mature leaf tissue, where the PCR protocol was modified to increase the amplification of tubulin that was out-competed by *ZmCNR02*'s high expression. This figure shows well that the expression level of *ZmCNR02* in both hybrids is significantly higher than the inbred parents.

### DETAILED DESCRIPTION OF THE INVENTION

Unless defined otherwise, all technical and scientific terms used herein have the same meaning as commonly understood by one of ordinary skill in the art to which this invention belongs. Unless mentioned otherwise, the techniques employed or contemplated herein are standard methodologies well known to one of ordinary skill in the art. The materials, methods and examples are illustrative only and not limiting. The following is presented by way of illustration and is not intended to limit the scope of the invention.

The present inventions now will be described more fully hereinafter with reference to the accompanying drawings, in which some, but not all embodiments of the invention are shown. Indeed, these inventions may be embodied in many different forms and should not be construed as limited to the embodiments set forth herein; rather, these embodiments are provided so that this disclosure will satisfy applicable legal requirements. Like numbers refer to like elements throughout.

Many modifications and other embodiments of the inventions set forth herein will come to mind to one skilled in the art to which these inventions pertain having the benefit of the teachings presented in the foregoing descriptions and the associated drawings. Therefore, it is to be understood that the inventions are not to be limited to the specific embodiments disclosed and that modifications and other embodiments are intended to be included within the scope of the appended claims. Although specific terms are employed herein, they are used in a generic and descriptive sense only and not for purposes of limitation.

The practice of the present invention will employ, unless otherwise indicated, conventional techniques of botany, microbiology, tissue culture, molecular biology, chemistry, biochemistry and recombinant DNA technology, which are within the skill of the art. Such techniques are explained fully in the literature. *See, e*.*g*., Langenheim and Thimann, BOTANY: PLANT BIOLOGY AND ITS RELATION TO HUMAN AFFAIRS, John Wiley (1982); CELL CULTURE AND SOMATIC CELL GENETICS OF PLANTS, vol. 1, Vasil, ed. (1984); Stanier et al., THE MICROBIAL WORLD, 5th ed., Prentice-Hall (1986); Dhringra and Sinclair, BASIC PLANT PATHOLOGY METHODS, CRC Press (1985); Maniatis et al., MOLECULAR CLONING: A LABORATORY MANUAL (1982); DNA CLONING, vols. I and II, Glover, ed. (1985); OLIGONUCLEOTIDE SYNTHESIS, Gait, ed. (1984); NUCLEIC ACID HYBRIDIZATION, Hames and Higgins, eds. (1984); and the series METHODS IN ENZYMOLOGY, Colowick and Kaplan, eds, Academic Press, Inc., San Diego, CA.

Units, prefixes, and symbols may be denoted in their SI accepted form. Unless otherwise indicated, nucleic acids are written left to right in 5' to 3' orientation; amino acid sequences are written left to right in amino to carboxy orientation, respectively. Numeric ranges are inclusive of the numbers defining the range. Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes. The terms defined below are more fully defined by reference to the specification as a whole.

In describing the present invention, the following terms will be employed, and are intended to be defined as indicated below.

By "microbe" is meant any microorganism (including both eukaryotic and prokaryotic microorganisms), such as fungi, yeast, bacteria, actinomycetes, algae and protozoa, as well as other unicellular structures.

By "amplified" is meant the construction of multiple copies of a nucleic acid sequence or multiple copies complementary to the nucleic acid sequence using at least one of the nucleic acid sequences as a template. Amplification systems include the polymerase chain reaction (PCR) system, ligase chain reaction (LCR) system, nucleic acid sequence based amplification (NASBA, Cangene, Mississauga, Ontario), Q-*Beta* Replicase systems, transcription-based amplification system (TAS), and strand displacement amplification (SDA). See, *e.g*., DIAGNOSTIC MOLECULAR MICROBIOLOGY: PRINCIPLES AND APPLICATIONS, Persing et a/., eds., American Society for Microbiology, Washington, DC (1993). The product of amplification is termed an amplicon.

The term "conservatively modified variants" applies to both amino acid and nucleic acid sequences. With respect to particular nucleic acid sequences, conservatively modified variants refer to those nucleic acids that encode identical or conservatively modified variants of the amino acid sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations" and represent one species of conservatively modified variation. Every nucleic acid sequence herein that encodes a polypeptide also describes every possible silent variation of the nucleic acid. One of ordinary skill will recognize that each codon in a nucleic acid (except AUG, which is ordinarily the only codon for methionine; one exception is *Micrococcus rubens*, for which GTG is the methionine codon (Ishizuka et al., J. Gen. Microbiol. 139:425-32 (1993)) can be modified to yield a functionally identical molecule. Accordingly, each silent variation of a nucleic acid, which encodes a polypeptide of the present invention, is implicit in each described polypeptide sequence.

As to amino acid sequences, one of skill will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" when the alteration results in the substitution of an amino acid with a chemically similar amino acid. Thus, any number of amino acid residues selected from the group of integers consisting of from 1 to 15 can be so altered. Thus, for example, 1, 2, 3, 4, 5, 7, or 10 alterations can be made. Conservatively modified variants typically provide similar biological activity as the unmodified polypeptide sequence from which they are derived. For example, substrate specificity, enzyme activity, or ligand/receptor binding is generally at least 30%, 40%, 50%, 60%, 70%, 80%, or 90%, preferably 60-90% of the native protein for it's native substrate. Conservative substitution tables providing functionally similar amino acids are well known in the art.

The following six groups each contain amino acids that are conservative substitutions for one another:
1) Alanine (A), Serine (S), Threonine (T);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V); and
6) Phenylalanine (F), Tyrosine (Y), Tryptophan (W).
See also, Creighton, PROTEINS, W.H. Freeman and Co. (1984).

As used herein, "consisting essentially of" means the inclusion of additional sequences to an object polynucleotide where the additional sequences do not selectively hybridize, under stringent hybridization conditions, to the same cDNA as the polynucleotide and where the hybridization conditions include a wash step in 0.1 X SSC and 0.1 % sodium dodecyl sulfate at 65°C.

By "encoding" or "encoded," with respect to a specified nucleic acid, is meant comprising the information for translation into the specified protein. A nucleic acid encoding a protein may comprise non-translated sequences (*e.g*., introns) within translated regions of the nucleic acid, or may lack such intervening non-translated sequences (*e.g*., as in cDNA). The information by which a protein is encoded is specified by the use of codons. Typically, the amino acid sequence is encoded by the nucleic acid using the "universal" genetic code. However, variants of the universal code, such as is present in some plant, animal, and fungal mitochondria, the bacterium *Mycoplasma capricolum* (Yamao et al., Proc. Natl. Acad. Sci. USA 82:2306-9 (1985)), or the ciliate *Macronucleus*, may be used when the nucleic acid is expressed using these organisms.

When the nucleic acid is prepared or altered synthetically, advantage can be taken of known codon preferences of the intended host where the nucleic acid is to be expressed. For example, although nucleic acid sequences of the present invention may be expressed in both monocotyledonous and dicotyledonous plant species, sequences can be modified to account for the specific codon preferences and GC content preferences of monocotyledonous plants or dicotyledonous plants as these preferences have been shown to differ (Murray et al., Nucleic Acids Res. 17:477-98 (1989)). Thus, the maize preferred codon for a particular amino acid might be derived from known gene sequences from maize. Maize codon usage for 28 genes from maize plants is listed in Table 4 of Murray *et al*., *supra*.

As used herein, "heterologous° in reference to a nucleic acid is a nucleic acid that originates from a foreign species, or, if from the same species, is substantially modified from its native form in composition and/or genomic locus by deliberate human intervention. For example, a promoter operably linked to a heterologous structural gene is from a species different from that from which the structural gene was derived or, if from the same species, one or both are substantially modified from their original form. A heterologous protein may originate from a foreign species or, if from the same species, is substantially modified from its original form by deliberate human intervention.

By "host cell, is meant a cell, which comprises a heterologous nucleic acid sequence of the invention, which contains a vector and supports the replication and/or expression of the expression vector. Host cells may be prokaryotic cells such as *E. coli*, or eukaryotic cells such as yeast, insect, plant, amphibian, or mammalian cells. Preferably, host cells are monocotyledonous or dicotyledonous plant cells, including but not limited to maize, sorghum, sunflower, soybean, wheat, alfalfa, rice, cotton, canola, barley, millet, and tomato. A particularly preferred monocotyledonous host cell is a maize host cell.

The term "hybridization complex" includes reference to a duplex nucleic acid structure formed by two single-stranded nucleic acid sequences selectively hybridized with each other.

The term "introduced" in the context of inserting a nucleic acid into a cell, means "transfection" or "transformation" or "transduction" and includes reference to the incorporation of a nucleic acid into a eukaryotic or prokaryotic cell where the nucleic acid may be incorporated into the genome of the cell (*e*.*g*., chromosome, plasmid, plastid or mitochondrial DNA), converted into an autonomous replicon, or transiently expressed (*e.g*., transfected mRNA).

The terms "isolated" refers to material, such as a nucleic acid or a protein, which is substantially or essentially free from components which normally accompany or interact with it as found in its naturally occurring environment. The isolated material optionally comprises material not found with the material in its natural environment. Nucleic acids, which are "isolated", as defined herein, are also referred to as "heterologous" nucleic acids. Unless otherwise stated, the term "CNR nucleic acid" means a nucleic acid comprising a polynucleotide ("CNR polynucleotide") encoding a full length or partial length CNR polypeptide.

As used herein, "nucleic acid" includes reference to a deoxyribonucleotide or ribonucleotide polymer in either single- or double-stranded form, and unless otherwise limited, encompasses known analogues having the essential nature of natural nucleotides in that they hybridize to single-stranded nucleic acids in a manner similar to naturally occurring nucleotides (*e.g*., peptide nucleic acids).

By "nucleic acid library" is meant a collection of isolated DNA or RNA molecules, which comprise and substantially represent the entire transcribed fraction of a genome of a specified organism. Construction of exemplary nucleic acid libraries, such as genomic and cDNA libraries, is taught in standard molecular biology references such as Berger and Kimmel, GUIDE TO MOLECULAR CLONING TECHNIQUES, from the series METHODS IN ENZYMOLOGY, vol. 152, Academic Press, Inc., San Diego, CA (1987); Sambrook et al., MOLECULAR CLONING: A LABORATORY MANUAL, 2nd ed., vols. 1-3 (1989); and CURRENT PROTOCOLS IN MOLECULAR BIOLOGY, Ausubel et al., eds, Current Protocols, a joint venture between Greene Publishing Associates, Inc. and John Wiley & Sons, Inc. (1994 Supplement).

As used herein "operably linked" includes reference to a functional linkage between a first sequence, such as a promoter, and a second sequence, wherein the promoter sequence initiates and mediates transcription of the DNA corresponding to the second sequence. Generally, operably linked means that the nucleic acid sequences being linked are contiguous and, where necessary to join two protein coding regions, contiguous and in the same reading frame.

As used herein, the term "plant" includes reference to whole plants, plant organs (*e.g*., leaves, stems, roots, etc.), seeds and plant cells and progeny of same. Plant cell, as used herein includes, without limitation, seeds, suspension cultures, embryos, meristematic regions, callus tissue, leaves, roots, shoots, gametophytes, sporophytes, pollen, and microspores. The class of plants, which can be used in the methods of the invention, is generally as broad as the class of higher plants amenable to transformation techniques, including both monocotyledonous and dicotyledonous plants including species from the genera: *Cucurbita, Rosa, Vitis, Juglans, Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersicon, Nicotiana, Solanum, Petunia, Digitalis, Majorana, Ciahorium, Helianthus, Lactuca, Bromus, Asparagus, Antirrhinum, Heterocallis, Nemesis, Pelargonium, Panieum, Pennisetum, Ranunculus, Senecio, Salpiglossis, Cucumis, Browaalia, Glycine, Pisum, Phaseolus, Lolium, Oryza, Avena, Hordeum, Secale, Allium,* and *Triticum*. A particularly preferred plant is *Zea mays.*

As used herein, "yield" may include reference to bushels per acre of a grain crop at harvest, as adjusted for grain moisture (15% typically for maize, for example). *(There are also references to alfalfa yield, root compound yield, & others.)* Grain moisture is measured in the grain at harvest. The adjusted test weight of grain is determined to be the weight in pounds per bushel, adjusted for grain moisture level at harvest.

As used herein, "polynucleotide" includes reference to a deoxyribopolynucleotide, ribopolynucleotide, or analogs thereof that have the essential nature of a natural ribonucleotide in that they hybridize, under stringent hybridization conditions, to substantially the same nucleotide sequence as naturally occurring nucleotides and/or allow translation into the same amino acid(s) as the naturally occurring nucleotide(s). A polynucleotide can be full-length or a subsequence of a native or heterologous structural or regulatory gene. Unless otherwise indicated, the term includes reference to the specified sequence as well as the complementary sequence thereof. Thus, DNAs or RNAs with backbones modified for stability or for other reasons are "polynucleotides" as that term is intended herein. Moreover, DNAs or RNAs comprising unusual bases, such as inosine, or modified bases, such as tritylated bases, to name just two examples, are polynucleotides as the term is used herein. It will be appreciated that a great variety of modifications have been made to DNA and RNA that serve many useful purposes known to those of skill in the art. The term polynucleotide as it is employed herein embraces such chemically, enzymatically or metabolically modified forms of polynucleotides, as well as the chemical forms of DNA and RNA characteristic of viruses and cells, including *inter alia,* simple and complex cells.

The terms "polypeptide," "peptide," and "protein" are used interchangeably herein to refer to a polymer of amino acid residues. The terms apply to amino acid polymers in which one or more amino acid residue is an artificial chemical analogue of a corresponding naturally occurring amino acid, as well as to naturally occurring amino acid polymers.

As used herein "promoter" includes reference to a region of DNA upstream from the start of transcription and involved in recognition and binding of RNA polymerase and other proteins to initiate transcription. A "plant promoter" is a promoter capable of initiating transcription in plant cells. Exemplary plant promoters include, but are not limited to, those that are obtained from plants, plant viruses, and bacteria which comprise genes expressed in plant cells such *Agrobacterium* or *Rhizobium.* Examples are promoters that preferentially initiate transcription in certain tissues, such as leaves, roots, seeds, fibres, xylem vessels, tracheids, or sclerenchyma. Such promoters are referred to as "tissue preferred." A "cell type" specific promoter primarily drives expression in certain cell types in one or more organs, for example, vascular cells in roots or leaves. An "inducible" or "regulatable" promoter is a promoter, which is under environmental control. Examples of environmental conditions that may effect transcription by inducible promoters include anaerobic conditions or the presence of light. Another type of promoter is a developmentally regulated promoter, for example, a promoter that drives expression during pollen development. Tissue preferred, cell type specific, developmentally regulated, and inducible promoters constitute the class of "non-constitutive" promoters. A "constitutive" promoter is a promoter, which is active under most environmental conditions.

The term "CNR polypeptide" refers to one or more amino acid sequences. The term is also inclusive of fragments, variants, homologs, alleles or precursors (*e.g*., preproproteins or proproteins) thereof. A "CNR protein" comprises a CNR polypeptide. Unless otherwise stated, the term "CNR nucleic acid" means a nucleic acid comprising a polynucleotide ("CNR polynucleotide") encoding a CNR polypeptide.

As used herein "recombinant" includes reference to a cell or vector, that has been modified by the introduction of a heterologous nucleic acid or that the cell is derived from a cell so modified. Thus, for example, recombinant cells express genes that are not found in identical form within the native (non-recombinant) form of the cell or express native genes that are otherwise abnormally expressed, under expressed or not expressed at all as a result of deliberate human intervention; or may have reduced or eliminated expression of a native gene. The term "recombinant" as used herein does not encompass the alteration of the cell or vector by naturally occurring events (*e.g*., spontaneous mutation, natural transformation/transduction/transposition) such as those occurring without deliberate human intervention.

As used herein, a "recombinant expression cassette" is a nucleic acid construct, generated recombinantly or synthetically, with a series of specified nucleic acid elements, which permit transcription of a particular nucleic acid in a target cell. The recombinant expression cassette can be incorporated into a plasmid, chromosome, mitochondrial DNA, plastid DNA, virus, or nucleic acid fragment. Typically, the recombinant expression cassette portion of an expression vector includes, among other sequences, a nucleic acid to be transcribed, and a promoter.

The term "residue" or "amino acid residue" or "amino acid" are used interchangeably herein to refer to an amino acid that is incorporated into a protein, polypeptide, or peptide (collectively "protein"). The amino acid may be a naturally occurring amino acid and, unless otherwise limited, may encompass known analogs of natural amino acids that can function in a similar manner as naturally occurring amino acids.

The term "selectively hybridizes" includes reference to hybridization, under stringent hybridization conditions, of a nucleic acid sequence to a specified nucleic acid target sequence to a detectably greater degree (*e*.*g*., at least 2-fold over background) than its hybridization to non-target nucleic acid sequences and to the substantial exclusion of non-target nucleic acids. Selectively hybridizing sequences typically have about at least 40% sequence identity, preferably 60-90% sequence identity, and most preferably 100% sequence identity (i.e., complementary) with each other.

The terms "stringent conditions" or "stringent hybridization conditions" include reference to conditions under which a probe will hybridize to its target sequence, to a detectably greater degree than other sequences (*e*.*g*., at least 2-fold over background). Stringent conditions are sequence-dependent and will be different in different circumstances. By controlling the stringency of the hybridization and/or washing conditions, target sequences can be identified which can be up to 100% complementary to the probe (homologous probing). Alternatively, stringency conditions can be adjusted to allow some mismatching in sequences so that lower degrees of similarity are detected (heterologous probing). Optimally, the probe is approximately 500 nucleotides in length, but can vary greatly in length from less than 500 nucleotides to equal to the entire length of the target sequence.

Typically, stringent conditions will be those in which the salt concentration is less than about 1.5 M Na ion, typically about 0.01 to 1.0 M Na ion concentration (or other salts) at pH 7.0 to 8.3 and the temperature is at least about 30°C for short probes (*e.g*., 10 to 50 nucleotides) and at least about 60°C for long probes (*e.g*., greater than 50 nucleotides). Stringent conditions may also be achieved with the addition of destabilizing agents such as formamide or Denhardt's. Exemplary low stringency conditions include hybridization with a buffer solution of 30 to 35% formamide, 1 M NaCl, 1% SDS (sodium dodecyl sulphate) at 37°C, and a wash in 1X to 2X SSC (20X SSC = 3.0 M NaCl/0.3 M trisodium citrate) at 50 to 55°C. Exemplary moderate stringency conditions include hybridization in 40 to 45% formamide, 1 M NaCl, 1% SDS at 37°C, and a wash in 0.5X to 1X SSC at 55 to 60°C. Exemplary high stringency conditions include hybridization in 50% formamide, 1 M NaCl, 1% SDS at 37°C, and a wash in 0.1X SSC at 60 to 65°C. Specificity is typically the function of post-hybridization washes, the critical factors being the ionic strength and temperature of the final wash solution. For DNA-DNA hybrids, the Tₘ can be approximated from the equation of Meinkoth and Wahl, Anal. Biochem., 138:267-84 (1984): Tₘ = 81.5°C + 16.6 (log M) + 0.41 (%GC) - 0.61 (% form) - 500/L; where M is the molarity of monovalent cations, %GC is the percentage of guanosine and cytosine nucleotides in the DNA, % form is the percentage of formamide in the hybridization solution, and L is the length of the hybrid in base pairs. The Tₘ is the temperature (under defined ionic strength and pH) at which 50% of a complementary target sequence hybridizes to a perfectly matched probe. Tₘ is reduced by about 1°C for each 1% of mismatching; thus, Tₘ, hybridization and/or wash conditions can be adjusted to hybridize to sequences of the desired identity. For example, if sequences with ≥90% identity are sought, the Tₘ can be decreased 10°C. Generally, stringent conditions are selected to be about 5°C lower than the thermal melting point (Tₘ) for the specific sequence and its complement at a defined ionic strength and pH. However, severely stringent conditions can utilize a hybridization and/or wash at 1, 2, 3, or 4°C lower than the thermal melting point (Tₘ); moderately stringent conditions can utilize a hybridization and/or wash at 6, 7, 8, 9, or 10°C lower than the thermal melting point (Tₘ); low stringency conditions can utilize a hybridization and/or wash at 11, 12, 13, 14, 15, or 20°C lower than the thermal melting point (Tₘ). Using the equation, hybridization and wash compositions, and desired Tₘ, those of ordinary skill will understand that variations in the stringency of hybridization and/or wash solutions are inherently described. If the desired degree of mismatching results in a Tₘ of less than 45°C (aqueous solution) or 32°C (formamide solution) it is preferred to increase the SSC concentration so that a higher temperature can be used. An extensive guide to the hybridization of nucleic acids is found in Tijssen, LABORATORY TECHNIQUES IN BIOCHEMISTRY AND MOLECULAR BIOLOGY-HYBRIDIZATION WITH NUCLEIC ACID PROBES, part I, chapter 2, "Overview of principles of hybridization and the strategy of nucleic acid probe assays," Elsevier, New York (1993); and CURRENT PROTOCOLS IN MOLECULAR BIOLOGY, chapter 2, Ausubel et al., eds, Greene Publishing and Wiley-Interscience, New York (1995). Unless otherwise stated, in the present application high stringency is defined as hybridization in 4X SSC, 5X Denhardt's (5 g Ficoll, 5 g polyvinypyrrolidone, 5 g bovine serum albumin in 500ml of water), 0.1 mg/ml boiled salmon sperm DNA, and 25 mM Na phosphate at 65°C, and a wash in 0.1X SSC, 0.1% SDS at 65°C.

As used herein, "transgenic plant" includes reference to a plant, which comprises within its genome a heterologous polynucleotide. Generally, the heterologous polynucleotide is stably integrated within the genome such that the polynucleotide is passed on to successive generations. The heterologous polynucleotide may be integrated into the genome alone or as part of a recombinant expression cassette. "Transgenic" is used herein to include any cell, cell line, callus, tissue, plant part or plant, the genotype of which has been altered by the presence of heterologous nucleic acid including those transgenics initially so altered as well as those created by sexual crosses or asexual propagation from the initial transgenic. The term "transgenic" as used herein does not encompass the alteration of the genome (chromosomal or extra-chromosomal) by conventional plant breeding methods or by naturally occurring events such as random cross-fertilization, non-recombinant viral infection, non-recombinant bacterial transformation, non-recombinant transposition, or spontaneous mutation.

As used herein, "vector" includes reference to a nucleic acid used in transfection of a host cell and into which can be inserted a polynucleotide. Vectors are often replicons. Expression vectors permit transcription of a nucleic acid inserted therein.

The following terms are used to describe the sequence relationships between two or more nucleic acids or polynucleotides or polypeptides: (a) "reference sequence," (b) "comparison window," (c) "sequence identity," (d) "percentage of sequence identity," and (e) "substantial identity."

As used herein, "reference sequence" is a defined sequence used as a basis for sequence comparison. A reference sequence may be a subset or the entirety of a specified sequence; for example, as a segment of a full-length cDNA or gene sequence, or the complete cDNA or gene sequence.

As used herein, "comparison window" means includes reference to a contiguous and specified segment of a polynucleotide sequence, wherein the polynucleotide sequence may be compared to a reference sequence and wherein the portion of the polynucleotide sequence in the comparison window may comprise additions or deletions (i.e., gaps) compared to the reference sequence (which does not comprise additions or deletions) for optimal alignment of the two sequences. Generally, the comparison window is at least 20 contiguous nucleotides in length, and optionally can be 30, 40, 50, 100, or longer. Those of skill in the art understand that to avoid a high similarity to a reference sequence due to inclusion of gaps in the polynucleotide sequence a gap penalty is typically introduced and is subtracted from the number of matches.

Methods of alignment of nucleotide and amino acid sequences for comparison are well known in the art. The local homology algorithm (BESTFIT) of Smith and Waterman, Adv. Appl. Math 2:482 (1981), may conduct optimal alignment of sequences for comparison; by the homology alignment algorithm (GAP) of Needleman and Wunsch, J. Mol. Biol. 48:443-53 (1970); by the search for similarity method (Tfasta and Fasta) of Pearson and Lipman, Proc. Natl. Acad. Sci. USA 85:2444 (1988); by computerized implementations of these algorithms, including, but not limited to: CLUSTAL in the PC/Gene program by Intelligenetics, Mountain View, California, GAP, BESTFIT, BLAST, FASTA, and TFASTA in the Wisconsin Genetics Software Package, Version 8 (available from Genetics Computer Group ( GCG® programs (Accelrys, Inc., San Diego, CA).). The CLUSTAL program is well described by Higgins and Sharp, Gene 73:237-44 (1988); Higgins and Sharp, CABIOS 5:151-3 (1989); Corpet et al., Nucleic Acids Res. 16:10881-90 (1988); Huang et al., Computer Applications in the Biosciences 8:155-65 (1992), and Pearson et al., Meth. Mol. Biol. 24:307-31 (1984). The preferred program to use for optimal global alignment of multiple sequences is PileUp (Feng and Doolittle, J. Mol. Evol., 25:351-60 (1987) which is similar to the method described by Higgins and Sharp, CABIOS 5:151-53 (1989)). The BLAST family of programs which can be used for database similarity searches includes: BLASTN for nucleotide query sequences against nucleotide database sequences; BLASTX for nucleotide query sequences against protein database sequences; BLASTP for protein query sequences against protein database sequences; TBLASTN for protein query sequences against nucleotide database sequences; and TBLASTX for nucleotide query sequences against nucleotide database sequences. See CURRENT PROTOCOLS IN MOLECULAR BIOLOGY, Chapter 19, Ausubel et al., eds., Greene Publishing and Wiley-Interscience, New York (1995).

GAP uses the algorithm of Needleman and Wunsch, *supra*, to find the alignment of two complete sequences that maximizes the number of matches and minimizes the number of gaps. GAP considers all possible alignments and gap positions and creates the alignment with the largest number of matched bases and the fewest gaps. It allows for the provision of a gap creation penalty and a gap extension penalty in units of matched bases. GAP must make a profit of gap creation penalty number of matches for each gap it inserts. If a gap extension penalty greater than zero is chosen, GAP must, in addition, make a profit for each gap inserted of the length of the gap times the gap extension penalty. Default gap creation penalty values and gap extension penalty values in Version 10 of the Wisconsin Genetics Software Package are 8 and 2, respectively. The gap creation and gap extension penalties can be expressed as an integer selected from the group of integers consisting of from 0 to 100. Thus, for example, the gap creation and gap extension penalties can be 0, 1, 2, 3, 4, 5, 6, 7, 8, 9, 10, 15, 20, 30, 40, 50, or greater.

GAP presents one member of the family of best alignments. There may be many members of this family, but no other member has a better quality. GAP displays four figures of merit for alignments: Quality, Ratio, Identity, and Similarity. The Quality is the metric maximized in order to align the sequences. Ratio is the quality divided by the number of bases in the shorter segment. Percent Identity is the percent of the symbols that actually match. Percent Similarity is the percent of the symbols that are similar. Symbols that are across from gaps are ignored. A similarity is scored when the scoring matrix value for a pair of symbols is greater than or equal to 0.50, the similarity threshold. The scoring matrix used in Version 10 of the Wisconsin Genetics Software Package is BLOSUM62 (see Henikoff and Henikoff, Proc. Natl. Acad. Sci. USA 89:10915 (1989)).

Unless otherwise stated, sequence identity/similarity values provided herein refer to the value obtained using the BLAST 2.0 suite of programs using default parameters (Altschul et al., Nucleic Acids Res. 25:3389-402 (1997)).

As those of ordinary skill in the art will understand, BLAST searches assume that proteins can be modeled as random sequences. However, many real proteins comprise regions of nonrandom sequences, which may be homopolymeric tracts, short-period repeats, or regions enriched in one or more amino acids. Such low-complexity regions may be aligned between unrelated proteins even though other regions of the protein are entirely dissimilar. A number of low-complexity filter programs can be employed to reduce such low-complexity alignments. For example, the SEG (Wooten and Federhen, Comput. Chem. 17:149-63 (1993)) and XNU (Claverie and States, Comput. Chem. 17:191-201 (1993)) low-complexity filters can be employed alone or in combination.

As used herein, "sequence identity" or "identity" in the context of two nucleic acid or polypeptide sequences includes reference to the residues in the two sequences, which are the same when aligned for maximum correspondence over a specified comparison window. When percentage of sequence identity is used in reference to proteins it is recognized that residue positions which are not identical often differ by conservative amino acid substitutions, where amino acid residues are substituted for other amino acid residues with similar chemical properties (*e.g*. charge or hydrophobicity) and therefore do not change the functional properties of the molecule. Where sequences differ in conservative substitutions, the percent sequence identity may be adjusted upwards to correct for the conservative nature of the substitution. Sequences, which differ by such conservative substitutions, are said to have "sequence similarity" or "similarity." Means for making this adjustment are well known to those of skill in the art. Typically this involves scoring a conservative substitution as a partial rather than a full mismatch, thereby increasing the percentage sequence identity. Thus, for example, where an identical amino acid is given a score of 1 and a non-conservative substitution is given a score of zero, a conservative substitution is given a score between zero and 1. The scoring of conservative substitutions is calculated, *e.g*., according to the algorithm of Meyers and Miller, Computer Applic. Biol. Sci. 4:11-17 (1988), *e.g*., as implemented in the program PC/GENE (Intelligenetics, Mountain View, California, USA).

As used herein, "percentage of sequence identity" means the value determined by comparing two optimally aligned sequences over a comparison window, wherein the portion of the polynucleotide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity.

The term "substantial identity" of polynucleotide sequences means that a polynucleotide comprises a sequence that has between 50-100% sequence identity, preferably at least 50% sequence identity, preferably at least 60% sequence identity, preferably at least 70%, more preferably at least 80%, more preferably at least 90%, and most preferably at least 95%, compared to a reference sequence using one of the alignment programs described using standard parameters. One of skill will recognize that these values can be appropriately adjusted to determine corresponding identity of proteins encoded by two nucleotide sequences by taking into account codon degeneracy, amino acid similarity, reading frame positioning and the like. Substantial identity of amino acid sequences for these purposes normally means sequence identity of between 55-100%, preferably at least 55%, preferably at least 60%, more preferably at least 70%, 80%, 90%, and most preferably at least 95%.

Another indication that nucleotide sequences are substantially identical is if two molecules hybridize to each other under stringent conditions. The degeneracy of the genetic code allows for many amino acids substitutions that lead to variety in the nucleotide sequence that code for the same amino acid, hence it is possible that the DNA sequence could code for the same polypeptide but not hybridize to each other under stringent conditions. This may occur, *e.g*., when a copy of a nucleic acid is created using the maximum codon degeneracy permitted by the genetic code. One indication that two nucleic acid sequences are substantially identical is that the polypeptide, which the first nucleic acid encodes, is immunologically cross reactive with the polypeptide encoded by the second nucleic acid.

The terms "substantial identity" in the context of a peptide indicates that a peptide comprises a sequence with between 55-100% sequence identity to a reference sequence preferably at least 55% sequence identity, preferably 60% preferably 70%, more preferably 80%, most preferably at least 90% or 95% sequence identity to the reference sequence over a specified comparison window. Preferably, optimal alignment is conducted using the homology alignment algorithm of Needleman and Wunsch, *supra.* An indication that two peptide sequences are substantially identical is that one peptide is immunologically reactive with antibodies raised against the second peptide. Thus, a peptide is substantially identical to a second peptide, for example, where the two peptides differ only by a conservative substitution. In addition, a peptide can be substantially identical to a second peptide when they differ by a non-conservative change if the epitope that the antibody recognizes is substantially identical. Peptides, which are "substantially similar" share sequences as, noted above except that residue positions, which are not identical, may differ by conservative amino acid changes.

The nucleotides and proteins described herein have an expression pattern which indicates that they regulate cell number and thus play an important role in plant development. The polynucleotides are expressed in various plant tissues. The methods of the present invention thus provide an opportunity to manipulate plant development to alter seed and vegetative tissue development, timing or composition. This may be used to create a sterile plant, a seedless plant or a plant with altered endosperm composition.

Further, the polypeptide-encoding segments of the polynucleotides can be modified to after codon usage. Altered codon usage can be employed to alter translational efficiency and/or to optimize the coding sequence for expression in a desired host or to optimize the codon usage in a heterologous sequence for expression in maize. Codon usage in the coding regions of the polynucleotides can be analyzed statistically using commercially available software packages such as "Codon Preference" available from the University of Wisconsin Genetics Computer Group. See Devereaux et al., Nucleic Acids Res. 12:387-395 (1984)); or MacVector 4.1 (Eastman Kodak Co., New Haven, Conn.).

### Recombinant Expression Cassettes

The methods of the present invention use recombinant expression cassettes comprising a nucleic acid. of the A nucleic acid sequence can be used to construct a recombinant expression cassette which can be introduced into the desired host cell. A recombinant expression cassette will typically comprise a polynucleotide operably linked to transcriptional initiation regulatory sequences which will direct the transcription of the polynucleotide in the intended host cell, such as tissues of a transformed plant.

Such plant expression vectors may also contain, if desired, a promoter regulatory region (*e*.*g*., one conferring inducible or constitutive, environmentally- or developmentally-regulated, or cell- or tissue-specific/selective expression), a transcription initiation start site, a ribosome binding site, an RNA processing signal, a transcription termination site, and/or a polyadenylation signal.

A plant promoter fragment can be employed which will direct expression of a polynucleotide in all tissues of a regenerated plant. Such promoters are referred to herein as "constitutive" promoters and are active under most environmental conditions and states of development or cell differentiation. Examples of constitutive promoters include the 1'- or 2'- promoter derived from T-DNA of *Agrobacterium tumefaciens*, the Smas promoter, the cinnamyl alcohol dehydrogenase promoter (United States Patent No. 5,683,439), the Nos promoter, the rubisco promoter, the GRP1-8 promoter, the 35S promoter from cauliflower mosaic virus (CaMV), as described in Odell et al., Nature 313:810-2 (1985); rice actin (McElroy et al., Plant Cell 163-171 (1990)); ubiquitin (Christensen et al., Plant Mol. Biol. 12:619-632 (1992) and Christensen et al., Plant Mol. Biol. 18:675-89 (1992)); pEMU (Last et al., Theor. Appl. Genet. 81:581-8 (1991)); MAS (Velten et al., EMBO J. 3:2723-30 (1984)); and maize H3 histone (Lepetit et al., Mol. Gen. Genet. 231:276-85 (1992); and Atanassvoa et al., Plant Journal 2(3):291-300 (1992)); ALS promoter, as described in PCT Application No. WO 96/30530; and other transcription initiation regions from various plant genes known to those of skill. For the methods of present invention ubiquitin is the preferred promoter for expression in monocot plants.

Alternatively, the plant promoter can direct expression of a polynucleotide in a specific tissue or may be otherwise under more precise environmental or developmental control. Such promoters are referred to here as "inducible" promoters. Environmental conditions that may effect transcription by inducible promoters include pathogen attack, anaerobic conditions, or the presence of light. Examples of inducible promoters are the Adh1 promoter, which is inducible by hypoxia or cold stress, the Hsp70 promoter, which is inducible by heat stress, and the PPDK promoter, which is inducible by light.

Examples of promoters under developmental control include promoters that initiate transcription only, or preferentially, in certain tissues, such as leaves, roots, fruit, seeds, or flowers. The operation of a promoter may also vary depending on its location in the genome. Thus, an inducible promoter may become fully or partially constitutive in certain locations.

If polypeptide expression is desired, it is generally desirable to include a polyadenylation region at the 3'-end of a polynucleotide coding region. The polyadenylation region can be derived from a variety of plant genes, or from T-DNA. The 3' end sequence to be added can be derived from, for example, the nopaline synthase or octopine synthase genes, or alternatively from another plant gene, or less preferably from any other eukaryotic gene. Examples of such regulatory elements include, but are not limited to, 3' termination and/or polyadenylation regions such as those of the *Agrobacterium tumefaciens* nopaline synthase (nos) gene (Bevan et al., Nucleic Acids Res. 12:369-85 (1983)); the potato proteinase inhibitor II (PINII) gene (Keil et al., Nucleic Acids Res. 14:5641-50 (1986); and An et al., Plant Cell 1:115-22 (1989)); and the CaMV 19S gene (Mogen et al., Plant Cell 2:1261-72 (1990)).

An intron sequence can be added to the 5' untranslated region or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg, Mol. Cell Blot. 8:4395-4405 (1988); Callis et al., Genes Dev. 1:1183-200 (1987)). Such intron enhancement of gene expression is typically greatest when placed near the 5' end of the transcription unit. Use of maize introns Adh1-S intron 1, 2, and 6, the Bronze-1 intron are known in the art. *See generally*, THE MAIZE HANDBOOK, Chapter 116, Freeling and Walbot, eds., Springer, New York (1994).

Plant signal sequences, including, but not limited to, signal-peptide encoding DNA/RNA sequences which target proteins to the extracellular matrix of the plant cell (Dratewka-Kos et al., J. Biol. Chem. 264:4896-900 (1989)), such as the *Nicotiana plumbaginifolia* extension gene (DeLoose et al., Gene 99:95-100 (1991)); signal peptides which target proteins to the vacuole, such as the sweet potato sporamin gene (Matsuka et al., Proc. Natl. Acad. Sci. USA 88:834 (1991)) and the barley lectin gene (Wilkins et al., Plant Cell, 2:301-13 (1990)); signal peptides which cause proteins to be secreted, such as that of PRlb (Lind et al., Plant Mol. Biol. 18:47-53 (1992)) or the barley alpha amylase (BAA) (Rahmatullah et al., Plant Mol. Biol. 12:119 (1989)), or signal peptides which target proteins to the plastids such as that of rapeseed enoyl-Acp reductase (Verwaert et al., Plant Mol. Biol. 26:189-202 (1994)) are useful in the methods of invention. The barley alpha amylase signal sequence fused to the CNR polynucleotide is the preferred construct for expression in maize for the methods of the present invention.

The vector comprising the sequences from a polynucleotide will typically comprise a marker gene, which confers a selectable phenotype on plant cells. Usually, the selectable marker gene will encode antibiotic resistance, with suitable genes including genes coding for resistance to the antibiotic spectinomycin (*e*.*g*., the aada gene), the streptomycin phosphotransferase (SPT) gene coding for streptomycin resistance, the neomycin phosphotransferase (NPTII) gene encoding kanamycin or geneticin resistance, the hygromycin phosphotransferase (HPT) gene coding for hygromycin resistance, genes coding for resistance to herbicides which act to inhibit the action of acetolactate synthase (ALS), in particular the sulfonylurea-type herbicides (*e*.*g*., the acetolactate synthase (ALS) gene containing mutations leading to such resistance in particular the S4 and/or Hra mutations), genes coding for resistance to herbicides which act to inhibit action of glutamine synthase, such as phosphinothricin or basta (*e*.*g*., the bar gene), or other such genes known in the art. The *bar* gene encodes resistance to the herbicide basta, and the ALS gene encodes resistance to the herbicide chlorsulfuron.

Typical vectors useful for expression of genes in higher plants are well known in the art and include vectors derived from the tumor-inducing (Ti) plasmid of *Agrobacterium tumefaciens* described by Rogers et al., Meth. Enzymol. 153:253-77 (1987). These vectors are plant integrating vectors in that on transformation, the vectors integrate a portion of vector DNA into the genome of the host plant. Exemplary *A. tumefaciens* vectors useful herein are plasmids pKYLX6 and pKYLX7 of Schardl et al., Gene 61:1-11 (1987), and Berger et al., Proc. Natl. Acad. Sci. USA, 86:8402-6 (1989). Another useful vector herein is plasmid pBI101.2 that is available from CLONTECH Laboratories, Inc. (Palo Alto, CA).

### Expression of Proteins in Host Cells

Using the above-described nucleic acids, one may express a protein in a recombinantly engineered cell such as bacteria, yeast, insect, mammalian, or preferably plant cells. The cells produce the protein in a non-natural condition (*e*.*g*., in quantity, composition, location, and/or time), because they have been genetically altered through human intervention to do so.

It is expected that those of skill in the art are knowledgeable in the numerous expression systems available for expression of a nucleic acid encoding a protein of the present invention. No attempt to describe in detail the various methods known for the expression of proteins in prokaryotes or eukaryotes will be made.

In brief summary, the expression of isolated nucleic acids encoding a protein of the present invention will typically be achieved by operably linking, for example, the DNA or cDNA to a promoter (which is either constitutive or inducible), followed by incorporation into an expression vector. The vectors can be suitable for replication and integration in either prokaryotes or eukaryotes. Typical expression vectors contain transcription and translation terminators, initiation sequences, and promoters useful for regulation of the expression of the DNA encoding a protein. To obtain high level expression of a cloned gene, it is desirable to construct expression vectors which contain, at the minimum, a strong promoter, such as ubiquitin, to direct transcription, a ribosome binding site for translational initiation, and a transcription/translation terminator Constitutive promoters are classified as providing for a range of constitutive expression. Thus, some are weak constitutive promoters, and others are strong constitutive promoters. Generally, by "weak promoter" is intended a promoter that drives expression of a coding sequence at a low level. By "low level" is intended at levels of about 1/10,000 transcripts to about 1/100,000 transcripts to about 1/500,000 transcripts. Conversely, a "strong promoter" drives expression of a coding sequence at a "high level," or about 1/10 transcripts to about 1/100 transcripts to about 1/1,000 transcripts.

One of skill would recognize that modifications could be made to a protein of the present invention without diminishing its biological activity. Some modifications may be made to facilitate the cloning, expression, or incorporation of the targeting molecule into a fusion protein. Such modifications are well known to those of skill in the art and include, for example, a methionine added at the amino terminus to provide an initiation site, or additional amino acids (*e*.*g*., poly His) placed on either terminus to create conveniently located restriction sites or termination codons or purification sequences.

### Expression in Plant Cells

A variety of eukaryotic expression systems such as yeast, insect cell lines, plant and mammalian cells, are known to those of skill in the art.

When plant host cells are employed, polyadenlyation or transcription terminator sequences are typically incorporated into the vector. An example of a terminator sequence is the polyadenlyation sequence from the bovine growth hormone gene. Sequences for accurate splicing of the transcript may also be included. An example of a splicing sequence is the VP1 intron from SV40 (Sprague et al., J. Virol. 45:773-81 (1983)). Additionally, gene sequences to control replication in the host cell may be incorporated into the vector such as those found in bovine papilloma virus type-vectors (Saveria-Campo, "Bovine Papilloma Virus DNA a Eukaryotic Cloning Vector," in DNA CLONING: A PRACTICAL APPROACH, vol. II, Glover, ed., IRL Press, Arlington, VA, pp. 213-38 (1985)).

In addition, the gene for CNR placed in the appropriate plant expression vector can be used to transform plant cells in the methods of the present invention. The polypeptide can then be isolated from plant callus or the transformed cells can be used to regenerate transgenic plants. Such transgenic plants can be harvested, and the appropriate tissues (seed or leaves, for example) can be subjected to large scale protein extraction and purification techniques.

### Plant Transformation Methods

Numerous methods for introducing foreign genes into plants are known and can be used to insert a CNR polynucleotide into a plant host, including biological and physical plant transformation protocols. See, *e*.*g*., Miki et al., "Procedure for Introducing Foreign DNA into Plants," in METHODS IN PLANT MOLECULAR BIOLOGY AND BIOTECHNOLOGY, Glick and Thompson, eds., CRC Press, Inc., Boca Raton, pp. 67-88 (1993). The methods chosen vary with the host plant, and include chemical transfection methods such as calcium phosphate, microorganism-mediated gene transfer such as *Agrobacterium* (Horsch et al., Science 227:1229-31 (1985)), electroporation, mlcro-injection, and biolistic bombardment.

Expression cassettes and vectors and *in vitro* culture methods for plant cell or tissue transformation and regeneration of plants are known and available. See, *e*.*g*., Grower et al., "Vectors for Plant Transformation," in METHODS IN PLANT MOLECULAR BIOLOGY AND BIOTECHNOLOGY, supra, pp.89-119.

The polynucleotides or polypeptides described above may be introduced into the plant by one or more techniques typically used for direct delivery into cells. Such protocols may vary depending on the type of organism, cell, plant or plant cell, i.e. monocot or dicot, targeted for gene modification. Suitable methods of transforming plant cells include microinjection (Crossway et al. (1986) Biotechniques 4:320-334; and U.S. Patent 6,300,543), electroporation (Riggs et al. (1986) Proc. Natl. Acad. Sci. USA 83:5602-5606, direct gene transfer (Paszkowski at al. (1984) EMBO J. 3:2717-2722), and ballistic particle acceleration (see, for example, Sanford et al., U.S. Patent No. 4,945,050; WO 91/10725; and McCabe et al. (1988) Biotechnology 6:923-926). Also see, Tomes et al., Direct DNA Transfer into Intact Plant Cells Via Microprojectile Bombardment. pp.197-213 in Plant Cell, Tissue and Organ Culture, Fundamental Methods. eds. O. L. Gamborg & G.C. Phillips. Springer-Veriag Berlin Heidelberg New York, 1995; U.S. Patent 5,736,369 (meristem); Weissinger et al. (1988) Ann. Rev. Genet. 22:421-477; Sanford et al. (1987) Particulate Science and Technology 5:27-37 (onion); Christou et al. (1988) Plant Physiol. 87:671-674 (soybean); Datta et al. (1990) Biotechnology 8:736-740 (rice); Klein et al. (1988) Proc. Natl. Acad. Sci. USA 85:4305-4309 (maize); Klein et al. (1988) Biotechnology 6:559-563 (maize); WO 91/10725 (maize); Klein et al. (1988) Plant Physiol. 91:440-444 (maize); Fromm et al. (1990) Biotechnology 8:833-839; and Gordon-Kamm et al. (1990) Plant Cell 2:603-618 (maize); Hooydaas-Van Slogteren & Hooykaas (1984) Nature (London) 311:763-784; Bytebier et al. (1987) Proc. Natl. Acad. Sci. USA 84:5345-5349 (Liliaceae); De Wet et al. (1985) In The Experimental Manipulation of Ovule Tissues, ed. G.P. Chapman et al., pp. 197-209. Longman, NY (pollen); Kaeppler et al. (1990) Plant Cell Reports 9:415-418; and Kaeppler et al. (1992) Theor. Appl. Genet. 84:560-566 (whisker-mediated transformation); U.S. Patent No. 5,693,512 (sonication); D'Halluin et al. (1992) Plant Cell 4:1495-1505 (electroporation); Li et al. (1993) Plant Cell Reports 12:250-255; and Christou & Ford (1995) Annals of Botany 75:407-413 (rice); Osjoda et al. (1996) Nature Biotech. 14:745-750; Agrobacterium mediated maize transformation (U.S. Patent 5,981,840); silicon carbide whisker methods (Frame et al. (1994) Plant J. 6:941-948); laser methods (Guo et al. (1995) Physiologia Plantarum 93:19-24); sonication methods (Bao et al. (1997) Ultrasound in Medicine & Biology 23:953-959; Finer & Finer (2000) Lett Appl Microbiol. 30:406-10; Amoah et al. (2001) J Exp Bot 52:1135-42); polyethylene glycol methods (Krens et al. (1982) Nature 296:72-77); protoplasts of monocot and dicot cells can be transformed using electroporation (Fromm et al. (1985) Proc. Natl. Acad. Sci. USA 82:5824-5828) and microinjection (Crossway et al. (1986) Mol. Gen. Genet. 202:179-185).

### Agrobacterium-mediated Transformation

The most widely utilized method for introducing an expression vector into plants is based on the natural transformation system of *Agrobacterium*. *A. tumefaciens* and *A. rhizogenes* are plant pathogenic soil bacteria, which genetically transform plant cells. The Ti and Ri plasmids of *A. tumefaciens* and *A. rhizogenes,* respectively, carry genes responsible for genetic transformation of plants. See, *e*.*g*., Kado, Crit. Rev. Plant Sci. 10:1 (1991). Descriptions of the *Agrobacterium* vector systems and methods for *Agrobacterium*-mediated gene transfer are provided in Gruber *et al*., *supra*; Miki *et al*., *supra*; and Moloney et al., Plant Cell Reports 8:238 (1989).

Similarly, the gene can be inserted into the T-DNA region of a Ti or Ri plasmid derived from *A. tumefaciens* or *A. rhizogenes*, respectively. Thus, expression cassettes can be constructed as above, using these plasmids. Many control sequences are known which when coupled to a heterologous coding sequence and transformed into a host organism show fidelity in gene expression with respect to tissue/organ specificity of the original coding sequence. See, *e*.*g*., Benfey and Chua, Science 244:174-81 (1989). Particularly suitable control sequences for use in these plasmids are promoters for constitutive leaf-specific expression of the gene in the various target plants. Other useful control sequences include a promoter and terminator from the nopaline synthase gene (NOS). The NOS promoter and terminator are present in the plasmid pARC2, available from the American Type Culture Collection and designated ATCC 67238. If such a system is used, the virulence (*vir*) gene from either the Ti or Ri plasmid must also be present, either along with the T-DNA portion, or via a binary system where the *vir* gene is present on a separate vector. Such systems, vectors for use therein, and methods of transforming plant cells are described in United States Patent No. 4,658,082; *United States Patent* No. 5,262,306, issued November 16, 1993; and Simpson et al., Plant Mol. Biol. 6: 403-15 (1986) (also referenced in the '306 patent).

Once constructed, these plasmids can be placed into *A. rhizogenes* or *A. tumefaciens* and these vectors used to transform cells of plant species, which are ordinarily susceptible to *Fusarium* or *Alternaria* infection. Several other transgenic plants are also contemplated by the present invention including but not limited to soybean, com, sorghum, alfalfa, rice, clover, cabbage, banana, coffee, celery, tobacco, cowpea, cotton, melon and pepper. The selection of either *A. tumefaciens* or *A. rhizogenes* will depend on the plant being transformed thereby. In general *A. tumefaciens* is the preferred organism for transformation. Most dicotyledonous plants, some gymnosperms, and a few monocotyledonous plants (*e*.*g*., certain members of the *Liliales* and *Arales*) are susceptible to infection with *A. tumefaciens. A. rhizogenes* also has a wide host range, embracing most dicots and some gymnosperms, which includes members of the *Leguminosae, Compositae*, and *Chenopodiaceae.* Monocot plants can now be transformed with some success. European Patent Application No. 604 662 A1 discloses a method for transforming monocots using *Agrobacterium*. European Application No. 672 752 A1 discloses a method for transforming monocots with *Agrobacterium* using the scutellum of immature embryos. Ishida *et al*. discuss a method for transforming maize by exposing immature embryos to *A. tumefaciens* (Nature Biotechnology 14:745-50 (1996)).

Once transformed, these cells can be used to regenerate transgenic plants. For example, whole plants can be infected with these vectors by wounding the plant and then introducing the vector into the wound site. Any part of the plant can be wounded, including leaves, stems and roots. Alternatively, plant tissue, in the form of an explant, such as cotyledonary tissue or leaf disks, can be inoculated with these vectors, and cultured under conditions, which promote plant regeneration. Roots or shoots transformed by inoculation of plant tissue with *A. rhizogenes* or *A. tumefaciens*, containing the gene coding for the fumonisin degradation enzyme, can be used as a source of plant tissue to regenerate fumonisin-resistant transgenic plants, either via somatic embryogenesis or organogenesis. Examples of such methods for regenerating plant tissue are disclosed in Shahin. Theor. Appl. Genet. 69:235-40 (1985); United States Patent No. 4,658,082; Simpson, *et al*., *supra*; and United States Patent No. 5,262,306, issued November 16, 1993.

### Direct Gene Transfer

Despite the fact that the host range for *Agrobacterium*-mediated transformation is broad, some major cereal crop species and gymnosperms have generally been recalcitrant to this mode of gene transfer, even though some success has recently been achieved in rice (Hiei et al., The Plant Journal 6:271-82 (1994)). Several methods of plant transformation, collectively referred to as direct gene transfer, have been developed as an alternative to *Agrobacterium*-mediated transformation.

A generally applicable method of plant transformation is microprojectile-mediated transformation, where DNA is carried on the surface of microprojectiles measuring about 1 to 4 µm. The expression vector is introduced into plant tissues with a biolistic device that accelerates the microprojectiles to speeds of 300 to 600 m/s which is sufficient to penetrate the plant cell walls and membranes (Sanford et al., Part. Sci. Technol. 5:27 (1987); Sanford, Trends Biotech 6:299 (1988); Sanford, Physiol. Plant 79:206 (1990); and Klein *et al*., *Biotechnology* 10:268 (1992)).

Another method for physical delivery of DNA to plants is sonication of target cells as described in Zang *et al*., *BioTechnology* 9:996 (1991). Alternatively, liposome or spheroplast fusions have been used to introduce expression vectors into plants. See, *e*.*g*., Deshayes et al., EMBO J. 4:2731 (1985); and Christou et al., Proc. Natl. Acad. Sci. USA 84:3962 (1987). Direct uptake of DNA into protoplasts using CaCl₂ precipitation, polyvinyl alcohol, or poly-L-omithine has also been reported. See, *e*.*g*., Hain et al., Mol. Gen. Genet. 199:161 (1985); and Draper et al., Plant Cell Physiol. 23:451 (1982).

Electroporation of protoplasts and whole cells and tissues has also been described. *See*, *e*.*g*., Donn et al., in Abstracts of the VIIth Int'l. Congress on Plant Cell and Tissue Culture IAPTC, A2-38, p. 53 (1990); D'Halluin et al., Plant Cell 4:1495-505 (1992); and Spencer et al., Plant Mol. Biol. 24:51-61 (1994).

### Increasing the Activity and/or Level of a CNR Polypeptide

Methods are provided to increase the activity and/or level of the CNR polypeptide of the invention. An increase in the level and/or activity of the CNR polypeptide of the invention can be achieved by providing to the plant a CNR polypeptide. The CNR polypeptide can be provided by introducing the amino acid sequence encoding the CNR polypeptide into the plant, introducing into the plant a nucleotide sequence encoding a CNR polypeptide or alternatively by modifying a genomic locus encoding the CNR polypeptide of the invention.

As discussed elsewhere herein, many methods are known the art for providing a polypeptide to a plant including, but not limited to, direct introduction of the polypeptide into the plant, introducing into the plant (transiently or stably) a polynucleotide construct encoding a polypeptide having cell number regulator activity. It is also recognized that the methods of the invention may employ a polynucleotide that is not capable of directing, in the transformed plant, the expression of a protein or an RNA. Thus, the level and/or activity of a CNR polypeptide may be increased by altering the gene encoding the CNR polypeptide or its promoter. See, e.g., Kmiec, U.S. Patent 5,565,350; Zarling et al., PCT/US93103868. Therefore mutagenized plants that carry mutations in CNR genes, where the mutations increase expression of the CNR gene or increase the cell number regulator activity of the encoded CNR polypeptide are provided.

### Reducing the Activity and/or Level of a CNR Polypeptide

Methods are provided to reduce or eliminate the activity of a CNR polypeptide of the invention by transforming a plant cell with an expression cassette that expresses a polynucleotide that inhibits the expression of the CNR polypeptide. The polynucleotide may inhibit the expression of the CNR polypeptide directly, by preventing transcription or translation of the CNR messenger RNA, or indirectly, by encoding a polypeptide that inhibits the transcription or translation of a CNR gene encoding a CNR polypeptide. Methods for inhibiting or eliminating the expression of a gene in a plant are well known in the art, and any such method may be used in the present invention to inhibit the expression of a CNR polypeptide.

In accordance with the present invention, the expression of a CNR polypeptide is inhibited if the protein level of the CNR polypeptide is less than 70% of the protein level of the same CNR polypeptide in a plant that has not been genetically modified or mutagenized to inhibit the expression of that CNR polypeptide. In particular embodiments of the invention, the protein level of the CNR polypeptide in a modified plant according to the invention is less than 60%, less than 50%, less than 40%, less than 30%, less than 20%, less than 10%, less than 5%, or less than 2% of the protein level of the same CNR polypeptide in a plant that is not a mutant or that has not been genetically modified to inhibit the expression of that CNR polypeptide. The expression level of the CNR polypeptide may be measured directly, for example, by assaying for the level of CNR polypeptide expressed in the plant cell or plant, or indirectly, for example, by measuring the cell number regulator activity of the CNR polypeptide in the plant cell or plant, or by measuring the cell number in the plant. Methods for performing such assays are described elsewhere herein.

In other embodiments of the invention, the activity of the CNR polypeptides is reduced or eliminated by transforming a plant cell with an expression cassette comprising a polynucleotide encoding a polypeptide that inhibits the activity of a CNR polypeptide. The cell number regulator activity of a CNR polypeptide is inhibited according to the present invention if the cell number regulator activity of the CNR polypeptide is less than 70% of the cell number regulator activity of the same CNR polypeptide in a plant that has not been modified to inhibit the cell number regulator activity of that CNR polypeptide. In particular embodiments of the invention, the cell number regulator activity of the CNR polypeptide in a modified plant according to the invention is less than 60%, less than 50%, less than 40%, less than 30%, less than 20%, less than 10%, or less than 5% of the cell number regulator activity of the same CNR polypeptide in a plant that that has not been modified to inhibit the expression of that CNR polypeptide. The cell number regulator activity of a CNR polypeptides is "eliminated" according to the invention when it is not detectable by the assay methods described elsewhere herein. Methods of determining the cell number regulator activity of a CNR polypeptide are described elsewhere herein.

In other embodiments, the activity of a CNR polypeptide may be reduced or eliminated by disrupting the gene encoding the CNR polypeptide. The invention encompasses mutagenized plants that carry mutations in CNR genes, where the mutations reduce expression of the CNR gene or inhibit the cell number regulator activity of the encoded CNR polypeptide.

Thus, many methods may be used to reduce or eliminate the activity of a CNR polypeptide. In addition, more than one method may be used to reduce the activity of a single CNR polypeptide. Non-limiting examples of methods of reducing or eliminating the expression of CNR polypeptides are given below.

### 1. Polynucleotide-Based Methods:

In some embodiments of the present invention, a plant is transformed with an expression cassette that is capable of expressing a polynucleotide that inhibits the expression of a CNR polypeptide of the invention. The term "expression" as used herein refers to the biosynthesis of a gene product, including the transcription and/or translation of said gene product. For example, for the purposes of the present invention, an expression cassette capable of expressing a polynucleotide that inhibits the expression of at least one CNR polypeptide is an expression cassette capable of producing an RNA molecule that inhibits the transcription and/or translation of at least one CNR polypeptide of the invention. The "expression" or "production" of a protein or polypeptide from a DNA molecule refers to the transcription and translation of the coding sequence to produce the protein or polypeptide, while the "expression" or "production" of a protein or polypeptide from an RNA molecule refers to the translation of the RNA coding sequence to produce the protein or polypeptide.

Examples of polynucleotides that inhibit the expression of a CNR polypeptide are given below.

### i. Sense Suppression/Cosuppression

In some embodiments of the invention, inhibition of the expression of a CNR polypeptide may be obtained by sense suppression or cosuppression. For cosuppression, an expression cassette is designed to express an RNA molecule corresponding to all or part of a messenger RNA encoding a CNR polypeptide in the "sense" orientation. Over expression of the RNA molecule can result in reduced expression of the native gene. Accordingly, multiple plant lines transformed with the cosuppression expression cassette are screened to identify those that show the greatest inhibition of CNR polypeptide expression.

The polynucleotide used for cosuppression may correspond to all or part of the sequence encoding the CNR polypeptides, all or part of the 5' and/or 3' untranslated region of a CNR polypeptide transcript, or all or part of both the coding sequence and the untranslated regions of a transcript encoding a CNR polypeptide. In some embodiments where the polynucleotide comprises all or part of the coding region for the CNR polypeptide, the expression cassette is designed to eliminate the start codon of the polynucleotide so that no protein product will be translated.

Cosuppression may be used to inhibit the expression of plant genes to produce plants having undetectable protein levels for the proteins encoded by these genes. See, for example, Broin et al. (2002) Plant Cell 14:1417-1432. Cosuppression may also be used to inhibit the expression of multiple proteins in the same plant. See, for example, U.S. Patent No. 5,942,657. Methods for using cosuppression to inhibit the expression of endogenous genes in plants are described in Flavell et al. (1994) Proc. Natl. Acad. Sci. USA 91:3490-3496; Jorgensen et al. (1996) Plant Mol. Biol. 31:957-973; Johansen and Carrington (2001) Plant Physiol. 126:930-938; Broin et al. (2002) Plant Cell 14:1417-1432; Stoutjesdijk et al. (2002) Plant Physiol. 129:1723-1731; Yu et al. (2003) Phytochemistry 63:753-763; and U.S. Patent Nos. 5,034,323, 5,283,184, and 5,942,657. The efficiency of cosuppression may be increased by including a poly-dT region in the expression cassette at a position 3' to the sense sequence and 5' of the polyadenylation signal. See, U.S. Patent Publication No. 20020048814. Typically, such a nucleotide sequence has substantial sequence identity to the sequence of the transcript of the endogenous gene, optimally greater than about 65% sequence identity, more optimally greater than about 85% sequence identity, most optimally greater than about 95% sequence identity. See U.S. Patent Nos. 5,283,184 and 5,034,323.

### ii. Antisense Suppression

In some embodiments of the invention, inhibition of the expression of the CNR polypeptide may be obtained by antisense suppression. For antisense suppression, the expression cassette is designed to express an RNA molecule complementary to all or part of a messenger RNA encoding the CNR polypeptide. Over expression of the antisense RNA molecule can result in reduced expression of the native gene. Accordingly, multiple plant lines transformed with the antisense suppression expression cassette are screened to identify those that show the greatest inhibition of CNR polypeptide expression.

The polynucleotide for use in antisense suppression may correspond to all or part of the complement of the sequence encoding the CNR polypeptide, all or part of the complement of the 5' and/or 3' untranslated region of the CNR transcript, or all or part of the complement of both the coding sequence and the untranslated regions of a transcript encoding the CNR polypeptide. In addition, the antisense polynucleotide may be fully complementary (i.e., 100% identical to the complement of the target sequence) or partially complementary (i.e., less than 100% identical to the complement of the target sequence) to the target sequence. Antisense suppression may be used to inhibit the expression of multiple proteins in the same plant. See, for example, U.S. Patent No. 5,942,657. Furthermore, portions of the antisense nucleotides may be used to disrupt the expression of the target gene. Generally, sequences of at least 50 nucleotides, 100 nucleotide, 200 nucleotides, 300, 400, 450, 500, 550, or greater may be used. Methods for using antisense suppression to inhibit the expression of endogenous genes in plants are described, for example, in Liu et al. (2002) Plant Physiol. 129:1732-1743 and U.S. Patent Nos. 5,759,829 and 5,942,657. Efficiency of antisense suppression may be increased by including a poly-dT region in the expression cassette at a position 3' to the antisense sequence and 5' of the polyadenylation signal. See, U.S. Patent Publication No. 20020048814.

### iii. Double-Stranded RNA Interference

In some embodiments of the invention, inhibition of the expression of a CNR polypeptide may be obtained by double-stranded RNA (dsRNA) interference. For dsRNA interference, a sense RNA molecule like that described above for cosuppression and an antisense RNA molecule that is fully or partially complementary to the sense RNA molecule are expressed in the same cell, resulting in inhibition of the expression of the corresponding endogenous messenger RNA.

Expression of the sense and antisense molecules can be accomplished by designing the expression cassette to comprise both a sense sequence and an antisense sequence. Alternatively, separate expression cassettes may be used for the sense and antisense sequences. Multiple plant lines transformed with the dsRNA interference expression cassette or expression cassettes are then screened to identify plant lines that show the greatest inhibition of CNR polypeptide expression. Methods for using dsRNA interference to inhibit the expression of endogenous plant genes are described in Waterhouse et al. (1998) Proc. Natl. Acad. Sci. USA 95:13959-13964, Liu et al. (2002) Plant Physiol. 129:1732-1743, and WO 99/49029, WO 99/53050, WO 99/61631, and WO 00/49035.

### iv. Hairpin RNA Interference and Intron-Containing Hairpin RNA Interference

In some embodiments of the invention, inhibition of the expression of a CNR polypeptide may be obtained by hairpin RNA (hpRNA) interference or intron-containing hairpin RNA (ihpRNA) interference. These methods are highly efficient at inhibiting the expression of endogenous genes. See, Waterhouse and Helliwell (2003) Nat. Rev. Genet. 4:29-38.

For hpRNA interference, the expression cassette is designed to express an RNA molecule that hybridizes with itself to form a hairpin structure that comprises a single-stranded loop region and a base-paired stem. The base-paired stem region comprises a sense sequence corresponding to all or part of the endogenous messenger RNA encoding the gene whose expression is to be inhibited, and an antisense sequence that is fully or partially complementary to the sense sequence. Alternatively, the base-paired stem region may correspond to a portion of a promoter sequence controlling expression of the gene to be inhibited. Thus, the base-paired stem region of the molecule generally determines the specificity of the RNA interference. hpRNA molecules are highly efficient at inhibiting the expression of endogenous genes, and the RNA interference they induce is inherited by subsequent generations of plants. See, for example, Chuang and Meyerowitz (2000) Proc. Natl. Acad. Sci. USA 97:4985-4990; Stoutjesdijk et al. (2002) Plant Physiol. 129:1723-1731; and Waterhouse and Helliwell (2003) Nat. Rev. Genet. 4:29-38. Methods for using hpRNA interference to inhibit or silence the expression of genes are described, for example, in Chuang and Meyerowitz (2000) Proc. Natl. Acad. Sci. USA 97:4985-4990; Stoutjesdijk et a/. (2002) Plant Physiol. 129:1723-1731; Waterhouse and Helliwell (2003) Nat. Rev. Genet. 4:29-38; Pandolfini *et a*/. *BMC Biotechnology* 3:7, and U.S. Patent Publication No. 20030175965. A transient assay for the efficiency of hpRNA constructs to silence gene expression *in vivo* has been described by Panstruga et al. (2003) Mol. Biol. Rep. 30:135-140.

For ihpRNA, the interfering molecules have the same general structure as for hpRNA, but the RNA molecule additionally comprises an intron that is capable of being spliced in the cell in which the ihpRNA is expressed. The use of an intron minimizes the size of the loop in the hairpin RNA molecule following splicing, and this increases the efficiency of interference. See, for example, Smith et al. (2000) Nature 407:319-320. In fact, Smith *et al.* show 100% suppression of endogenous gene expression using ihpRNA-mediated interference. Methods for using ihpRNA interference to inhibit the expression of endogenous plant genes are described, for example, in Smith et al. (2000) Nature 407:319-320; Wesley et al. (2001) Plant J. 27:581-590; Wang and Waterhouse (2001) Curr. Opin. Plant Biol. 5:146-150; Waterhouse and Helliwell (2003) Nat. Rev. Genet. 4:29-38; Helliwell and Waterhouse (2003) Methods 30:289-295, and U.S. Patent Publication No. 20030180945.

The expression cassette for hpRNA interference may also be designed such that the sense sequence and the antisense sequence do not correspond to an endogenous RNA. In this embodiment, the sense and antisense sequence flank a loop sequence that comprises a nucleotide sequence corresponding to all or part of the endogenous messenger RNA of the target gene. Thus, it is the loop region that determines the specificity of the RNA interference. See, for example, WO 02/00904, Mette et al. (2000) EMBO J 19:5194-5201; Matzke et al. (2001) Curr. Opin. Genet. Devel. 11:221-227; Scheid et al. (2002) Proc. Natl. Acad. Sci., USA 99:13659-13662; Aufsaftz et al (2002) Proc. Nat'l. Acad. Sci. 99(4):16499-16506; Sijen et al., Curr. Biol. (2001) 11:436-440).

### v. Amplicon-Mediated Interference

Amplicon expression cassettes comprise a plant virus-derived sequence that contains all or part of the target gene but generally not all of the genes of the native virus. The viral sequences present in the transcription product of the expression cassette allow the transcription product to direct its own replication. The transcripts produced by the amplicon may be either sense or antisense relative to the target sequence (i.e., the messenger RNA for the CNR polypeptide). Methods of using amplicons to inhibit the expression of endogenous plant genes are described, for example, in Angell and Baulcombe (1997) EMBO J. 16:3675-3684, Angell and Baulcombe (1999) Plant J. 20:357-362, and U.S. Patent No. 6,646,805.

### vi. Ribozymes

in some embodiments, the polynucleotide expressed by the expression cassette of the invention is catalytic RNA or has ribozyme activity specific for the messenger RNA of the CNR polypeptide. Thus, the polynucleotide causes the degradation of the endogenous messenger RNA, resulting in reduced expression of the CNR polypeptide. This method is described, for example, in U.S. Patent No. 4,987,071.

### vii. Small Interfering RNA or Micro RNA

In some embodiments of the invention, inhibition of the expression of a CNR polypeptide may be obtained by RNA interference by expression of a gene encoding a micro RNA (miRNA). miRNAs are regulatory agents consisting of about 22 ribonucleotides. miRNA are highly efficient at inhibiting the expression of endogenous genes. See, for example Javier et al. (2003) Nature 425: 257-263.

For miRNA interference, the expression cassette is designed to express an RNA molecule that is modeled on an endogenous miRNA gene. The miRNA gene encodes an RNA that forms a hairpin structure containing a 22-nucleotide sequence that is complementary to another endogenous gene (target sequence). For suppression of CNR expression, the 22-nucleotide sequence is selected from a CNR transcript sequence and contains 22 nucleotides of said CNR sequence in sense orientation and 21 nucleotides of a corresponding antisense sequence that is complementary to the sense sequence. miRNA molecules are highly efficient at inhibiting the expression of endogenous genes, and the RNA interference they induce is inherited by subsequent generations of plants.

### 2. Polypeptide-Based Inhibition of Gene Expression

In one embodiment, the polynucleotide encodes a zinc finger protein that binds to a gene encoding a CNR polypeptide, resulting in reduced expression of the gene. In particular embodiments, the zinc finger protein binds to a regulatory region of a CNR gene. In other embodiments, the zinc finger protein binds to a messenger RNA encoding a CNR polypeptide and prevents its translation. Methods of selecting sites for targeting by zinc finger proteins have been described, for example, in U.S. Patent No. 6,453,242, and methods for using zinc finger proteins to inhibit the expression of genes in plants are described, for example, in U.S. Patent Publication No. 20030037355.

### 3. Polypeptide-Based Inhibition of Protein Activity

In some embodiments of the invention, the polynucleotide encodes an antibody that binds to at least one CNR polypeptide, and reduces the cell number regulator activity of the CNR polypeptide. In another embodiment, the binding of the antibody results in increased turnover of the antibody-CNR complex by cellular quality control mechanisms. The expression of antibodies in plant cells and the inhibition of molecular pathways by expression and binding of antibodies to proteins in plant cells are well known in the art. See, for example, Conrad and Sonnewald (2003) Nature Biotech. 21:35-36.

### 4. Gene Disruption

In some embodiments of the present invention, the activity of a CNR polypeptide is reduced or eliminated by disrupting the gene encoding the CNR polypeptide. The gene encoding the CNR polypeptide may be disrupted by any method known in the art. For example, in one embodiment, the gene is disrupted by transposon tagging. In another embodiment, the gene is disrupted by mutagenizing plants using random or targeted mutagenesis, and selecting for plants that have reduced cell number regulator activity.

### i. Transposon Tagging

In one embodiment of the invention, transposon tagging is used to reduce or eliminate the CNR activity of one or more CNR polypeptide. Transposon tagging comprises inserting a transposon within an endogenous CNR gene to reduce or eliminate expression of the CNR polypeptide. "CNR gene" is Intended to mean the gene that encodes a CNR polypeptide.

In this embodiment, the expression of one or more CNR polypeptide is reduced or eliminated by inserting a transposon within a regulatory region or coding region of the gene encoding the CNR polypeptide. A transposon that is within an exon, intron, 5' or 3' untranslated sequence, a promoter, or any other regulatory sequence of a CNR gene may be used to reduce or eliminate the expression and/or activity of the encoded CNR polypeptide.

Methods for the transposon tagging of specific genes in plants are well known in the art. See, for example, Maes et al. (1999) Trends Plant Sci. 4:90-96; Dharmapuri and Sonti (1999) FEMS Microbio/. Lett. 179:53-59; Meissner et al. (2000) Plant J. 22:265-274; Phogat et al. (2000) J. Biosci. 25:57-63; Walbot (2000) Curr. Opin. Plant Bio/. 2:103-107; Gai et al. (2000) Nucleic Acids Res. 28:94-96; Fitzmaurice et al. (1999) Genetics 153:1919-1928). In addition, the TUSC process for selecting Mu insertions in selected genes has been described in Bensen et al. (1995) Plant Cell 7:75-84; Mena et al. (1996) Science 274:1537-1540; and U.S. Patent No. 5,962,764.

### ii. Mutant Plants with Reduced Activity

Additional methods for decreasing or eliminating the expression of endogenous genes in plants are also known in the art and can be similarly applied to the instant invention. These methods include other forms of mutagenesis, such as ethyl methanesulfonate-induced mutagenesis, deletion mutagenesis, and fast neutron deletion mutagenesis used in a reverse genetics sense (with PCR) to identify plant lines in which the endogenous gene has been deleted. For examples of these methods see Ohshima et al. (1998) Virology 243:472-481; Okubara et al. (1994) Genetics 137:867-874; and Quesada et a/. (2000) Genetics 154:421-436. In addition, a fast and automatable method for screening for chemically induced mutations, TILLING (Targeting Induced Local Lesions In Genomes), using denaturing HPLC or selective endonuclease digestion of selected PCR products is also applicable to the instant invention. See McCallum et al. (2000) Nat. Biotechno/. 18:455-457.

Mutations that impact gene expression or that interfere with the function (cell number regulator activity) of the encoded protein are well known in the art. Insertional mutations in gene exons usually result in null-mutants. Mutations in conserved residues are particularly effective in inhibiting the cell number regulator activity of the encoded protein. Conserved residues of plant CNR polypeptides suitable for mutagenesis with the goal to eliminate cell number regulator activity have been described. Such mutants can be isolated according to well-known procedures, and mutations in different CNR loci can be stacked by genetic crossing. See, for example, Gruis et al. (2002) Plant Cell 14:2863-2882.

In another embodiment of this invention, dominant mutants can be used to trigger RNA silencing due to gene inversion and recombination of a duplicated gene locus. See, for example, Kusaba et al. (2003) Plant Cell 15:1455-1467.

The invention encompasses additional methods for reducing or eliminating the activity of one or more CNR polypeptide. Examples of other methods for altering or mutating a genomic nucleotide sequence in a plant are known in the art and include, but are not limited to, the use of RNA:DNA vectors, RNA:DNA mutational vectors, RNA:DNA repair vectors, mixed-duplex oligonucleotides, self-complementary RNA:DNA oligonucleotides, and recombinogenic oligonucleobases. Such vectors and methods of use are known in the art. See, for example, U.S. Patent Nos. 5,565,350; 5,731,181; 5,756,325; 5,760,012; 5,795,972; and 5,871,984. See also, WO 98/49350, WO 99/07865, WO 99/25821, and Beetham et al. (1999) Proc. Natl. Acad. Sci. USA 96:8774-8778.

### iii. Modulating Cell number regulator activity

In specific methods, the level and/or activity of a cell number regulator in a plant is decreased by increasing the level or activity of the CNR polypeptide in the plant. Methods for increasing the level and/or activity of CNR polypeptides in a plant are discussed elsewhere herein. Briefly, such methods comprise providing a CNR polypeptide of the invention to a plant and thereby increasing the level and/or activity of the CNR polypeptide. In other embodiments, a CNR nucleotide sequence encoding a CNR polypeptide can be provided by introducing into the plant a polynucleotide comprising a CNR nucleotide sequence, expressing the CNR sequence, increasing the activity of the CNR polypeptide, and thereby decreasing the number of tissue cells in the plant or plant part. In other embodiments, the CNR nucleotide construct introduced into the plant is stably incorporated into the genome of the plant.

In other methods, the number of cells in a plant tissue is increased by decreasing the level and/or activity of the CNR polypeptide in the plant Such methods are disclosed in detail elsewhere herein. In one such method, a CNR nucleotide sequence is introduced into the plant and expression of said CNR nucleotide sequence decreases the activity of the CNR polypeptide, and thereby increasing the cell number in the plant or plant part. In other embodiments, the CNR nucleotide construct introduced into the plant is stably incorporated into the genome of the plant

As discussed above, one of skill will recognize the appropriate promoter to use to modulate the level/activity of a cell number regulator in the plant. Exemplary promoters for this embodiment have been disclosed elsewhere herein.

Accordingly, the present invention further provides plants having a modified number of cells when compared to the number of cells of a control plant tissue. In one embodiment, the plant of the invention has an increased level/activity of the CNR polypeptide of the invention and thus has a decreased number of cells in the plant tissue. In other embodiments, the plant of the invention has a reduced or eliminated level of the CNR polypeptide of the invention and thus has an increased number of cells in the plant tissue. In other embodiments, such plants have stably incorporated into their genome a nucleic acid molecule comprising a CNR nucleotide sequence of the invention operably linked to a promoter that drives expression in the plant cell.

### iv. Modulating Root Development

Methods for modulating root development in a plant are provided. By "modulating root development" is intended any alteration in the development of the plant root when compared to a control plant. Such alterations in root development include, but are not limited to, alterations in the growth rate of the primary root, the fresh root weight, the extent of lateral and adventitious root formation, the vasculature system, meristem development, or radial expansion.

Methods for modulating root development in a plant are provided. The methods comprise modulating the level and/or activity of the CNR polypeptide in the plant In one method, a CNR sequence is provided to the plant In another method, the CNR nucleotide sequence is provided by introducing into the plant a polynucleotide comprising a CNR nucleotide sequence, expressing the CNR sequence, and thereby modifying root development. In still other methods, the CNR nucleotide construct introduced into the plant is stably incorporated into the genome of the plant.

In other methods, root development is modulated by altering the level or activity of the CNR polypeptide in the plant A decrease in CNR activity can result in at least one or more of the following alterations to root development, including, but not limited to, larger root meristems, increased in root growth, enhanced radial expansion, an enhanced vasculature system, increased root branching, more adventitious roots, and/or an increase in fresh root weight when compared to a control plant.

As used herein, "root growth" encompasses all aspects of growth of the different parts that make up the root system at different stages of its development in both monocotyledonous and dicotyledonous plants. It is to be understood that enhanced root growth can result from enhanced growth of one or more of its parts including the primary root, lateral roots, adventitious roots, etc.

Methods of measuring such developmental alterations in the root system are known in the art. See, for example, U.S. Application No. 2003/0074698 and Wemer et al. (2001) PNAS 18:10487-10492.

As discussed above, one of skill will recognize the appropriate promoter to use to modulate root development in the plant. Exemplary promoters for this embodiment include constitutive promoters and root-preferred promoters. Exemplary root-preferred promoters have been disclosed elsewhere herein.

Stimulating root growth and increasing root mass by decreasing the activity and/or level of the CNR polypeptide also finds use in improving the standability of a plant. The term "resistance to lodging" or "standability" refers to the ability of a plant to fix itself to the soil. For plants with an erect or semi-erect growth habit, this term also refers to the ability to maintain an upright position under adverse (environmental) conditions. This trait relates to the size, depth and morphology of the root system. In addition, stimulating root growth and increasing root mass by decreasing the level and/or activity of the CNR polypeptide also finds use in promoting in vitro propagation of explants.

Furthermore, higher root biomass production due to an decreased level and/or activity of CNR activity has a direct effect on the yield and an indirect effect of production of compounds produced by root cells or transgenic root cells or cell cultures of said transgenic root cells. One example of an interesting compound produced in root cultures is shikonin, the yield of which can be advantageously enhanced by said methods.

Accordingly, the present invention further provides plants having modulated root development when compared to the root development of a control plant. In some embodiments, the plant has an increased level/activity of the CNR polypeptide and has enhanced root growth and/or root biomass. In other embodiments, such plants have stably incorporated into their genome a nucleic acid molecule comprising a CNR nucleotide sequence operably linked to a promoter that drives expression in the plant cell.

### v. Modulating Shoot and Leaf Development

Methods are also provided for modulating shoot and leaf development in a plant. By "modulating shoot and/or leaf development" is intended any alteration in the development of the plant shoot and/or leaf. Such alterations in shoot and/or leaf development include, but are not limited to, alterations in shoot meristem development, in leaf number, leaf size, leaf and stem vasculature, internode length, and leaf senescence. As used herein, "leaf development" and "shoot development" encompasses all aspects of growth of the different parts that make up the leaf system and the shoot system, respectively, at different stages of their development, both in monocotyledonous and dicotyledonous plants. Methods for measuring such developmental alterations in the shoot and leaf system are known in the art. See, for example, Wemer et al. (2001) PNAS 98:10487-10492 and U.S. Application No. 2003/0014698.

The method for modulating shoot and/or leaf development in a plant comprises modulating the activity and/or level of a CNR polypeptide. In one embodiment, a CNR sequence is provided. In other embodiments, the CNR nucleotide sequence can be provided by introducing into the plant a polynucleotide comprising a CNR nucleotide sequence, expressing the CNR sequence, and thereby modifying shoot and/or leaf development. In other embodiments, the CNR nucleotide construct introduced into the plant is stably incorporated into the genome of the plant.

In specific embodiments, shoot or leaf development is modulated by increasing the level and/or activity of the CNR polypeptide in the plant. An increase in CNR activity can result in at least one or more of the following alterations in shoot and/or leaf development, including, but not limited to, reduced leaf number, reduced leaf surface, reduced vascular, shorter internodes and stunted growth, and retarded leaf senescence, when compared to a control plant.

As discussed above, one of skill will recognize the appropriate promoter to use to modulate shoot and leaf development of the plant. Exemplary promoters for this embodiment include constitutive promoters, shoot-preferred promoters, shoot meristem-preferred promoters, and leaf-preferred promoters. Exemplary promoters have been disclosed elsewhere herein.

Increasing CNR activity and/or level in a plant results in shorter internodes and stunted growth. Thus, the methods of the invention find use in producing dwarf plants. In addition, as discussed above, modulation CNR activity in the plant modulates both root and shoot growth. Thus, the present invention further provides methods for altering the root/shoot ratio. Shoot or leaf development can further be modulated by decreasing the level and/or activity of the CNR polypeptide in the plant.

### vi Modulating Reproductive Tissue Development

Methods for modulating reproductive tissue development are provided. In one embodiment, methods are provided to modulate floral development in a plant. By "modulating floral development" is intended any alteration in a structure of a plant's reproductive tissue as compared to a control plant in which the activity or level of the CNR polypeptide has not been modulated. "Modulating floral development" further includes any alteration in the timing of the development of a plant's reproductive tissue (i.e., a delayed or a accelerated timing of floral development) when compared to a control plant in which the activity or level of the CNR polypeptide has not been modulated. Macroscopic alterations may include changes in size, shape, number, or location of reproductive organs, the developmental time period that these structures form, or the ability to maintain or proceed through the flowering process in times of environmental stress. Microscopic alterations may include changes to the types or shapes of cells that make up the reproductive organs.

The method for modulating floral development in a plant comprises modulating CNR activity in a plant. In one method, a CNR sequence is provided. A CNR nucleotide sequence can be provided by introducing into the plant a polynucleotide comprising a CNR nucleotide sequence, expressing the CNR sequence, and thereby modifying floral development. In other embodiments, the CNR nucleotide construct introduced into the plant is stably incorporated into the genome of the plant

In specific methods, floral development is modulated by increasing the level or activity of the CNR polypeptide in the plant. An increase in CNR activity can result in at least one or more of the following alterations in floral development, including, but not limited to, retarded flowering, reduced number of flowers, partial male sterility, and reduced seed set, when compared to a control plant. Inducing delayed flowering or inhibiting flowering can be used to enhance yield in forage crops such as alfalfa. Methods for measuring such developmental alterations in floral development are known in the art. See, for example, Mouradov et al. (2002) The Plant Cell S111-S130.

As discussed above, one of skill will recognize the appropriate promoter to use to modulate floral development of the plant Exemplary promoters for this embodiment include constitutive promoters, inducible promoters, shoot-preferred promoters, and inflorescence-preferred promoters.

In other methods, floral development is modulated by decreasing the level and/or activity of the CNR sequence. Such methods can comprise introducing a CNR nucleotide sequence into the plant and decreasing the activity of the CNR polypeptide. In other methods, the CNR nucleotide construct introduced into the plant is stably incorporated into the genome of the plant Decreasing expression of the CNR sequence can modulate floral development during periods of stress. Such methods are described elsewhere herein.

Methods are also provided for the use of the CNR sequences to increase seed size and/or weight The method comprises increasing the activity of the CNR sequences in a plant or plant part, such as the seed. An increase in seed size and/or weight comprises an increased size or weight of the seed and/or an increase in the size or weight of one or more seed part including, for example, the embryo, endosperm, seed coat, aleurone, or cotyledon.

As discussed above, one of skill will recognize the appropriate promoter to use to increase seed size and/or seed weight. Exemplary promoters of this embodiment include constitutive promoters, inducible promoters, seed-preferred promoters, embryo-preferred promoters, and endosperm-preferred promoters.

The method for decreasing seed size and/or seed weight in a plant comprises increasing CNR activity in the plant. In one embodiment, the CNR nucleotide sequence can be provided by introducing into the plant a polynucleotide comprising a CNR nucleotide sequence, expressing the CNR sequence, and thereby decreasing seed weight and/or size. In other embodiments, the CNR nucleotide construct introduced into the plant is stably incorporated into the genome of the plant.

It is further recognized that increasing seed size and/or weight can also be accompanied by an increase in the speed of growth of seedlings or an increase in early vigor. As used herein, the term "early vigor" refers to the ability of a plant to grow rapidly during early development, and relates to the successful establishment, after germination, of a well-developed root system and a well-developed photosynthetic apparatus. In addition, an increase in seed size and/or weight can also result in an increase in plant yield when compared to a control.

### EXAMPLES

### Example 1. Isolation of CNR sequences

A routine for identifying all members of a gene family was employed to search for the CNR genes of interest A diverse set of all the known members of the gene family as protein sequences was prepared. This data includes sequences from other species. These species are searched against a proprietary maize sequence dataset and a nonredundant set of overlapping hits is identified. Separately, one takes the nucleotide sequences of any genes of interest in hand and searches against the database and a nonredundant set of all overlapping hits are retrieved. The set of protein hits are then compared to the nucleotide hits. If the gene family is complete, all of the protein hits are contained within the nucleotide hits. The CNR family of genes consists of 12 members.

### Example 2. CNR Sequence Analysis

The CNR polypeptides of the current invention have common characteristics with tomato fw2.2 polypeptide (SEQ ID NO: 45). The relationship between the genes of the invention and the tomato fw2.2 is shown in Table 2.

**Table 2**

| **Gene** | **% Identity to Fw2-2** | **%Similarity to Fw2-2** |
|---|---|---|
| ZmCNR02 | 53.59 | 62.98 |
| ZmCNR10 | 49.69 | 62.58 |
| ZmCNR09 | 47.43 | 58.29 |
| ZmCNR03 | 46.71 | 59.28 |
| ZmCNR01 | 46.6 | 57.59 |
| ZmCNR07 | 46.11 | 58.89 |
| ZmCNR04 | 41.1 | 55.83 |
| ZmCNR11 | 41.1 | 55.21 |
| ZmCNR12 | 36.2 | 49.08 |
| ZmCNR05 | 33.7 | 48.91 |
| ZmCNR08 | 27.9 | 35.19 |
| ZmCNR06 | 21.34 | 30.54 |

In addition, a clustal dendrogram and alignment of the Tomato fw2.2 sequence (SEQ ID NO: 2) with the 12 maize gene translations is provided in Figures 1 and 2 respectively.

### Example 3. CNR Expression Patterns in Maize using MPSS

MPSS stands for Massively Parallel Signature Sequencing, a technique invented and commercialized by Lynx Therapeutics, Inc. of Hayward, California. MPSS and related technologies have been described in publications by Brenner et al. (Nature Biotechnol. [2000] 18:630-634, and PNAS [2000] 97:1665-1670). Like SAGE (Serial Analysis of Gene Expression), MPSS produces short sequence signatures produced from a defined position within an mRNA, and the relative abundance of these signatures in a given library represents a quantitative estimate of expression of that gene. The MPSS signatures are 17 bp in length, and can uniquely identify >95% of all genes in Arabidopsis.

The CNR sequences were matched to MPSS data, and matching tags (GATC-17mers) were curated. Ideally, the correct tag for a gene is in the plus strand proximal to but just up from the poly A tail, and it is gene specific. Where more than one tag matches a gene, one will usually choose the one closest to the poly A tail, which is also usually the one with the highest gene expression. Where the tag matches more than one gene, the correct gene association is usually the one that has an EST distribution that best corresponds to the expression pattern revealed by the MPSS data.
Expression of the various CNR sequences revealed that:
ZmCNR 1 was weakly expressed in various tissues - most consistently in stalk and tassel spikelets
ZmCNR 2 was expressed more strongly in various tissues, and appears to be silk preferred
ZmCNR 3 is the only member of the group which demonstrated strong pollen preferred expression
ZmCNR 4 expression was not detectable
ZmCNR 5 was weakly expressed in various tissues
ZmCNR 6 was strongly expressed in many tissues - but not pollen - expressed rather abundantly in seed tissues
ZmCNR 7 and 9 were expressed in various tissues, but were silk preferred
ZmCNR 8 was expressed moderately in various tissues, with a bias toward tassel spikelets.

### Specific tissue expression data relating to ZmCNR02

Endosperm Development - The pattern of ZmCNR02 gene expression as revealed by MPSS data reveals that the gene expression is very low in the early stages of endosperm development (in early days after pollination - DAP), but that as the endosperm matures (higher DAP), the expression of ZmCNR02 increases as illustrated in Figure 3. Thus this pattern of expression in endosperm is consistent with a role of ZmCNR02 in negatively regulating cell number.

Embryo Development - The seed embryo development is scored in terms of days after pollination (DAP). The pattern of ZmCNR02 expression rises towards the end of embryo development after 30 DAP, with the highest expression at 45 DAP, see Figure 4A. This corresponds to the period of completion of cell number growth, this pattern of expression is consistent with a role for ZmCNR02 as a negative cell number regulator.

Ovule Development - Figure 4B illustrates results from 35 cycles of RT-PCR performed with different maize tissues, including endosperm (14 DAP), shoot apical meristem, pericarp, seedling, root, brace root, mature and immature leaf, immature ear, immature tassel, node, and ovule. Consistent with the Lynx MPSS profiling data, the expression of this gene is detected mostly in the tissue where there is little growth activity, such as mature leaf. Interestingly, a very high expression is detected in the ovule tissue. The ovule (pre-fertilization) has no cell division activity and is at a rest stage. ZmCNR02 is expressed at a very high level in the ovule, comparable to the level in the mature leaf tissue. However, immediately after fertilization when active cell division begins, the ZmCNR02 expression is dramatically reduced to a minimal level, as shown in the early embryo and endosperm development (See expression demonstrated in Figures 3 and 4A).

Leaf Development - Several samples were assayed in relation to developing maize leaves as shown in Figure 5. The basal region of immature leaves, the region of most active cell division, showed no ZmCNR02 expression. The distal expanding and expanded portions of the same immature leaves showed a small but noticeable ZmCNR02 expression. A series of whole leaves from young plants (V2) to middle stage leaves (V7-V8) to mature leaves, showed progressively higher ZmCNR02 expression. This expression pattern is consistent with ZmCNR02 being related to negative control of cell number, its expression is highest in leaf stages that are undergoing little cell division.

Carpels, Silk Development, and Pollen - The silks, ovary walls and pericarp are analogous to the dicot flower carpel. ZmCNR02 expression is detected in the latter two. The ZmCNR02 expression is in the maize 'carpels' by virtue of the silk and pericarp expression. The pericarp samples assayed are fairly late in development and are compromised by remaining endosperm tissue. The silk tissues are fairly easy to gather and assay for gene expression.In the young growing silks (those still attached to the ovaries) the expression of ZmCNR02 expression is not detected. Then moving through a series of pre-emergent to post emergent silks, and thence through a post pollination series, the expression of ZmCNR02 increases. For comparison the pollen sample is offered indicating that the increase of ZmCNR02 expression is not derived from the pollen landing on the silks. As silks mature, and especially after they are pollinated, the cell division slows and stops. The pattern of ZmCNR02 expression in silks (a carpel tissue) is consistent with a negative cell number regulator, see Figure 6.

Root and Root Meristems - A comparison of whole roots (with meristems) to root tips (meristem enriched), as presented in Figure 7, shows that ZmCNR02 expression is higher in whole roots than root tips. The ZmCNR02 expression having higher expression in areas of the root not actively dividing, and the expression pattern in roots is consistent with as a negative regulator of cell number (division).

Cytokinin Treatment - Data from an experiment showing that the ZmCNR02 genes' expression, as revealed by MPSS transcript assay, decreases in excised maize leaf discs, when 10 micromolar benzyladenine is added for 6 hours and is shown in Figure 8. The experimental and control samples used:
Corn leaf disc, Ctrl - [leaf discs from ear leaf (cerca L9) of R1 plants, discs 5 mm diameter. Cultured 6 hours at 25C.]
Corn leaf disc, +BA - [leaf discs from ear leaf (cerca L9) of R1 plants, discs 5 mm diameter. Cultured 6 hours at 25C in 10 microMol Benzyladenine]

This result offers additional evidence that the expression of ZmCNR02 is consistent with a role in negatively regulating cell number. The addition of a plant hormone that is known to induce cell number (cell division) results in the *DECUNE* in expression of ZmCNR02, as expected per the hypothesis that this gene negatively regulates cell number.

### Example 4. Transformation and Regeneration of Transgenic Plants

Immature maize embryos from greenhouse donor plants are bombarded with a plasmid containing the CNR sequence operably linked to the drought-inducible promoter RAB17 promoter (Vilardell et al. (1990) Plant Mol Biol 14:423-432) and the selectable marker gene PAT, which confers resistance to the herbicide Bialaphos. Alternatively, the selectable marker gene is provided on a separate plasmid. Transformation is performed as follows. Media recipes follow below.

### Preparation of Target Tissue:

The ears are husked and surface sterilized in 30% Clorox bleach plus 0.5% Micro detergent for 20 minutes, and rinsed two times with sterile water. The immature embryos are excised and placed embryo axis side down (scutellum side up), 25 embryos per plate, on 560Y medium for 4 hours and then aligned within the 2.5-cm target zone in preparation for bombardment.

### Preparation of DNA:

A plasmid vector comprising the CNR sequence operably linked to an ubiquitin promoter is made. This plasmid DNA plus plasmid DNA containing a PAT selectable marker is precipitated onto 1.1 µm (average diameter) tungsten pellets using a CaCl₂ precipitation procedure as follows:
100 µl prepared tungsten particles in water
10 µl (1 µg) DNA in Tris EDTA buffer (1 µg total DNA)
100 µl 2.5 M CaC1₂
10 µl 0.1 M spermidine

Each reagent is added sequentially to the tungsten particle suspension, while maintained on the multitube vortexer. The final mixture is sonicated briefly and allowed to incubate under constant vortexing for 10 minutes. After the precipitation period, the tubes are centrifuged briefly, liquid removed, washed with 500 ml 100% ethanol, and centrifuged for 30 seconds. Again the liquid is removed, and 105 µl 100% ethanol is added to the final tungsten particle pellet. For particle gun bombardment, the tungsten/DNA particles are briefly sonicated and 10 µl spotted onto the center of each macrocarrier and allowed to dry about 2 minutes before bombardment.

### Particle Gun Treatment:

The sample plates are bombarded at level #4 in particle gun #HE34-1 or #HE34-2. All samples receive a single shot at 650 PSI, with a total of ten aliquots taken from each tube of prepared particles/DNA.

### Subsequent Treatment:

Following bombardment, the embryos are kept on 560Y medium for 2 days, then transferred to 560R selection medium containing 3 mg/liter Bialaphos, and subcultured every 2 weeks. After approximately 10 weeks of selection, selection-resistant callus clones are transferred to 288J medium to initiate plant regeneration. Following somatic embryo maturation (2-4 weeks), well-developed somatic embryos are transferred to medium for germination and transferred to the lighted culture room. Approximately 7-10 days later, developing plantlets are transferred to 272V hormone-free medium in tubes for 7-10 days until plantlets are well established. Plants are then transferred to inserts in flats (equivalent to 2.5" pot) containing potting soil and grown for 1 week in a growth chamber, subsequently grown an additional 1-2 weeks in the greenhouse, then transferred to classic 600 pots (1.6 gallon) and grown to maturity. Plants are monitored and scored for increased drought tolerance. Assays to measure improved drought tolerance are routine in the art and include, for example, increased kernel-earring capacity yields under drought conditions when compared to control maize plants under identical environmental conditions. Alternatively, the transformed plants can be monitored for a modulation in meristem development (i.e., a decrease in spikelet formation on the ear). See, for example, Bruce et al. (2002) Journal of Experimental Botany 53:1-13.

### Bombardment and Culture Media:

Bombardment medium (560Y) comprises 4.0 g/l N6 basal salts (SIGMA C-1416), 1.0 ml/l Eriksson's Vitamin Mix (1000X SIGMA-1511), 0.5 mg/l thiamine HCl, 120.0 g/l sucrose, 1.0 mg/l 2,4-D, and 2.88 g/l L-proline (brought to volume with D-I H₂O following adjustment to pH 5.8 with KOH); 2.0 g/l Gelrite (added after bringing to volume with D-I H₂O); and 8.5 mg/l silver nitrate (added after sterilizing the medium and cooling to room temperature). Selection medium (560R) comprises 4.0 g/l N6 basal salts (SIGMA C-1416), 1.0 ml/l Eriksson's Vitamin Mix (1000X SIGMA-1511), 0.5 mg/l thiamine HCl, 30.0 g/l sucrose, and 2.0 mg/l 2,4-D (brought to volume with D-I H₂O following adjustment to pH 5.8 with KOH); 3.0 g/l Gelrite (added after bringing to volume with D-I H₂O); and 0.85 mg/l silver nitrate and 3.0 mg/l bialaphos (both added after sterilizing the medium and cooling to room temperature).

Plant regeneration medium (288J) comprises 4.3 g/l MS salts (GIBCO 11117-074), 5.0 ml/l MS vitamins stock solution (0.100 g nicotinic acid, 0.02 g/l thiamine HCL, 0.10 g/l pyridoxine HCL, and 0.40 g/l glycine brought to volume with polished D-I H₂O) (Murashige and Skoog (1962) Physiol. Plant. 15:473), 100 mg/l myo-inositol, 0.5 mg/l zeatin, 60 g/l sucrose, and 1.0 ml/l of 0.1 mM abscisic acid (brought to volume with polished D-I H₂O after adjusting to pH 5.6); 3.0 g/l Gelrite (added after bringing to volume with D-I H₂O); and 1.0 mg/l indoleacetic acid and 3.0 mg/l bialaphos (added after sterilizing the medium and cooling to 60°C). Hormone-free medium (272V) comprises 4.3 g/l MS salts (GIBCO 11117-074), 5.0 ml/l MS vitamins stock solution (0.100 g/l nicotinic acid, 0.02 g/l thiamine HCL, 0.10 g/l pyridoxine HCL, and 0.40 g/l glycine brought to volume with polished D-I H₂O), 0.1 g/l myo-inositol, and 40.0 g/l sucrose (brought to volume with polished D-I H₂O after adjusting pH to 5.6); and 6 g/l bacto-agar (added after bringing to volume with polished D-I H₂O), sterilized and cooled to 60°C.

### Example 5. Agrobacterium-mediated Transformation

For *Agrobacterium*-mediated transformation of maize with an antisense sequence of the CNR sequence, preferably the method of Zhao is employed (U.S. Patent No. 5,981,840, and PCT patent publication WO98/32326). Briefly, immature embryos are isolated from maize and the embryos contacted with a suspension of *Agrobacterium,* where the bacteria are capable of transferring the antisense CNR sequences to at least one cell of at least one of the immature embryos (step 1: the infection step). In this step the immature embryos are preferably immersed in an *Agrobacterium* suspension for the initiation of inoculation. The embryos are co-cultured for a time with the *Agrobacterium* (step 2: the co-cultivation step). Preferably the immature embryos are cultured on solid medium following the infection step. Following this co-cultivation period an optional "resting" step is contemplated. In this resting step, the embryos are incubated in the presence of at least one antibiotic known to inhibit the growth of *Agrobacterium* without the addition of a selective agent for plant transformants (step 3: resting step). Preferably the immature embryos are cultured on solid medium with antibiotic, but without a selecting agent, for elimination of *Agrobacterium* and for a resting phase for the infected cells. Next, inoculated embryos are cultured on medium containing a selective agent and growing transformed callus is recovered (step 4: the selection step). Preferably, the immature embryos are cultured on solid medium with a selective agent resulting in the selective growth of transformed cells. The callus is then regenerated into plants (step 5: the regeneration step), and preferably calli grown on selective medium are cultured on solid medium to regenerate the plants. Plants are monitored and scored for a modulation in meristem development. For instance, alterations of size and appearance of the shoot and floral meristems and/or increased yields of leaves, flowers, and/or fruits.

### Reference Example 6. Analysis of ZmCNR02 expression in maize leaf tissue

### Collections of maize leaf section series by growth activity:

Leaf sections of different growth activity are collected from seedlings at V3 stage. The leaf blades of 1^{st}, 2^{nd}, 3^{rd} leaves that are fully opened are collected and pooled as the mature leaf tissue. The sheath of these 3 leaves are removed and discarded. The remaining whorl tissue (mostly leaf tissue) is then sectioned from the base to top as:
1. 0-6 mm: mostly cell dividing zone (including shoot apical meristem)
2. 6-20 mm: mostly cell expanding zone
3. 20 mm-tip: transition zone
4. Mature leaf: the fully opened leaf blades as described above with little growth activity
5. The whole seedling that has mixture of growing and mature leaf tissues

The growth activity of these tissues is in the order as 1>2>3>4. #5 is a mixture. Figure 9 shows the RT-PCR analysis of *ZmCNR02* multiplexing with tubulin as a control. There are two main points from this data:
1. The expression of *ZmCNR02* (shown as a ratio of *ZmCNR02*/tubulin) is negatively correlated with the growth activity, increasing from sample #1 to #4.
2. This trend is consistent seen in all four genotypes, including inbreds and their reciprocal hybrids B73, Mo17, B73/Mo17 and Mo17/B73.

The Figure 10 is a repeated RT-PCR assay with the mature leaf tissue, where we had to modify the PCR protocol to increase the amplification of tubulin that was out-competed by *ZmCNR02*'s high expression. The figure shows well that the expression level of *ZmCNR02* in both hybrids is significantly higher than the inbred parents, which is consistent with that hybrids grow faster and are more vigorous than inbreds.

### Example 7. Soybean Embryo Transformation

Soybean embryos are bombarded with a plasmid containing an antisense CNR sequences operably linked to an ubiquitin promoter as follows. To induce somatic embryos, cotyledons, 3-5 mm in length dissected from surface-sterilized, immature seeds of the soybean cultivar A2872, are cultured in the light or dark at 26°C on an appropriate agar medium for six to ten weeks. Somatic embryos producing secondary embryos are then excised and placed into a suitable liquid medium. After repeated selection for clusters of somatic embryos that multiplied as early, globular-staged embryos, the suspensions are maintained as described below.

Soybean embryogenic suspension cultures can be maintained in 35 ml liquid media on a rotary shaker, 150 rpm, at 26°C with florescent lights on a 16:8 hour day/night schedule. Cultures are subcultured every two weeks by inoculating approximately 35 mg of tissue into 35 ml of liquid medium.

Soybean embryogenic suspension cultures may then be transformed by the method of particle gun bombardment (Klein et al. (1987) Nature (London) 327:70-73, U.S. Patent No. 4,945,050). A Du Pont Biolistic PDS1000/HE instrument (helium retrofit) can be used for these transformations.

A selectable marker gene that can be used to facilitate soybean transformation is a transgene composed of the 35S promoter from Cauliflower Mosaic Virus (Odell et al. (1985) Nature 313:810-812), the hygromycin phosphotransferase gene from plasmid pJR225 (from *E. coli*; Gritz et al. (1983) Gene 25:179-188), and the 3' region of the nopaline synthase gene from the T-DNA of the Ti plasmid of *Agrobacterium tumefaciens*. The expression cassette comprising an antisense CNR sequence operably linked to the ubiquitin promoter can be isolated as a restriction fragment This fragment can then be inserted into a unique restriction site of the vector carrying the marker gene.

To 50 µl of a 60 mg/ml 1 µm gold particle suspension is added (in order): 5 µl DNA (1 µg/µl), 20 µl spermidine (0.1 M), and 50 µl CaCl₂ (2.5 M). The particle preparation is then agitated for three minutes, spun in a microfuge for 10 seconds and the supernatant removed. The DNA-coated particles are then washed once in 400 µl 70% ethanol and resuspended in 40 µl of anhydrous ethanol. The DNA/particle suspension can be sonicated three times for one second each. Five microliters of the DNA-coated gold particles are then loaded on each macro carrier disk.

Approximately 300-400 mg of a two-week-old suspension culture is placed in an empty 60x15 mm petri dish and the residual liquid removed from the tissue with a pipette. For each transformation experiment, approximately 5-10 plates of tissue are normally bombarded. Membrane rupture pressure is set at 1100 psi, and the chamber is evacuated to a vacuum of 28 inches mercury. The tissue is placed approximately 3.5 inches away from the retaining screen and bombarded three times. Following bombardment, the tissue can be divided in half and placed back into liquid and cultured as described above.

Five to seven days post bombardment, the liquid media may be exchanged with fresh media, and eleven to twelve days post-bombardment with fresh media containing 50 mg/ml hygromycin. This selective media can be refreshed weekly. Seven to eight weeks post-bombardment, green, transformed tissue may be observed growing from untransformed, necrotic embryogenic clusters. Isolated green tissue is removed and inoculated into individual flasks to generate new, clonally propagated, transformed embryogenic suspension cultures. Each new line may be treated as an independent transformation event These suspensions can then be subcultured and maintained as clusters of immature embryos or regenerated into whole plants by maturation and germination of individual somatic embryos.

### Example 8. Sunflower Meristem Tissue Transformation

Sunflower meristem tissues are transformed with an expression cassette containing an antisense CNR sequences operably linked to a ubiquitin promoter as follows (see also European Patent Number EP 0 486233 and Malone-Schoneberg et al. (1994) Plant Science 103:199-207). Mature sunflower seed (*Helianthus annuus* L.) are dehulled using a single wheat-head thresher. Seeds are surface sterilized for 30 minutes in a 20% Clorox bleach solution with the addition of two drops of Tween 20 per 50 ml of solution. The seeds are rinsed twice with sterile distilled water.

Split embryonic axis explants are prepared by a modification of procedures described by Schrammeijer *et al*. (Schrammeijer et al. (1990) Plant Cell Rep. 9:55-60). Seeds are imbibed in distilled water for 60 minutes following the surface sterilization procedure. The cotyledons of each seed are then broken off, producing a clean fracture at the plane of the embryonic axis. Following excision of the root tip, the explants are bisected longitudinally between the primordial leaves. The two halves are placed, cut surface up, on GBA medium consisting of Murashige and Skoog mineral elements (Murashige et al. (1962) Physiol. Plant., 15: 473-497), Shepard's vitamin additions (Shepard (1980) in *Emergent Techniques for the Genetic Improvement of Crops* (University of Minnesota Press, St. Paul, Minnesota), 40 mg/l adenine sulfate, 30 g/l sucrose, 0.5 mg/l 6-benzyl-aminopurine (BAP), 0.25 mg/l indole-3-acetic acid (IAA), 0.1 mg/l gibberellic acid (GA₃), pH 5.6, and 8 g/l Phytagar.

The explants are subjected to microprojectile bombardment prior to *Agrobacterium* treatment (Bldney et a/. (1992) Plant Mol. Biol. 18:301-313). Thirty to forty explants are placed in a circle at the center of a 60 X 20 mm plate for this treatment. Approximately 4.7 mg of 1.8 mm tungsten microprojectiles are resuspended in 25 ml of sterile TE buffer (10 mM Tris HCl, 1 mM EDTA, pH 8.0) and 1.5 ml aliquots are used per bombardment. Each plate is bombarded twice through a 150 mm nytex screen placed 2 cm above the samples in a PDS 1000^{®} particle acceleration device.

Disarmed *Agrobacterium tumefaciens* strain EHA105 is used in all transformation experiments. A binary plasmid vector comprising the expression cassette that contains the CNR gene operably linked to the ubiquitin promoter is introduced into *Agrobacterium* strain EHA105 via freeze-thawing as described by Holsters et al. (1978) Mol. Gen. Genet 163:181-187. This plasmid further comprises a kanamycin selectable marker gene (i.e, *nptII*). Bacteria for plant transformation experiments are grown overnight (28°C and 100 RPM continuous agitation) in liquid YEP medium (10 gm/l yeast extract, 10 gm/l Bactopeptone, and 5 gm/l NaCl, pH 7.0) with the appropriate antibiotics required for bacterial strain and binary plasmid maintenance. The suspension is used when it reaches an OD₆₀₀ of about 0.4 to 0.8. The *Agrobacterium* cells are pelleted and resuspended at a final OD₆₀₀ of 0.5 in an inoculation medium comprised of 12.5 mM MES pH 5,7, 1 gm/l NH₄Cl, and 0.3 gm/l MgSO₄.

Freshly bombarded explants are placed in an *Agrobacterium* suspension, mixed, and left undisturbed for 30 minutes. The explants are then transferred to GBA medium and co-cultivated, cut surface down, at 26°C and 18-hour days. After three days of co-cultivation, the explants are transferred to 374B (GBA medium lacking growth regulators and a reduced sucrose level of 1%) supplemented with 250 mg/l cefotaxime and 50 mg/l kanamycin sulfate. The explants are cultured for two to five weeks on selection and then transferred to fresh 374B medium lacking kanamycin for one to two weeks of continued development. Explants with differentiating, antibiotic-resistant areas of growth that have not produced shoots suitable for excision are transferred to GBA medium containing 250 mg/l cefotaxime for a second 3-day phytohormone treatment. Leaf samples from green, kanamycin-resistant shoots are assayed for the presence of NPTII by ELISA and for the presence of transgene expression by assaying for a modulation in meristem development (i.e., an alteration of size and appearance of shoot and floral meristems).

NPTII-positive shoots are grafted to Pioneer^{®} hybrid 6440 *in vitro*-grown sunflower seedling rootstock. Surface sterilized seeds are germinated in 48-0 medium (half-strength Murashige and Skoog salts, 0.5% sucrose, 0.3% gelrite, pH 5.6) and grown under conditions described for explant culture. The upper portion of the seedling is removed, a 1 cm vertical slice is made in the hypocotyl, and the transformed shoot inserted into the cut. The entire area is wrapped with parafilm to secure the shoot. Grafted plants can be transferred to soil following one week of *in vitro* culture. Grafts in soil are maintained under high humidity conditions followed by a slow acclimatization to the greenhouse environment. Transformed sectors of To plants (parental generation) maturing in the greenhouse are identified by NPTII ELISA and/or by CNR activity analysis of leaf extracts while : transgenic seeds harvested from NPTII-positive To plants are identified by CNR activity analysis of small portions of dry seed cotyledon.

An alternative sunflower transformation protocol allows the recovery of transgenic progeny without the use of chemical selection pressure. Seeds are dehulled and surface-sterilized for 20 minutes in a 20% Clorox bleach solution with the addition of two to three drops of Tween 20 per 100 ml of solution, then rinsed three times with distilled water. Sterilized seeds are imbibed in the dark at 26°C for 20 hours on filter paper moistened with water. The cotyledons and root radical are removed, and the meristem explants are cultured on 374E (GBA medium consisting of MS salts, Shepard vitamins, 40 mg/l adenine sulfate, 3% sucrose, 0.5 mg/l 6-BAP, 0.25 mg/l IAA, 0.1 mg/l GA, and 0.8% Phytagar at pH 5.6) for 24 hours under the dark. The primary leaves are removed to expose the apical meristem, around 40 explants are placed with the apical dome facing upward in a 2 cm circle in the center of 374M (GBA medium with 1.2% Phytagar), and then cultured on the medium for 24 hours in the dark.

Approximately 18.8 mg of 1.8 µm tungsten particles are resuspended in 150 µl absolute ethanol. After sonication, 8 µl of it is dropped on the center of the surface of macrocarrier. Each plate is bombarded twice with 650 psi rupture discs in the first shelf at 26 mm of Hg helium gun vacuum.

The plasmid of interest is introduced into *Agrobacterium tumefaciens* strain EHA105 via freeze thawing as described previously. The pellet of overnight-grown bacteria at 28°C in a liquid YEP medium (10 g/l yeast extract, 10 g/l Bactopeptone, and 5 g/l NaCl, pH 7.0) in the presence of 50 µg/l kanamycin is resuspended in an inoculation medium (12.5 mM 2-mM 2-(N-morpholino) ethanesulfonic acid, MES, 1 g/l NH₄Cl and 0.3 g/l MgSO₄ at pH 5.7) to reach a final concentration of 4.0 at OD 600. Particle-bombarded explants are transferred to GBA medium (374E), and a droplet of bacteria suspension is placed directly onto the top of the meristem. The explants are co-cultivated on the medium for 4 days, after which the explants are transferred to 374C medium (GBA with 1% sucrose and no BAP, IAA, GA3 and supplemented with 250 µg/ml cefotaxime). The plantlets are cultured on the medium for about two weeks under 16-hour day and 26°C incubation conditions.

Explants (around 2 cm long) from two weeks of culture in 374C medium are screened for a modulation in meristem development (i.e., an alteration of size and appearance of shoot and floral meristems). After positive (i.e., a decrease in CNR expression) explants are identified, those shoots that fail to exhibit a decrease in CNR activity are discarded, and every positive explant is subdivided into nodal explants. One nodal explant contains at least one potential node. The nodal segments are cultured on GBA medium for three to four days to promote the formation of auxiliary buds from each node. Then they are transferred to 374C medium and allowed to develop for an additional four weeks. Developing buds are separated and cultured for an additional four weeks on 374C medium. Pooled leaf samples from each newly recovered shoot are screened again by the appropriate protein activity assay. At this time, the positive shoots recovered from a single node will generally have been enriched in the transgenic sector detected in the initial assay prior to nodal culture.

Recovered shoots positive for a decreased CNR expression are grafted to Pioneer hybrid 6440 *in vitro*-grown sunflower seedling rootstock. The rootstocks are prepared in the following manner. Seeds are dehulled and surface-sterilized for 20 minutes in a 20% Clorox bleach solution with the addition of two to three drops of Tween 20 per 100 ml of solution, and are rinsed three times with distilled water. The sterilized seeds are germinated on the filter moistened with water for three days, then they are transferred into 48 medium (half-strength MS salt, 0.5% sucrose, 0.3% gelrite pH 5.0) and grown at 26°C under the dark for three days, then incubated at 16-hour-day culture conditions. The upper portion of selected seedling is removed, a vertical slice is made in each hypocotyl, and a transformed shoot is inserted into a V-cut. The cut area is wrapped with parafilm. After one week of culture on the medium, grafted plants are transferred to soil. In the first two weeks, they are maintained under high humidity conditions to acclimatize to a greenhouse environment

### Example 9. Variants of CNR Sequences

### A. Variant Nucleotide Sequences of CNR That Do Not Alter the Encoded Amino Acid Sequence

The CNR nucleotide sequences are used to generate variant nucleotide sequences having the nucleotide sequence of the open reading frame with about 70%, 75%, 80%, 85%, 90%, and 95% nucleotide sequence identity when compared to the starting unaltered ORF nucleotide sequence of the corresponding SEQ ID NO. These functional variants are generated using a standard codon table. While the nucleotide sequence of the variants are altered, the amino acid sequence encoded by the open reading frames do not change.

### B. Variant Amino Acid Sequences of CNR Polypeptides

Variant amino acid sequences of the CNR polypeptides are generated. In this example, one amino acid is altered. Specifically, the open reading frames are reviewed to determine the appropriate amino acid alteration. The selection of the amino acid to change is made by consulting the protein alignment (with the other orthologs and other gene family members from various species). An amino acid is selected that is deemed not to be under high selection pressure (not highly conserved) and which is rather easily substituted by an amino acid with similar chemical characteristics (i.e., similar functional side-chain). Using the protein alignment set forth in Figure 2, an appropriate amino acid can be changed. Once the targeted amino acid is identified, the procedure outlined in the following section C is followed. Variants having about 70%, 75%, 80%, 85%, 90%, and 95% nucleic acid sequence identity are generated using this method.

### C. Additional Variant Amino Acid Sequences of CNR Polypeptides

In this example, artificial protein sequences are created having 80%, 85%, 90%, and 95% identity relative to the reference protein sequence. This latter effort requires identifying conserved and variable regions from the alignment set forth in Figure 2 and then the judicious application of an amino acid substitutions table. These parts will be discussed in more detail below.

Largely, the determination of which amino acid sequences are altered is made based on the conserved regions among CNR protein or among the other CNR polypeptides. Based on the sequence alignment, the various regions of the CNR polypeptide that can likely be altered are represented in lower case letters, while the conserved regions are represented by capital letters. It is recognized that conservative substitutions can be made in the conserved regions below without altering function. In addition, one of skill will understand that functional variants of the CNR sequence of the invention can have minor non-conserved amino acid alterations in the conserved domain.

Artificial protein sequences are then created that are different from the original in the intervals of 80-85%, 85-90%, 90-95%, and 95-100% identity. Midpoints of these intervals are targeted, with liberal latitude of plus or minus 1%, for example. The amino acids substitutions will be effected by a custom Perl script. The substitution table is provided below in Table 3.

**Table 3. Substitution Table**

| **Amino Acid** | **Strongly Similar and Optimal Substitution** | **Rank of Order to Change** | **Comment** |
|---|---|---|---|
| I | L,V | 1 | 50:50 substitution |
| L | I,V | 2 | 50:50 substitution |
| V | I,L | 3 | 50:50 substitution |
| A | G | 4 | |
| G | A | 5 | |
| D | E | 6 | |
| E | D | 7 | |
| W | Y | 8 | |
| Y | W | 9 | |
| S | T | 10 | |
| T | S | 11 | |
| K | R | 12 | |
| R | K | 13 | |
| N | Q | 14 | |
| Q | N | 15 | |
| F | Y | 16 | |
| M | L | 17 | First methionine cannot change |
| H | | Na | No good substitutes |
| C | | Na | No good substitutes |
| P | | Na | No good substitutes |

First, any conserved amino acids in the protein that should not be changed is identified and "marked off" for insulation from the substitution. The start methionine will of course be added to this list automatically. Next, the changes are made.

H, C, and P are not changed in any circumstance. The changes will occur with isoleucine first, sweeping N-terminal to C-terminal. Then leucine, and so on down the list until the desired target it reached. Interim number substitutions can be made so as not to cause reversal of changes. The list is ordered 1-17, so start with as many isoleucine changes as needed before leucine, and so on down to methionine. Clearly many amino acids will in this manner not need to be changed. L, I and V will involve a 50:50 substitution of the two alternate optimal substitutions.

The variant amino acid sequences are written as output. Perl script is used to calculate the percent identities. Using this procedure, variants of the CNR polypeptides are generating having about 80%, 85%, 90%, and 95% amino acid identity to the starting unaltered ORF nucleotide sequence of SEQ ID NO:3, 6, 10, or 14.

### Example 10. Transgenic Maize Plants

25 T0 transgenic maize plants containing the ZmCNR01 construct under the control of the ubiquitin promoter were generated. These transgenic plants were grown in greenhouse conditions. Each of the 25 plants was found to have suppressed growth throughout their development. The extent of the growth suppression correlated with the copy number of the transgene. Transgenic plants with higher copy number had corresponding reductions in plant growth. Transgenic maize plants having one, two and four copies of the transgene, show approximately 30-50%, 60-70% and 80-90% reduction in plant height, respectively. These transgenic plants also contained reduced organ and tissue size, including smaller tassels, ears and leaves. The reduction in growth of organs and tissues may be associated with reduced cell number.

### Example 11. Transgenic Maize Callus

Transgenic maize callus tissue expressing the ZmCNR02 gene under the control of the ubiquitin promoter exhibited significantly inhibited growth in cell (callus) culture. Individual plants associated with these calli would be expected to have a reduction in plant size and/or reduced organ and tissue size. Further evaluation of transgenic plants would present a more uniform genetic background for comparison. ZmCNR01 callus did not reveal the same characteristic reduction in callus growth, but the transgenic plants did. The differences in expression of the two genes may be related to protein action strength or tissue-response to the gene function.

Together the results for ZmCNR01 and ZmCNR02 indicate that these genes are capable of negatively regulating maize tissue growth. While the general presumed function of the tomato FW2.2, namely negative cell number regulation, and associated reduction in tissue size, is apparently preserved for these maize genes, these experiments demonstrate that cell number/tissue size control is exhibited in a very different plant and plant architecture, and in diverse tissues that are distinct from the specific instance of the tomato fruit carpels. Accordingly, this information argues that ZmCNR01 and ZmCNR02, and likely other maize ZmCNR genes, could be used to control tissue growth as exemplified.

All publications and patent applications in this specification are indicative of the level of ordinary skill in the art to which this invention pertains.

## Claims

1. A method of reducing or eliminating the activity of a cell number regulatory (CNR) polypeptide in a plant,
wherein said CNR polypeptide is encoded by a polynucleotide of SEQ ID NO: 1 or a a polynucleotide having at least 90% sequence identity, as determined by the GAP algorithm under default parameters, to the full length sequence of a polynucleotide of SEQ ID NO: 1;
said method comprising:
(a) transformation of a plant cell with an expression cassette that expresses a polynucleotide that inhibits the expression of said CNR polypeptide, or
(b) gene disruption of said polynucleotide encoding said CNR polypeptide, and
and regeneration of a plant in which the activity of said CNR polypeptide is reduced or eliminated;
said plant having enhanced root growth, increased seed size, increased seed weight, increased embryo size, increased leaf size, increased seedling vigor, enhanced silk emergence, or increased ear size.

2. A method of claim 1 further comprising obtaining a seed from said plant, said seed comprising the polynucleotide of claim 1(a) that inhibits the expression of said CNR polypeptide or the gene disruption of claim 1(b).

3. A method of claim 1 or 2 wherein said reduction or elimination is mediated:
(i) by cosuppression with an expression cassette that expresses an RNA molecule corresponding to all or part of a messenger RNA encoding said CNR polypeptide in the sense orientation;
(ii) by antisense suppression with an expression cassette that expresses an RNA molecule complementary to all or part of a messenger RNA encoding the CNR polypeptide;
(iii) by double-stranded RNA interference in which a sense RNA molecule corresponding to all or part of a messenger RNA encoding said CNR polypeptide in the sense orientation and an antisense RNA molecule that is fully or partially complementary to the sense RNA molecule are expressed in the same cell;
(iv) by hairpin RNA interference (hpRNA) with an expression cassette that expresses an RNA molecule that hybridizes with itself to form a hairpin structure that comprises a single-stranded loop region and a base-paired stem, or by intron-containing hairpin RNA (ihpRNA) interference (ihpRNA) with an expression cassette that expresses an RNA molecule that hybridizes with itself to form a hairpin structure that comprises a single-stranded loop region and a base-paired stem but the RNA molecule additionally comprises an intron that is capable of being spliced in the cell in which the ihpRNA is expressed;
(v) by amplicon-mediated interference;
(vi) by expression of a ribozyme, whereby the polynucleotide expressed by the expression cassette is catalytic RNA or has ribozyme activity specific for the messenger RNA of said CNR polypeptide;
(vii) by micro RNA with an expression cassette that expresses an RNA molecule that forms a hairpin structure containing a 22-nucleotide sequence that is complementary to a target sequence selected from the mRNA transcript sequence of said CNR polypeptide and contains 22 nucleotides of said CNR sequence in sense orientation and 21 nucleotides of a corresponding antisense sequence that is complementary to the sense sequence;
(viii) by polypeptide-based inhibition of gene expression with an expression cassette that expresses a polynucleotide that encodes a zinc finger protein that binds to the gene encoding the CNR polypeptide;
(ix) by polypeptide-based inhibition of protein Activity with an expression cassette that expresses an antibody that binds to said CNR polypeptide, or wherein the binding of the antibody results in increased turnover of the antibody-CNR complex by cellular quality control mechanisms;
(x) by transposon tagging of the polynucleotide encoding the CNR polypeptide; or
(xi) by mutagenesis of the polynucleotide encoding the CNR polypeptide.

4. A method of any one of the preceding claims, wherein said plant is a monocot.

5. A method of any one of claims 1 to 3, wherein said plant is a dicot.

6. A method of any one of the preceding claims wherein said plant is maize, sorghum, sunflower, soybean, wheat, alfalfa, rice, cotton, canola, barley, millet, or tomato.

7. A method of claim 1 comprising:
(a) providing a nucleotide sequence comprising at least 15 consecutive nucleotides of the complement of SEQ ID NO: 1;
(b) providing a plant cell comprising an mRNA having the sequence set forth in SEQ ID NO: 1; and
introducing the nucleotide sequence of step (a) into the plant cell, wherein the nucleotide sequence inhibits expression of the mRNA in the plant cell.

8. The method of claim 7, wherein said plant cell is from a monocot.

9. The method of claim 7, wherein said monocot is maize, wheat, rice, barley, sorghum or rye.

10. The method of claim 7, wherein said plant cell is from a dicot.

11. A method of increasing the cell number regulatory activity in a plant, comprising:
(a) introducing into a plant cell a recombinant expression cassette, wherein said cassette comprises a polynucleotide selected from:
(i) a polynucleotide of SEQ ID NO: 1 or a polynucleotide having at least 90% sequence identity, as determined by the GAP algorithm under default parameters, to the full length sequence of a polynucleotide of SEQ ID NO: 1; wherein the polynucleotide encodes a polypeptide that functions as a modifier of cell number; and
(ii) a polynucleotide encoding a polypeptide of SEQ ID NO: 2 or a polypeptide having at least 90% sequence identity, as determined by the GAP algorithm under default parameters, to the full length sequence of a polypeptide of SEQ ID NO: 2, wherein the polypeptide functions as a modifier of cell number;
operably linked, in sense or anti-sense orientation, to a promoter; and
(b) regenerating a plant from said plant cell;
wherein the cell number regulatory activity in said plant is increased and the plant has decreased root growth, decreased seed size, decreased seed weight, decreased embryo size, or decreased tassel production.

12. The method of claim 11, wherein said plant is a monocot.

13. The method of claim 11, wherein said plant is a dicot.

14. The method of any one of claims 11 to 13, wherein said plant is selected from maize, soybean, sunflower, sorghum, canola, wheat, alfalfa, cotton, rice, barley, millet, peanut and cocoa.

## Patentansprüche

1. Verfahren zur Reduzierung oder Eliminierung der Aktivität eines für die Zellzahl regulatorischen (cell number regulatory (CNR)) Polypeptids in einer Pflanze,
wobei das CNR-Polypeptid durch ein Polynukleotid der SEQ ID NO:1 oder ein Polynukleotid, das wenigstens 90% Sequenzidentität, wie sie durch den GAP-Algorithmus unter Verwendung von Default-Parametern bestimmt wird, zu der Volllängensequenz eines Polynukleotids der SEQ ID NO:1 hat, codiert wird;
wobei das Verfahren umfasst:
(a) Transformation einer Pflanzenzelle mit einer Expressionskassette, die ein Polynukleotid exprimiert, das die Expression des CNR-Polypeptids inhibiert, oder
(b) Genunterbrechung des Polynukleotids, das das CNR-Polypeptid codiert, und
Regeneration einer Pflanze, in der die Aktivität des CNR-Polypeptids reduziert oder eliminiert ist;
wobei die Pflanze ein verstärktes Wurzelwachstum, eine erhöhte Samengröße, ein erhöhtes Samengewicht, eine erhöhte Embryogröße, eine erhöhte Blattgröße, eine erhöhte Wuchsstärke der Keimpflanze, ein verstärktes Narbenfädenerscheinen oder eine erhöhte Ährengröße hat.

2. Verfahren gemäß Anspruch 1, außerdem umfassend: Erhalten eines Samens aus der Pflanze, wobei der Samen das Polynukleotid von Anspruch 1(a), das die Expression des CNR-Polypeptids inhibiert, oder die Genunterbrechung von Anspruch 1(b) umfasst.

3. Verfahren gemäß Anspruch 1 oder 2, wobei die Reduzierung oder Eliminierung vermittelt wird:
(i) durch Cosuppression mit einer Expressionskassette, die ein RNA-Molekül exprimiert, entsprechend der gesamten oder einem Teil einer Messenger-RNA, die das CNR-Polypeptid codiert, in der Sense-Orientierung;
(ii) durch Antisense-Suppression mit einer Expressionskassette, die ein RNA-Molekül exprimiert, das komplementär ist zu der Gesamtheit oder einem Teil einer Messenger-RNA, die das CNR-Polypeptid codiert;
(iii) durch Doppelstrang-RNA-Interferenz, bei der ein Sense-RNA-Molekül, das der Gesamtheit oder einem Teil einer Messenger-RNA, die das CNR-Polypeptid codiert, entspricht, in Sense-Orientierung und ein Antisense-RNA-Molekül, das vollständig oder teilweise komplementär zu dem Sense-RNA-Molekül ist, in derselben Zelle exprimiert werden;
(iv) durch Haarnadelschleifen-RNA-Interferenz (hpRNA) mit einer Expressionskassette, die ein RNA-Molekül exprimiert, das mit sich selbst unter Bildung einer Haarnadelstruktur hybridisiert, welche eine einzelsträngige Schleifenregion und einen Basen-gepaarten Stamm umfasst, oder durch Intron-enthaltende Haarnadelschleifen-RNA (ihpRNA)-Interferenz (ihpRNA) mit einer Expressionskassette, die ein RNA-Molekül exprimiert, das mit sich selbst unter Bildung einer Haarnadelstruktur hybridisiert, welche eine einzelsträngige Schleifenregion und einen Basen-gepaarten Stamm umfasst, das RNA-Molekül aber zusätzlich ein Intron umfasst, das fähig ist, in der Zelle, in der die ihpRNA exprimiert wird, gespliced zu werden;
(v) durch Amplicon-vermittelte Interferenz;
(vi) durch Expression eines Ribozyms, wobei das durch die Expressionskassette exprimierte Polynukleotid katalytische RNA ist oder Ribozymaktivität hat, die spezifisch für die Messenger-RNA des CNR-Polypeptids ist;
(vii) durch Mikro-RNA mit einer Expressionskassette, die ein RNA-Molekül exprimiert, das eine Haarnadelschleifenstruktur bildet, die eine 22-Nukleotid-Sequenz enthält, die komplementär zu einer Target-Sequenz, ausgewählt aus der mRNA-Transkriptsequenz des CNR-Polypeptids ist und 22 Nukleotide der CNR-Sequenz in Sense-Orientierung und 21 Nukleotide einer entsprechenden Antisense-Sequenz, die komplementär zu der Sense-Sequenz ist, enthält;
(viii) durch Polypeptid-basierte Inhibierung der Genexpression mit einer Expressionskassette, die ein Polynukleotid exprimiert, das ein Zinkfingerprotein codiert, das an das Gen bindet, das das CNR-Polypeptid codiert;
(ix) durch Polypeptid-basierte Inhibierung von Proteinaktivität mit einer Expressionskassette, die einen Antikörper exprimiert, der an das CNR-Polypeptid bindet, oder wobei die Bindung des Antikörpers in einem erhöhten Turnover des Antikörper-CNR-Komplexes durch zelluläre Qualitätskontrollmechanismen resultiert;
(x) durch Transposon-Tagging des Polynukleotids, das das CNR-Polypeptid codiert, oder
(xi) durch Mutagenese des Polynukleotids, das das CNR-Polypeptid codiert.

4. Verfahren gemäß einem der vorangehenden Ansprüche, wobei die Pflanze eine Monokotyle ist.

5. Verfahren gemäß einem der Ansprüche 1 bis 3, wobei die Pflanze eine Dikotyle ist.

6. Verfahren gemäß einem der vorangehenden Ansprüche, wobei die Pflanze Mais, Sorghum, Sonnenblume, Sojabohne, Weizen, Alfalfa, Reis, Baumwolle, Canola, Gerste, Hirse oder Tomate ist.

7. Verfahren gemäß Anspruch 1, umfassend:
(a) Bereitstellen einer Nukleotidsequenz, die wenigstens 15 fortlaufende Nukleotide des Komplements von SEQ ID NO:1 umfasst;
(b) Bereitstellen einer Pflanzenzelle, die eine mRNA umfasst, die die in SEQ ID NO:1 angegebene Sequenz hat, und
Einführen der Nukleotidsequenz von Schritt (a) in die Pflanzenzelle, wobei die Nukleotidsequenz eine Expression der mRNA in der Pflanzenzelle inhibiert.

8. Verfahren gemäß Anspruch 7, wobei die Pflanzenzelle aus einer Monokotyle ist.

9. Verfahren gemäß Anspruch 7, wobei die Monokotyle Mais, Weizen, Reis, Gerste, Sorghum oder Roggen ist.

10. Verfahren gemäß Anspruch 7, wobei die Pflanzenzelle aus einer Dikotyle ist.

11. Verfahren zur Erhöhung der für die Zellzahl regulatorischen Aktivität in einer Pflanze, umfassend:
(a) Einführen einer rekombinanten Expressionskassette in eine Pflanzenzelle, wobei die Kassette ein Polynukleotid umfasst, das ausgewählt wird aus:
(i) einem Polynukleotid der SEQ ID NO:1 oder einem Polynukleotid, das wenigstens 90% Sequenzidentität, wie sie durch den GAP-Algorithmus unter Verwendung von Default-Parametern bestimmt wird, zu der Volllängensequenz eines Polynukleotids der SEQ ID NO:1 hat; wobei das Polynukleotid ein Polypeptid codiert, das als Modifizierungsmittel der Zellzahl fungiert, und
(ii) einem Polynukleotid, das ein Polypeptid der SEQ ID NO:2 oder ein Polypeptid, das wenigstens 90% Sequenzidentität, wie sie durch den GAP-Algorithmus unter Verwendung von Default-Parametern bestimmt wird, zu der Volllängensequenz eines Polypeptids der SEQ ID NO:2 hat, codiert, wobei das Polypeptid als Modifizierungsmittel der Zellzahl fungiert;
funktionell verknüpft, in Sense- oder Antisense-Orientierung mit einem Promotor, und
(b) Regenerieren einer Pflanze aus der Pflanzenzelle,
wobei die für die Zellzahl regulatorische Aktivität in der Pflanze erhöht wird und die Pflanze ein verringertes Wurzelwachstum, eine verringerte Samengröße, ein verringertes Samengewicht, eine verringerte Embryogröße oder eine verringerte Fahnenproduktion hat.

12. Verfahren gemäß Anspruch 11, wobei die Pflanze eine Monokotyle ist.

13. Verfahren gemäß Anspruch 11, wobei die Pflanze eine Dikotyle ist.

14. Verfahren gemäß einem der Ansprüche 11 bis 13, wobei die Pflanze aus Mais, Sojabohne, Sonnenblume, Sorghum, Canola, Weizen, Alfalfa, Baumwolle, Reis, Gerste, Hirse, Erdnuss und Kakao ausgewählt ist.

## Revendications

1. Méthode de réduction ou d'élimination de l'activité d'un polypeptide régulant le nombre de cellules (CNR) dans une plante,
dans laquelle ledit polypeptide CNR est codé par un polynucléotide de SED ID NO : 1 ou un polynucléotide ayant au moins 90 % d'identité de séquence, tel que déterminé par
l'algorithme GAP avec des paramètres par défaut, avec la séquence de longueur complète d'un polynucléotide de SEQ ID NO :1 ;
ladite méthode comprenant :
(a) la transformation d'une cellule de plante avec une cassette d'expression qui exprime un polynucléotide qui inhibe l'expression dudit polypeptide CNR, ou
(b) la disruption génique dudit polynucléotide codant pour ledit polypeptide CNR, et
et la régénération d'une plante dans laquelle l'activité dudit polypeptide CNR est réduite ou éliminée ;
ladite plante présentant une croissance racinaire stimulée, une taille de semence accrue, un poids de semence accru, une taille d'embryon accrue, une taille foliaire accrue, une vigueur du semis accru, une émergence de soies stimulée, ou une taille d'épi accrue.

2. Méthode selon la revendication 1 comprenant en outre l'obtention d'une semence de ladite plante, ladite semence comprenant le polynucléotide de la revendication 1 (a) qui inhibe l'expression dudit polypeptide CNR ou la disruption génique de la revendication 1(b).

3. Méthode selon la revendication 1 ou 2 dans laquelle ladite réduction ou élimination est produite :
(i) par co-suppression avec une cassette d'expression qui exprime une molécule d'ARN correspondant à tout ou partie d'un ARN messager codant pour ledit polypeptide CNR en orientation sens ;
(ii) par suppression antisens avec une cassette d'expression qui exprime une molécule d'ARN complémentaire de tout ou d'une partie d'un ARN messager codant pour ledit polypeptide CNR ;
(iii) par interférence d'un ARN double-brin dans lequel une molécule d'ARN sens correspondant à tout ou partie d'un ARN messager codant pour ledit polypeptide CNR en orientation sens et une molécule d'ARN antisens qui est totalement ou partiellement complémentaire de la molécule d'ARN sens sont exprimées dans la même cellule ;
(iv) par interférence d'un ARN en épingle à cheveux (hpRNA) avec une cassette d'expression qui exprime une molécule d'ARN qui s'hybride avec elle-même pour former une structure en épingle à cheveux qui comprend une région en boucle simple brin et une région tige à bases appariées, ou par interférence d'un ARN en épingle à cheveux contenant un intron (ihpRNA) avec une cassette d'expression qui exprime une molécule d'ARN qui s'hybride avec elle-même pour former une structure en épingle à cheveux qui comprend une région de boucle simple brin et une tige à bases appariées mais la molécule d'ARN comprend en outre un intron qui peut être épissé dans la cellule dans laquelle l'ihpRNA est exprimé ;
(v) par interférence sous la médiation d'un amplicon ;
(vi) par expression d'un ribozyme, dans lequel le polynucléotide exprimé par la cassette d'expression est un ARN catalytique ou a une activité de ribozyme spécifique de l'ARN messager dudit polypeptide CNR ;
(vii) par un micro ARN avec une cassette d'expression qui exprime une molécule d'ARN qui forme une structure en épingle à cheveux contenant une séquence de 22 nucléotides qui est complémentaire d'une séquence cible choisie parmi la séquence de l'ARNm transcrit dudit polypeptide CNR et qui contient 22 nucléotides de ladite séquence CNR en orientation sens et 21 nucléotides d'une séquence antisens correspondante qui est complémentaire de la séquence sens ;
(viii)par inhibition basée sur un polypeptide de l'expression génique avec une cassette d'expression qui exprime un polynucléotide qui code pour une protéine à doigt de zinc qui se lie au gène codant pour le polypeptide CNR ;
(ix) par inhibition basée sur un polypeptide de l'activité d'une protéine avec une cassette d'expression qui exprime un anticorps qui se lie audit polypeptide CNR, ou dans laquelle la liaison de l'anticorps résulte en l'accroissement de la rotation du complexe anticorps-CNR par des mécanismes cellulaires de contrôle de qualité ;
(x) par étiquetage avec un transposon du polynucléotide codant pour le polypeptide CNR ; ou
(xi) par mutagenèse du polynucléotide codant pour le polypeptide CNR.

4. Méthode selon l'une quelconque des revendications précédentes, dans laquelle ladite plante est une monocotylédone.

5. Méthode selon l'une quelconque des revendications 1 à 3, dans laquelle ladite plante est une dicotylédone.

6. Méthode selon l'une quelconque des revendications précédentes, dans laquelle ladite plante est le maïs, le sorgho, le tournesol, le soja, le blé, la luzerne, le riz, le coton, le colza, l'orge, le millet ou la tomate.

7. Méthode selon la revendication 1 comprenant :
(a) la fourniture d'une séquence nucléotidique comprenant au moins 15 nucléotides consécutifs du complément de SEQ ID NO :1 ;
(b) la fourniture d'une cellule de plante comprenant un ARNm ayant la séquence présentée en SEQ ID NO :1 ; et
l'introduction de la séquence nucléotidique de l'étape (a) dans la cellule de plante, dans laquelle la séquence nucléotidique inhibe l'expression de l'ARNm dans la cellule de plante.

8. Méthode selon la revendication 7, dans laquelle ladite cellule de plante provient d'une monocotylédone.

9. Méthode selon la revendication 7, dans laquelle ladite monocotylédone est le maïs, le blé, le riz, l'orge, le sorgho ou le seigle.

10. Méthode selon la revendication 7, dans laquelle ladite cellule de plante provient d'une dicotylédone.

11. Méthode d'accroissement de l'activité de régulation du nombre de cellules dans une plante comprenant :
(a) l'introduction dans une cellule de plante d'une cassette d'expression recombinante, dans laquelle ladite cassette comprend un polynucléotide choisi parmi :
(i) un polynucléotide de SEQ ID NO :1 ou un polynucléotide ayant au moins 90 % d'identité de séquence, tel que déterminé par l'algorithme GAP avec des paramètres par défaut, avec la séquence de longueur complète d'un polynucléotide de SEQ ID NO :1 ; dans laquelle le polynucléotide code pour un polypeptide qui fonctionne comme un modificateur du nombre de cellules ; et
(ii) un polynucléotide codant pour un polypeptide de SEQ ID NO :2 ou un polynucléotide ayant au moins 90 % d'identité de séquence, tel que déterminé par l'algorithme GAP avec des paramètres par défaut, avec la séquence de longueur complète d'un polypeptide de SEQ ID NO :2 ; dans laquelle le polypeptide fonctionne comme un modificateur du nombre de cellules ;
lié de manière fonctionnelle, en orientation sens ou antisens, à un promoteur ; et
(b) la régénération d'une plante à partir de ladite cellule de plante.
dans laquelle l'activité de régulation du nombre de cellules dans ladite plante est accrue et la plante présente une croissance racinaire réduite, une taille de semence réduite, un poids de semence réduit, une taille d'embryon réduite ou une production de gland réduite.

12. Méthode selon la revendication 11, dans laquelle ladite plante est une monocotylédone.

13. Méthode selon la revendication 11, dans laquelle ladite plante est une dicotylédone.

14. Méthode selon l'une quelconque des revendications 11 à 13, dans laquelle ladite plante est choisie parmi le maïs, le soja, le tournesol, le sorgho, le colza, le blé, la luzerne, le coton, le riz, l'orge, le millet, l'arachide et le cacao.


### SEQUENCE LISTING

<110> Pioneer Hi-Bred International, Inc.
<120> Cell Number Polynucleotides and Polypeptides and Methods of Use Thereof.
<130> 1874-PCT
<150> 60/583,340
   <151> 2004-06-28
<160> 45
<170> FastSEQ for Windows Version 4.0
<210> 1
   <211> 910
   <212> DNA
   <213> Zea mays
<400> 1
   actcgagtcg agtcttgccg acctggggcc tgctgcgcct ggatgaagag ctgtgctgag 60
   agaatcatta aagcagcacc gggtccttgc tttgcgcgtt gctgttcgca taaacaagag 120
   atcctagttc tactcccaag caatgtatcc gtcagcccct ccggacgcgt ataacaagtt 180
   cagcgccggg gctccgccaa cggcgccgcc gccgccggca gcgtaccacc agcagcagca 240
   gcagcacgga gcgaacatgg acacttcacg ccccggcggc gggctgagga aatggtccac 300
   cggcctcttc cactgcatgg acgacccggg gaactgtctc atcacatgcc tgtgcccctg 360
   cgtcacgttc gggcaggtcg ctgacatcgt ggacaagggc acctgcccat gtatcgcgag 420
   cggactggtg tacgggctca tctgcgcgtc gacggggatg gggtgcctgt actcgtgcct 480
   ctaccggtcc aagctgaggg ccgagtacga cctggacgaa ggggagtgcc cggacatcct 540
   ggtgcactgc tgctgcgagc acctggctct gtgccaagag taccgcgagc tcaagaaccg 600
   cggcttcgac ctggggatcg gctgggaggc taacatggac cggcagaggc gaggagttgc 660
   cggcggcggc gcggtgatgg gggcgccgcc ggccataccg ctgggcatga ttaggtagat 720
   gattgattga tgcggtggca gaacttgctt tcgtggttct tttgtttgac tatgtaatgt 780
   atcttcgttt ccatcgattc tatgtaattt aactgtccct ttttttggct ctctattgtg 840
   gaggacgctg ggtgtgaata gcactgtttt ggatgggttt gtgattggtc taaaaaaaaa 900
   aaaaaaaaaa                                                        910
<210> 2
   <211> 191
   <212> PRT
   <213> Zea mays
<400> 2
   Met Tyr Pro Ser Ala Pro Pro Asp Ala Tyr Asn Lys Phe Ser Ala Gly
   1               5                   10                  15
   Ala Pro Pro Thr Ala Pro Pro Pro Pro Ala Ala Tyr His Gln Gln Gln
               20                  25                  30
   Gln Gln His Gly Ala Asn Met Asp Thr Ser Arg Pro Gly Gly Gly Leu
           35                  40                  45
   Arg Lys Trp Ser Thr Gly Leu Phe His Cys Met Asp Asp Pro Gly Asn
       50                  55                  60
   Cys Leu Ile Thr Cys Leu Cys Pro Cys Val Thr Phe Gly Gln Val Ala
   65                  70                  75                  80
   Asp Ile Val Asp Lys Gly Thr Cys Pro Cys Ile Ala Ser Gly Leu Val
                   85                  90                  95
   Tyr Gly Leu Ile Cys Ala Ser Thr Gly Met Gly Cys Leu Tyr Ser Cys
               100                 105                 110
   Leu Tyr Arg Ser Lys Leu Arg Ala Glu Tyr Asp Leu Asp Glu Gly Glu
           115                 120                 125
   Cys Pro Asp Ile Leu Val His Cys Cys Cys Glu His Leu Ala Leu Cys
       130                 135                 140
   Gln Glu Tyr Arg Glu Leu Lys Asn Arg Gly Phe Asp Leu Gly Ile Gly
   145                 150                 155                 160
   Trp Glu Ala Asn Met Asp Arg Gln Arg Arg Gly Val Ala Gly Gly Gly
                   165                 170                 175
   Ala Val Met Gly Ala Pro Pro Ala Ile Pro Leu Gly Met Ile Arg
               180                 185                 190
<210> 3
   <211> 813
   <212> DNA
   <213> Zea mays
<400> 3
   gcacaaggca ataaataaaa agcccaacgc tgggctccgc tagctctcgg tctctctgga 60
   gtctggactc gcctccacgg ctccaccatg tatcccaagg cggcagacga aggtgcgcag 120
   ccgctggcca cgggcatccc cttcagcggc ggcggcggct actaccaggc gggcggcgcg 180
   atggcggcgg cgttcgcggt gcaggcgcag gcgcccgtcg ccgcctggtc caccgggctc 240
   tgcaactgct tcgacgactg ccacaactgc tgcgtgacgt gcgtgtgccc gtgcatcacg 300
   ttcgggcaga ccgcggagat catcgaccgg ggctccacgt cctgcggcac cagcggggcg 360
   ctgtacgcgc tcgtcatgct gctcaccggc tgtcagtgcg tctactcctg cttctaccgc 420
   gccaagatgc gcgcgcagta cggcctccag gtgagcccct gctccgactg ctgcgtgcac 480
   tgctgctgcc agtgctgcgc gctctgccag gagtaccgcg agctcaagaa gcgaggcttc 540
   gacatgagca taggatggca tgcgaacatg gagaggcagg ggcgcgccgc cgccgccgtg 600
   ccgccgcaca tgcatcctgg gatgacccgc tgacgctctg ccgctcgcct cacttctgct 660
   gaggaaatca agtgatttgg tattggtccg ctcccagcag gcagtatcaa ctactgtaac 720
   caatccatga tctgtatgcg gtatcgggct gaactgatac tttatggact tgttgcttca 780
   aaaaaaaaaa aaaaaaaaaa aaaaaaaaaa aaa                              813
<210> 4
   <211> 181
   <212> PRT
   <213> Zea mays
<400> 4
   Met Tyr Pro Lys Ala Ala Asp Glu Gly Ala Gln Pro Leu Ala Thr Gly
    1               5                  10                  15
   Ile Pro Phe Ser Gly Gly Gly Gly Tyr Tyr Gln Ala Gly Gly Ala Met
               20                  25                  30
   Ala Ala Ala Phe Ala Val Gln Ala Gln Ala Pro Val Ala Ala Trp Ser
           35                  40                  45
   Thr Gly Leu Cys Asn Cys Phe Asp Asp Cys His Asn Cys Cys Val Thr
       50                  55                  60
   Cys Val Cys Pro Cys Ile Thr Phe Gly Gln Thr Ala Glu Ile Ile Asp
   65                  70                  75                  80
   Arg Gly Ser Thr Ser Cys Gly Thr Ser Gly Ala Leu Tyr Ala Leu Val
                   85                  90                  95
   Met Leu Leu Thr Gly Cys Gln Cys Val Tyr Ser Cys Phe Tyr Arg Ala
               100                 105                 110
   Lys Met Arg Ala Gln Tyr Gly Leu Gln Val Ser Pro Cys Ser Asp Cys
           115                 120                 125
   Cys Val His Cys Cys Cys Gln Cys Cys Ala Leu Cys Gln Glu Tyr Arg
       130                 135                 140
   Glu Leu Lys Lys Arg Gly Phe Asp Met Ser Ile Gly Trp His Ala Asn
   145                 150                 155                 160
   Met Glu Arg Gln Gly Arg Ala Ala Ala Ala Val Pro Pro His Met His
                   165                 170                 175
   Pro Gly Met Thr Arg
               180
<210> 5
   <211> 836
   <212> DNA
   <213> Zea mays
<400> 5
   cgaccacctg cgctgccgct gccgtgccaa gtcgccgggc aagctccgga tcggggcgcg 60
   cgcgcgcggg gccgccgccg ccgccattat attgggttat tagttaggcc agctggtcat 120
   tgcatcggag agatgtatcc ggccactacg ccctacgaga cggcgtccgg ggtgggcgtg 180
   gcgccggtgg ccggcttgtt ccccgtcgcc ggagaggcca gggagtggtc gtcgaggctc 240
   ctggactgct tcgacgactt cgacatctgc tgcatgacgt tttggtgccc gtgcatcacg 300
   ttcgggcgga cggcggagat cgtggaccac ggcatgacgt cgtgcgggac gagcgcggcg 360
   ctgttcgcgc tgatccagtg gctgtcgggg tcgcagtgca cgtgggcctt ctcctgcacg 420
   taccgcacca ggctgcgcgc gcagcacggg ctccccgagg cgccctgcgc cgacttcctc 480
   gtccacctct gctgcctcca ctgcgcgctc tgccaggagt acagggagct aaaggcgcgc 540
   ggctacgagc ccgtcctcgg ctgggagttc aacgcccaga gggccgccgc cggcgtcgcc 600
   atgtgcccgc cggcctcgca ggggatgggg cgctgatcat ccgtcgacca gccagccagc 660
   ccgtcggggt ttgattcatg tctccgatct gttggttcat gcatgcatgt ctccatttaa 720
   ttcatctgtt catatattca tgtctccaga tccagacggt acatataata atatattcag 780
   ttgttttacg taaaatcgag aaaaaaaaaa aaaaaaaaaa aaaaaaaaaa aaaaaa     836
<210> 6
   <211> 167
   <212> PRT
   <213> Zea mays
<400> 6
   Met Tyr Pro Ala Thr Thr Pro Tyr Glu Thr Ala Ser Gly Val Gly Val
    1               5                  10                  15
   Ala Pro Val Ala Gly Leu Phe Pro Val Ala Gly Glu Ala Arg Glu Trp
               20                  25                  30
   Ser Ser Arg Leu Leu Asp Cys Phe Asp Asp Phe Asp Ile Cys Cys Met
           35                  40                  45
   Thr Phe Trp Cys Pro Cys Ile Thr Phe Gly Arg Thr Ala Glu Ile Val
       50                  55                  60
   Asp His Gly Met Thr Ser Cys Gly Thr Ser Ala Ala Leu Phe Ala Leu
   65                  70                  75                  80
   Ile Gln Trp Leu Ser Gly Ser Gln Cys Thr Trp Ala Phe Ser Cys Thr
                   85                  90                  95
   Tyr Arg Thr Arg Leu Arg Ala Gln His Gly Leu Pro Glu Ala Pro Cys
               100                 105                 110
   Ala Asp Phe Leu Val His Leu Cys Cys Leu His Cys Ala Leu Cys Gln
           115                 120                 125
   Glu Tyr Arg Glu Leu Lys Ala Arg Gly Tyr Glu Pro Val Leu Gly Trp
       130                 135                 140
   Glu Phe Asn Ala Gln Arg Ala Ala Ala Gly Val Ala Met Cys Pro Pro
   145                 150                 155                 160
   Ala Ser Gln Gly Met Gly Arg
                   165
<210> 7
   <211> 527
   <212> DNA
   <213> Zea mays
<400> 7
   agtatcaatt atcataacct ccgactccga cgatctgaac tgtgaagatg agcacttatc 60
   caccgccgac gggggaatgg accaccggcc tctgtggctg cttcagcgac tgcaagagct 120
   gttgcctgtc gttcttgtgc ccgtgtatcc cgttcggaca ggtggcagag gtcttggaca 180
   agggcatgac atcctgtggt ctggccggcc tcctctactg tttgctgctg catgccgggg 240
   tggctgtggt cccgtgccac tgcatctaca cctgcaccta ccgtcgcaag ctccgggcgg 300
   cgtacgacct gccgccggag ccgtgcgccg attgctgcgt ccacatgtgg tgcgggccat 360
   gcgccatctc ccagatgtac cgggagctca agaacagagg cgccgacccg gccatgggta 420
   ggcagccagc cttctctctc tctctcacca gtcactgccg tttctttctg aaatcaaaat 480
   attaccatat cataaaaaaa aattggcgca ccgttaaata cggataa               527
<210> 8
   <211> 159
   <212> PRT
   <213> Zea mays
<400> 8
   Met Ser Thr Tyr Pro Pro Pro Thr Gly Glu Trp Thr Thr Gly Leu Cys
   1               5                   10                  15
   Gly Cys Phe Ser Asp Cys Lys Ser Cys Cys Leu Ser Phe Leu Cys Pro
               20                  25                  30
   Cys Ile Pro Phe Gly Gln Val Ala Glu Val Leu Asp Lys Gly Met Thr
           35                  40                  45
   Ser Cys Gly Leu Ala Gly Leu Leu Tyr Cys Leu Leu Leu His Ala Gly
       50                  55                  60
   Val Ala Val Val Pro Cys His Cys Ile Tyr Thr Cys Thr Tyr Arg Arg
   65                  70                  75                  80
   Lys Leu Arg Ala Ala Tyr Asp Leu Pro Pro Glu Pro Cys Ala Asp Cys
                   85                  90                  95
   Cys Val His Met Trp Cys Gly Pro Cys Ala Ile Ser Gln Met Tyr Arg
               100                 105                 110
   Glu Leu Lys Asn Arg Gly Ala Asp Pro Ala Met Gly Arg Gln Pro Ala
           115                 120                 125
   Phe Ser Leu Ser Leu Thr Ser His Cys Arg Phe Phe Leu Lys Ser Lys
       130                 135                 140
   Tyr Tyr His Ile Ile Lys Lys Asn Trp Arg Thr Val Lys Tyr Gly
   145                 150                 155
<210> 9
   <211> 1009
   <212> DNA
   <213> Zea mays
<400> 9
   aattcctgcc cgggcgggcg tagaggcgaa gcattcgttg gggacgaacg tttagggccg 60
   gggatcgggg aacacgaggc tgcgcgttgg cgcaagcaag caaattttgg gtcctagctt 120
   gaaggactgc catctttttt ttttgggatg gctggaaaag gaagctatgt acctccacaa 180
   tatattccct tatacagcct agataccgaa gaggatcgtg tccctgccgt ggaagagaac 240
   catgctacgc gccctaaact aaaccaggat ccaacacaat ggtcatctgg catctgtgcc 300
   tgttttgatg acccccagag ctgttgtatt ggtgcgactt gcccctgttt cctttttgga 360
   aagaatgcac aattcttggg atctggaact cttgctggat catgcactac acattgcatg 420
   ttgtggggcc ttctcacaag tctatgctgt gtatttactg gaggtctagt attagcagtt 480
   ccaggatctg ccgttgcttg ttatgcttgc ggatatcgca gtgcactaag aacaaagtac 540
   aatcttccgg aagcaccctg tggcgatttg acgacacact tattctgtca cttgtgtgct 600
   atatgccagg agtacaggga gatccgtgag agaacaggca gtggctcctc accagctcct 660
   aatgtgactc cacctccagt tcagacgatg gatgagcttt gatctttaac tgatatgaat 720
   gtatgttatc tgacaaactt tggtgtgtgg cctggtggct agtgctgcct ttgcctgttg 780
   gcattttgac atatttttgt tcttgggctc tcacaacgct gctatgttgt ttgtagagtt 840
   gtaaacacgg gttttgaact agtgaaccac tgacagttca cggaagttgt aaacacagtt 900
   gtaccatgga actgtagtag aattattaag tgtatttttt tttggctatt tatattggtg 960
   agaggatggg aagggaaaaa aaaaaaaaaa aaaaaaaaaa aaaaaaaaa           1009
<210> 10
   <211> 184
   <212> PRT
   <213> Zea mays
<400> 10
   Met Ala Gly Lys Gly Ser Tyr Val Pro Pro Gln Tyr Ile Pro Leu Tyr
    1               5                  10                  15
   Ser Leu Asp Thr Glu Glu Asp Arg Val Pro Ala Val Glu Glu Asn His
               20                  25                  30
   Ala Thr Arg Pro Lys Leu Asn Gln Asp Pro Thr Gln Trp Ser Ser Gly
           35                  40                  45
   Ile Cys Ala Cys Phe Asp Asp Pro Gln Ser Cys Cys Ile Gly Ala Thr
       50                  55                  60
   Cys Pro Cys Phe Leu Phe Gly Lys Asn Ala Gln Phe Leu Gly Ser Gly
   65                  70                  75                  80
   Thr Leu Ala Gly Ser Cys Thr Thr His Cys Met Leu Trp Gly Leu Leu
                   85                  90                  95
   Thr Ser Leu Cys Cys Val Phe Thr Gly Gly Leu Val Leu Ala Val Pro
               100                 105                 110
   Gly Ser Ala Val Ala Cys Tyr Ala Cys Gly Tyr Arg Ser Ala Leu Arg
           115                 120                 125
   Thr Lys Tyr Asn Leu Pro Glu Ala Pro Cys Gly Asp Leu Thr Thr His
       130                 135                 140
   Leu Phe Cys His Leu Cys Ala Ile Cys Gln Glu Tyr Arg Glu Ile Arg
   145                 150                 155                 160
   Glu Arg Thr Gly Ser Gly Ser Ser Pro Ala Pro Asn Val Thr Pro Pro
                   165                 170                 175
   Pro Val Gln Thr Met Asp Glu Leu
               180
<210> 11
   <211> 976
   <212> DNA
   <213> Zea mays
<400> 11
   gcaaggccgg ggtcggcagc agaggggagc agagagatct cattctccga tccgcccagg 60
   ccaccatggc ggaggatgct acgagcagcc acccgtcacg ctacgtcaag ctcaccaagg 120
   accaggacgc ccccgccgag gacatccgcc ccggcgagct caaccagcca gttcacgtcc 180
   cgcagctcga aggccggagg tgtagcgagt gcggtcaggt cctgcccgag agctacgagc 240
   cgcccgccga cgagccctgg accaccggga tcttcggatg caccgatgac ccagagacct 300
   gccgaactgg attgttttgc ccctgcgtgc tgtttgggcg caacgttgag gctgttaggg 360
   aggacatccc ttggacaact ccttgcgtgt gccatgctgt attcgttgaa ggagggatca 420
   cgctggcgat tctgacggcg atatttcacg gtgttgatcc gaggacgtca ttcctgattg 480
   gagaaggtct ggtgttcagc tggtggttat gtgctaccta cactggcatc ttccgccagg 540
   ggcttcagag gaaatatcat ctcaagaact ctccgtgtga cccatgcatg gtccactgct 600
   gcttgcactg gtgtgccaac tgccaggagc atcgcgagag gacgggacgg cttgcagaga 660
   acaacgcagt gcccatgacg gttgtgaacc cgcctccggt gcaagagatg agcatgctgg 720
   aggaggtgga ggagaaggga gcagagaaga gtgaacacga tgatgtggag gtcattcctc 780
   tatagataat agggtcggag tccgagtgag gatagcacta gcagcatcag atgacgatca 840
   caacttctgg tcattcctct atagttagtt agttattgat ctttcaacaa caaaatgtgg 900
   tggtgtggca actgtccatt tttattatcc caaataataa tactgtacta cgttacggcc 960
   caaaaaaaaa aaaaaa                                                 976
<210> 12
   <211> 239
   <212> PRT
   <213> Zea mays
<400> 12
   Met Ala Glu Asp Ala Thr Ser Ser His Pro Ser Arg Tyr Val Lys Leu
   1               5                   10                  15
   Thr Lys Asp Gln Asp Ala Pro Ala Glu Asp Ile Arg Pro Gly Glu Leu
               20                  25                  30
   Asn Gln Pro Val His Val Pro Gln Leu Glu Gly Arg Arg Cys Ser Glu
           35                  40                  45
   Cys Gly Gln Val Leu Pro Glu Ser Tyr Glu Pro Pro Ala Asp Glu Pro
       50                  55                  60
   Trp Thr Thr Gly Ile Phe Gly Cys Thr Asp Asp Pro Glu Thr Cys Arg
   65                  70                  75                  80
   Thr Gly Leu Phe Cys Pro Cys Val Leu Phe Gly Arg Asn Val Glu Ala
                   85                  90                  95
   Val Arg Glu Asp Ile Pro Trp Thr Thr Pro Cys Val Cys His Ala Val
               100                 105                 110
   Phe Val Glu Gly Gly Ile Thr Leu Ala Ile Leu Thr Ala Ile Phe His
           115                 120                 125
   Gly Val Asp Pro Arg Thr Ser Phe Leu Ile Gly Glu Gly Leu Val Phe
       130                 135                 140
   Ser Trp Trp Leu Cys Ala Thr Tyr Thr Gly Ile Phe Arg Gln Gly Leu
   145                 150                 155                 160
   Gln Arg Lys Tyr His Leu Lys Asn Ser Pro Cys Asp Pro Cys Met Val
                   165                 170                 175
   His Cys Cys Leu His Trp Cys Ala Asn Cys Gln Glu His Arg Glu Arg
               180                 185                 190
   Thr Gly Arg Leu Ala Glu Asn Asn Ala Val Pro Met Thr Val Val Asn
           195                 200                 205
   Pro Pro Pro Val Gln Glu Met Ser Met Leu Glu Glu Val Glu Glu Lys
       210                 215                 220
   Gly Ala Glu Lys Ser Glu His Asp Asp Val Glu Val Ile Pro Leu
   225                 230                 235
<210> 13
   <211> 801
   <212> DNA
   <213> Zea mays
<400> 13
   cggacgcgtg gggtgaacgc gagagccgac cgtctcagtg tgcgcgcgaa gaagagaagg 60
   aggaggagga ggaggaagaa gaagaagaat gtatccggcc aagcccaccg tcgcgaccgc 120
   cagcgagccg gtaaccggga tggcggcgcc gccggtgacc ggcatcccca tcagcagccc 180
   cggccccgcc gtcgcggcca gccagtggtc ctcgggcctc tgcgcctgct tcgacgactg 240
   cggcctctgc tgcatgacgt gctggtgccc gtgcgtgacg ttcgggcgga tcgcggaggt 300
   cgtggaccgc ggcgcgacgt cgtgcgcggc cgcgggggcc atctacacgc tgctggcctg 360
   cttcacgggg ttccagtgcc actggatcta ctcctgcacg taccgctcca agatgcgcgc 420
   gcagctgggg ctccccgacg tcggctgctg cgactgctgc gtccacttct gctgcgagcc 480
   gtgcgcgctc tgccagcagt acagggagct cagggcacgc ggcttggacc ccgcgctcgg 540
   ctgggacgtc aacgcccaga aggcggccaa taataacgcc ggcgccggca tgacgatgta 600
   cccgccgacg gcgcagggga tgggccgcta attacgctac cagctccatc ggtaaactca 660
   tatgttgttg tgttgtgtca gccagatcat cgcgtacgta cgggtatact tgtgatatgt 720
   aatgtcaggg accggcatgc ccatgtgttt caagatattt cacgttcaag tcctttctgt 780
   ctgctcacta tccgaatgaa a                                           801
<210> 14
   <211> 180
   <212> PRT
   <213> Zea mays
<400> 14
   Met Tyr Pro Ala Lys Pro Thr Val Ala Thr Ala Ser Glu Pro Val Thr
    1               5                  10                  15
   Gly Met Ala Ala Pro Pro Val Thr Gly Ile Pro Ile Ser Ser Pro Gly
               20                  25                  30
   Pro Ala Val Ala Ala Ser Gln Trp Ser Ser Gly Leu Cys Ala Cys Phe
           35                  40                  45
   Asp Asp Cys Gly Leu Cys Cys Met Thr Cys Trp Cys Pro Cys Val Thr
       50                  55                  60
   Phe Gly Arg Ile Ala Glu Val Val Asp Arg Gly Ala Thr Ser Cys Ala
   65                  70                  75                  80
   Ala Ala Gly Ala Ile Tyr Thr Leu Leu Ala Cys Phe Thr Gly Phe Gln
                   85                  90                  95
   Cys His Trp Ile Tyr Ser Cys Thr Tyr Arg Ser Lys Met Arg Ala Gln
               100                 105                 110
   Leu Gly Leu Pro Asp Val Gly Cys Cys Asp Cys Cys Val His Phe Cys
           115                 120                 125
   Cys Glu Pro Cys Ala Leu Cys Gln Gln Tyr Arg Glu Leu Arg Ala Arg
       130                 135                 140
   Gly Leu Asp Pro Ala Leu Gly Trp Asp Val Asn Ala Gln Lys Ala Ala
   145                 150                 155                 160
   Asn Asn Asn Ala Gly Ala Gly Met Thr Met Tyr Pro Pro Thr Ala Gln
                   165                 170                 175
   Gly Met Gly Arg
               180
<210> 15
   <211> 1103
   <212> DNA
   <213> Zea mays
<400> 15
   gtccccttcc ctcgcctcgc gttaggtagc ctgcttccga cccgcacatc tccctccggt 60
   tctcgccatg ggcgccggcg ccaacaacca cgaggagtcg agccccctca tcccagctgc 120
   ggttgccgcc cccgcggacg agaagccccc gcaggctccg gcgccggagg ccgccaatta 180
   ctatgccgac ggggtcccgg tcgtgatggg cgagcccgtg tcggcccacg ccttcggcgg 240
   cgtcccgcgg gagagctgga actccgggat cctctcctgc ctcggccgca acgacgagtt 300
   ctgcagcagc gacgtcgaag tgtgtcttct tggaacggta gcaccatgtg tgctctatgg 360
   tagcaatgtt gagaggcttg ctgctggaca aggcacattt gcaaacagct gtttgcctta 420
   cactgggctg tatttgcttg ggaactctct ctttgggtgg aactgccttg ccccatggtt 480
   ctctcatccc actcgtactg ctattcgaca acgctacaat cttgagggta gctttgaggc 540
   tttcaccagg caatgtgggt gctgcggcga tctggtcgag gacgaggaga ggcgtgagca 600
   cctagaggcc gcctgtgacc ttgcgaccca ctacctctgc cacccctgcg ccctctgcca 660
   ggagggtcgc gagctgcgcc gcagggttcc ccaccctgga ttcaacaatg ggcactccgt 720
   ctttgtcatg atgccgccca tggagcagac catggggcgt ggcatgtgag ctattccacc 780
   accttccctg ccctagtttt atctgtgctt cccgtggttt atcatctgct gctgttggcc 840
   ttgatgtgtg atgtgtcgtt tgttcgtcag taaatacgag tttgtgatat gaggtcgtgc 900
   tgggaacttt ggattgtttg cttcttatcg actgcagttt ggattctgaa cagtcactta 960
   tctcctgtgt tactgtgttt gtacggtggt cctcgatggg cctgtaaatg tgaaatgcat 1020
   ttcgtcgtaa cttaggcccc gtttcaaaaa aaaaaaaaaa aaaaaaaaaa aaaaaaaaaa 1080
   aaaaaaaaaa aaaaaaaaaa aaa                                        1103
<210> 16
   <211> 233
   <212> PRT
   <213> Zea mays
<400> 16
   Met Gly Ala Gly Ala Asn Asn His Glu Glu Ser Ser Pro Leu Ile Pro
   1               5                   10                  15
   Ala Ala Val Ala Ala Pro Ala Asp Glu Lys Pro Pro Gln Ala Pro Ala
               20                  25                  30
   Pro Glu Ala Ala Asn Tyr Tyr Ala Asp Gly Val Pro Val Val Met Gly
           35                  40                  45
   Glu Pro Val Ser Ala His Ala Phe Gly Gly Val Pro Arg Glu Ser Trp
       50                  55                  60
   Asn Ser Gly Ile Leu Ser Cys Leu Gly Arg Asn Asp Glu Phe Cys Ser
   65                  70                  75                  80
   Ser Asp Val Glu Val Cys Leu Leu Gly Thr Val Ala Pro Cys Val Leu
                   85                  90                  95
   Tyr Gly Ser Asn Val Glu Arg Leu Ala Ala Gly Gln Gly Thr Phe Ala
               100                 105                 110
   Asn Ser Cys Leu Pro Tyr Thr Gly Leu Tyr Leu Leu Gly Asn Ser Leu
           115                 120                 125
   Phe Gly Trp Asn Cys Leu Ala Pro Trp Phe Ser His Pro Thr Arg Thr
       130                 135                 140
   Ala Ile Arg Gln Arg Tyr Asn Leu Glu Gly Ser Phe Glu Ala Phe Thr
   145                 150                 155                 160
   Arg Gln Cys Gly Cys Cys Gly Asp Leu Val Glu Asp Glu Glu Arg Arg
                   165                 170                 175
   Glu His Leu Glu Ala Ala Cys Asp Leu Ala Thr His Tyr Leu Cys His
               180                 185                 190
   Pro Cys Ala Leu Cys Gln Glu Gly Arg Glu Leu Arg Arg Arg Val Pro
           195                 200                 205
   His Pro Gly Phe Asn Asn Gly His Ser Val Phe Val Met Met Pro Pro
       210                 215                 220
   Met Glu Gln Thr Met Gly Arg Gly Met
   225                 230
<210> 17
   <211> 873
   <212> DNA
   <213> Zea mays
<400> 17
   gaattaagcg agcgccggcc gaggcagaca caacccgcag caacaacttg catcggcacg 60
   gacgaccgat tgagagcctc ggcgttcgta cgccacgcca ccaagagact ctgtgcaaca 120
   agtgaaggga cgaaatgtat ccggccaagc ccgccgcctc ctccagccag ccagcggccg 180
   agatggcgca gccggtggtc ggcatcccca tcagcagccc cggcgcggtc gcggtcgggc 240
   cggtcgtcgg caagtggtcc tccggcctct gcgcctgctc cgacgactgc ggcctctgct 300
   gcctgacgtg ctggtgcccg tgcatcacgt tcggccgcat cgcggagatc gtggaccgcg 360
   gcgcgacgtc gtgcggcgtg gcggggacca tctacacgct gctggcctgc ttcacgggct 420
   gccactggat ctactcctgc acgtaccgct ccaggatgcg cgcgcagctc ggcctccccg 480
   aagcctgctg ctgcgactgc tgcgtccact tctgctgcga gccctgcgcg ctctcccagc 540
   agtacaggga gctcaaggcc cgcggattcg accccgacct cggctgggac gtcaacgcgc 600
   agaaggccgc cgccgccgcc gccatgtacc cgccgccggc ggaggggatg atgatccgct 660
   aagctaagct agctacctcg tcgccgatcc ggcccaacct ccaacgctaa actcagatgc 720
   gttgcgtcag tcagatcatc gcgtacgtac gtgttttgta cgtatggtcg tgtatatttg 780
   tacgtgatgc gtatatatat aatgttatga ctgaatgtct gagttttaag atttgtacgt 840
   ttgaatcctt ttcttaaaaa aaaaaaaaaa aaa                             873
<210> 18
   <211> 175
   <212> PRT
   <213> Zea mays
<400> 18
   Met Tyr Pro Ala Lys Pro Ala Ala Ser Ser Ser Gln Pro Ala Ala Glu
    1               5                  10                  15
   Met Ala Gln Pro Val Val Gly Ile Pro Ile Ser Ser Pro Gly Ala Val
               20                  25                  30
   Ala Val Gly Pro Val Val Gly Lys Trp Ser Ser Gly Leu Cys Ala Cys
           35                  40                  45
   Ser Asp Asp Cys Gly Leu Cys Cys Leu Thr Cys Trp Cys Pro Cys Ile
       50                  55                  60
   Thr Phe Gly Arg Ile Ala Glu Ile Val Asp Arg Gly Ala Thr Ser Cys
   65                  70                  75                  80
   Gly Val Ala Gly Thr Ile Tyr Thr Leu Leu Ala Cys Phe Thr Gly Cys
                   85                  90                  95
   His Trp Ile Tyr Ser Cys Thr Tyr Arg Ser Arg Met Arg Ala Gln Leu
               100                 105                 110
   Gly Leu Pro Glu Ala Cys Cys Cys Asp Cys Cys Val His Phe Cys Cys
           115                 120                 125
   Glu Pro Cys Ala Leu Ser Gln Gln Tyr Arg Glu Leu Lys Ala Arg Gly
       130                 135                 140
   Phe Asp Pro Asp Leu Gly Trp Asp Val Asn Ala Gln Lys Ala Ala Ala
   145                 150                 155                 160
   Ala Ala Ala Met Tyr Pro Pro Pro Ala Glu Gly Met Met Ile Arg
                   165                 170                 175
<210> 19
   <211> 752
   <212> DNA
   <213> Zea mays
<400> 19
   caaacgcaac cgcagctaca gcacagcaca gcacagcaca acacgtcggc atgtatccgc 60
   ccaaggccag cggcgatccg gccgccgggg cggcgccggt gactggcttc cccgtcggcg 120
   ggcctgccgc ctcctcccag tggtcctccg gcctgttgga ctgcttcgac gactgcggcc 180
   tctgctgcct gacgtgctgg tgcccgtgca tcacgttcgg gcgcgtggcg gagatcgtgg 240
   accgcggcgc gacgtcgtgc ggcacggcgg gggcgctgta cgcggtgctg gcctacttca 300
   cgggctgcca gtggatctac tcgtgcacgt accgcgccaa gatgcgcgcc cagctcggcc 360
   tccccgagac cccctgctgc gactgcctcg tccacttctg ctgcgagccg tgcgcgctct 420
   gccagcagta caaggagctc aaggcccgcg gcttcgaccc cgtcctcggc tgggaccgca 480
   acgccactat gctgcctccg tccgcacagg ggatgggccg ctgaccgctg accggccagc 540
   ctctgcgtaa ataaataatt aatgcttata tatgtactag tatagtgccc gtgcgttgcg 600
   acggcacaca aatatagtgt ccgaacttgg caaagacgat gcccatgtcc atgcgtccta 660
   ccagatactg gagatattgt gtttcagatt cactgctaga agcaatcaac atatgcaagt 720
   cttaaaaaaa aaaaaaaaaa aaaaaaaaaa aa                               752
<210> 20
   <211> 157
   <212> PRT
   <213> Zea mays
<400> 20
   Met Tyr Pro Pro Lys Ala Ser Gly Asp Pro Ala Ala Gly Ala Ala Pro
    1              5                   10                  15
   Val Thr Gly Phe Pro Val Gly Gly Pro Ala Ala Ser Ser Gln Trp Ser
               20                  25                  30
   Ser Gly Leu Leu Asp Cys Phe Asp Asp Cys Gly Leu Cys Cys Leu Thr
           35                  40                  45
   Cys Trp Cys Pro Cys Ile Thr Phe Gly Arg Val Ala Glu Ile Val Asp
       50                  55                  60
   Arg Gly Ala Thr Ser Cys Gly Thr Ala Gly Ala Leu Tyr Ala Val Leu
   65                  70                  75                  80
   Ala Tyr Phe Thr Gly Cys Gln Trp Ile Tyr Ser Cys Thr Tyr Arg Ala
                   85                  90                  95
   Lys Met Arg Ala Gln Leu Gly Leu Pro Glu Thr Pro Cys Cys Asp Cys
               100                 105                 110
   Leu Val His Phe Cys Cys Glu Pro Cys Ala Leu Cys Gln Gln Tyr Lys
           115                 120                 125
   Glu Leu Lys Ala Arg Gly Phe Asp Pro Val Leu Gly Trp Asp Arg Asn
       130                 135                 140
   Ala Thr Met Leu Pro Pro Ser Ala Gln Gly Met Gly Arg
   145                 150                 155
<210> 21
   <211> 1753
   <212> DNA
   <213> Zea mays
<400> 21
   cttggatcat tgacgctgca ctgctgagcg cgttcaggag taatatatat aggaacgaaa 60
   gttaccgaga aactggaaga tcctaaactg ttgccaggac atggacatca tgcatgggga 120
   tctcagaagg aatggttcaa accagagcgt gcccagtcgg catggatgga tcgatatgga 180
   ggccctctct cctctggcgg atcaatccat gtatccatga gagagagaga gagagaggca 240
   taggccaaga ctaggccttt tgttttgaga tgggctgctg ctatctagag cgatactgct 300
   atatagtggt accaagagga ccaaaggtcg cgttgcctga aaccaccgag atcatacaga 360
   aactaatcgc cgcaacgcat ccaaacccga ccaactgacg gcgcgcgtct gcgtgaacag 420
   ctcgcagcat gccgggggag tggtccgtgg ggctctgcga ctgcttcggg gatcttcaca 480
   cctgtacgca gtgcaaacaa acctgctagc tatagcaagc tactctcctc tctctctctt 540
   cttcttcttc ccgtacgtac tacgatgcat gtttttcagc tctccgtccc ttctctcccg 600
   gccgacgatc gaccacgttg caggttgcct gacgctctgg tgcccctgcg tcacgttcgg 660
   ccgcaccgcg gagatcgtgg acagaggctc cacgtgtacg cgttccactt cactcccata 720
   ctactactgc ccccctcgtc tcgtctccat ctctcctgtt ccttttggct gacgatccgt 780
   cccgcattgc atatttgcag cgtgctgcat gagtggcaca ctgtactacc tgctgtcgac 840
   gataggctgg cagtggctgt acggctgcgc caagcgctcc tccatgcggt cgcagtacag 900
   cctgcgagag tccccctgca tggactgctg cgtccacttc tggtgcggcc cctgcgcgct 960
   ctgccaggag tacacggagc tccagaaacg cggcttccac atggccaaag gtatcagctc 1020
   ccccccccat cttcccacag tttaactagg cggcctttga tggttccatg atcaccgtgc 1080
   ctctacaaaa ccattatatt ccttttattt gcaggatggg aagggagcaa caaggtggtg 1140
   gggtgcttcc atgggatgac gacgccacca aggaagcaat ccatgtgctt ttaggatagc 1200
   atagctccat cgttctatat aatatgcgct ttattttgaa taaaaatata tgggtctgtc 1260
   tattgatgct ttatttagag ctcgttgggt ctcattagtt cctattgctc atagttatgg 1320
   tttccattta tttaaatcat cgattgttca ctttatttta cgttgatttt atgattatta 1380
   catggcgttt gagcctctgc cggcctctta cgtgagaccc agtaattaat aaagcaatca 1440
   aaacaaggat tagatatacc tatagatatg cattaagtgg atcagcttct aaataaacat 1500
   aagagcagtt ataatatgtt ttgccataga tttttgccaa gttagaggag aaagagggga 1560
   agaaactaac ggaccactaa ttatcagtcg agagattcca aaatagtgct ccatccgtca 1620
   caaaatataa ttctttatcc atttattaac cttaggatat agtttaaagt tggtatgtat 1680
   atctatattt attattattt attctaacgt gcatagaaaa agattattag aaagaactat 1740
   gttttgggac aga                                                   1753
<210> 22
   <211> 160
   <212> PRT
   <213> Zea mays
<400> 22
   Met Asp Ile Met His Gly Asp Leu Arg Arg Asn Gly Ser Asn Gln Ser
    1               5                  10                  15
   Val Pro Ser Arg His Gly Trp Ile Asp Met Glu Ala Leu Ser Pro Leu
               20                  25                  30
   Ala Asp Gln Ser Ile Met Pro Gly Glu Trp Ser Val Gly Leu Cys Asp
           35                  40                  45
   Cys Phe Gly Asp Leu His Thr Cys Cys Leu Thr Leu Trp Cys Pro Cys
       50                  55                  60
   Val Thr Phe Gly Arg Thr Ala Glu Ile Val Asp Arg Gly Ser Thr Ser
   65                  70                  75                  80
   Cys Cys Met Ser Gly Thr Leu Tyr Tyr Leu Leu Ser Thr Ile Gly Trp
                       85                  90                  95
   Gln Trp Leu Tyr Gly Cys Ala Lys Arg Ser Ser Met Arg Ser Gln Tyr
                   100                 105                 110
   Ser Leu Arg Glu Ser Pro Cys Met Asp Cys Cys Val His Phe Trp Cys
               115                 120                 125
   Gly Pro Cys Ala Leu Cys Gln Glu Tyr Thr Glu Leu Gln Lys Arg Gly
       130                 135                 140
   Phe His Met Ala Lys Gly Ile Ser Ser Pro Pro His Leu Pro Thr Val
   145                 150                 155                 160
<210> 23
   <211> 1177
   <212> DNA
   <213> Zea mays
<220>
   <221> unsure
   <222> (452)...(465)
   <223> n = A, C, G or T
<400> 23
   gcttatcgga gatgtatctg gcagctatgc cctacgaacc gtacggggtg gcggcggcgc 60
   cagtcgtgtc cttccccgtt gccggagcgg ccagggcaca ggcagtggtc gtcgggcctc 120
   ttcgactgct tggacgagtc ccgcgtctcc tgtaagccta gttagcaccc tcgatgttcg 180
   tcagctcgat cttgaacttt tgctaggtcg tatagtaggt agctgagctc accggcaggc 240
   acaggctgcc tgacgcactt gtgcccgtgc gtcacgttcg ggcgggatcg cggcgcgacg 300
   tcgtgcgcga cgggcggggc gctatacgcg ctcatcgcct gcctctcggc gtcgcggtgc 360
   cagtgggtgt attcctgcac gtaccgcgcc gtgatgcgct cgcagttggg cctcccggag 420
   gcgccatgcg ccgactgcct cgtccaccta annnnnnnnn nnnnngcgct ctgccagcag 480
   tacagggagc tcaaggcttg gggcctcgag ccctcaaccc cgccatcggc cgggacttga 540
   acgatgccat gtacccgccg ccggcgcagg ggatgcgacg gcactgatcg gtggatccct 600
   ccatgtatcg ggtccatttc gtttgtttgt tgacgacaga tgcagacaca gtacaaggtg 660
   tccacctaca ccacacgcca ggctggacca ccctccatat atatagggtg agtaaattgg 720
   aagtcatatg ctttcaaata ttatagaaag acctgaccct ttacccccga cctggctgaa 780
   acatgcatac gccaggctgt caactgtgtc actgcccgtg caccaggtca accttcccca 840
   cgatcatcag gcaaacacct ggtccaaaat caacctcccg catgggacgc aaaccataaa 900
   ttgtggttct gtttttacac aaatagagta aaactgtcaa cataaatagt gtaaatagtt 960
   cagagagata gcataaaaac cgcgccacat gctcatgcgc aggtgcatgc atgcagattc 1020
   tatttttatg atagatccaa taattatgac atggcgtgag aattttaatt atatgtaatc 1080
   aaattttaga tgacattttc gtttccattg catgcgcgca aaaaattaca tgacaggaat 1140
   gtgcgagatg aaaggaaatt gattgacata tatggaa                          1177
<210> 24
   <211> 148
   <212> PRT
   <213> Zea mays
<220>
   <221> UNSURE
   <222> (929)...(929)
   <223> Xaa = any amino acid
<400> 24
   Ala Tyr Arg Arg Cys Ile Trp Gln Leu Cys Pro Thr Asn Arg Thr Gly
   1               5                   10                  15
   Trp Arg Arg Arg Gln Ser Cys Pro Ser Pro Leu Pro Glu Arg Pro Gly
               20                  25                  30
   His Arg Gln Trp Ser Ser Gly Leu Phe Asp Cys Leu Asp Glu Ser Arg
           35                  40                  45
   Val Ser Cys Cys Leu Thr His Leu Cys Pro Cys Val Thr Phe Gly Arg
       50                  55                  60
   Asp Arg Gly Ala Thr Ser Cys Ala Thr Gly Gly Ala Leu Tyr Ala Leu
   65                  70                  75                  80
   Ile Ala Cys Leu Ser Ala Ser Arg Cys Gln Trp Val Tyr Ser Cys Thr
                   85                  90                  95
   Tyr Arg Ala Val Met Arg Ser Gln Leu Gly Leu Pro Glu Ala Pro Cys
               100                 105                 110
   Ala Asp Cys Leu Val His Leu Xaa Xaa Xaa Xaa Xaa Ala Leu Cys Gln
           115                 120                 125
   Gln Tyr Arg Glu Leu Lys Ala Trp Gly Leu Glu Pro Ser Thr Pro Pro
       130                 135                 140
   Ser Ala Gly Thr
   145
<210> 25
   <211> 813
   <212> DNA
   <213> Zea mays
<400> 25
   gcacaaggca ataaataaaa agcccaacgc tgggctccgc tagctctcgg tctctctgga 60
   gtctggactc gcctccacgg ctccaccatg tatcccaagg cggcagacga aggtgcgcag 120
   ccgctggcca cgggcatccc cttcagcggc ggcggcggct actaccaggc gggcggcgcg 180
   atggcggcgg cgttcgcggt gcaggcgcag gcgcccgtcg ccgcctggtc caccgggctc 240
   tgcaactgct tcgacgactg ccacaactgc tgcgtgacgt gcgtgtgccc gtgcatcacg 300
   ttcgggcaga ccgcggagat catcgaccgg ggctccacgt cctgcggcac cagcggggcg 360
   ctgtacgcgc tcgtcatgct gctcaccggc tgtcagtgcg tctactcctg cttctaccgc 420
   gccaagatgc gcgcgcagta cggcctccag gtgagcccct gctccgactg ctgcgtgcac 480
   tgctgctgcc agtgctgcgc gctctgccag gagtaccgcg agctcaagaa gcgaggcttc 540
   gacatgagca taggatggca tgcgaacatg gagaggcagg ggcgcgccgc cgccgccgtg 600
   ccgccgcaca tgcatcctgg gatgacccgc tgacgctctg ccgctcgcct cacttctgct 660
   gaggaaatca agtgatttgg tattggtccg ctcccagcag gcagtatcaa ctactgtaac 720
   caatccatga tctgtatgcg gtatcgggct gaactgatac tttatggact tgttgcttca 780
   aaaaaaaaaa aaaaaaaaaa aaaaaaaaaa aaa                              813
<210> 26
   <211> 546
   <212> DNA
   <213> Zea mays
<400> 26
   atgtatccca aggcggcaga cgaaggtgcg cagccgctgg ccacgggcat ccccttcagc 60
   ggcggcggcg gctactacca ggcgggcggc gcgatggcgg cggcgttcgc ggtgcaggcg 120
   caggcgcccg tcgccgcctg gtccaccggg ctctgcaact gcttcgacga ctgccacaac 180
   tgctgcgtga cgtgcgtgtg cccgtgcatc acgttcgggc agaccgcgga gatcatcgac 240
   cggggctcca cgtcctgcgg caccagcggg gcgctgtacg cgctcgtcat gctgctcacc 300
   ggctgtcagt gcgtctactc ctgcttctac cgcgccaaga tgcgcgcgca gtacggcctc 360
   caggtgagcc cctgctccga ctgctgcgtg cactgctgct gccagtgctg cgcgctctgc 420
   caggagtacc gcgagctcaa gaagcgaggc ttcgacatga gcataggatg gcatgcgaac 480
   atggagaggc aggggcgcgc cgccgccgcc gtgccgccgc acatgcatcc tgggatgacc 540
   cgctga                                                            546
<210> 27
   <211> 181
   <212> PRT
   <213> Zea mays
<400> 27
   Met Tyr Pro Lys Ala Ala Asp Glu Gly Ala Gln Pro Leu Ala Thr Gly
   1               5                   10                  15
   Ile Pro Phe Ser Gly Gly Gly Gly Tyr Tyr Gln Ala Gly Gly Ala Met
               20                  25                  30
   Ala Ala Ala Phe Ala Val Gln Ala Gln Ala Pro Val Ala Ala Trp Ser
           35                  40                  45
   Thr Gly Leu Cys Asn Cys Phe Asp Asp Cys His Asn Cys Cys Val Thr
       50                  55                  60
   Cys Val Cys Pro Cys Ile Thr Phe Gly Gln Thr Ala Glu Ile Ile Asp
   65                  70                  75                  80
   Arg Gly Ser Thr Ser Cys Gly Thr Ser Gly Ala Leu Tyr Ala Leu Val
                   85                  90                  95
   Met Leu Leu Thr Gly Cys Gln Cys Val Tyr Ser Cys Phe Tyr Arg Ala
               100                 105                 110
   Lys Met Arg Ala Gln Tyr Gly Leu Gln Val Ser Pro Cys Ser Asp Cys
           115                 120                 125
   Cys Val His Cys Cys Cys Gln Cys Cys Ala Leu Cys Gln Glu Tyr Arg
       130                 135                 140
   Glu Leu Lys Lys Arg Gly Phe Asp Met Ser Ile Gly Trp His Ala Asn
   145                 150                 155                 160
   Met Glu Arg Gln Gly Arg Ala Ala Ala Ala Val Pro Pro His Met His
                   165                 170                 175
   Pro Gly Met Thr Arg
               180
<210> 28
   <211> 997
   <212> DNA
   <213> Zea mays
<400> 28
   catctctttt ttgattgtta cattgccaaa agtatatgga gaatcattta ctttgcttta 60
   aaaatagaca tgcccattag cattaatcat attatcgggt cttgggaatc taataatgct 120
   ctggcataca aaacattatt actgactgga atatccgctt tattttggtc catctggctc 180
   tgtcgaaatg aggtggtgtt taatcataaa ccaataccat caattgtgca ggttattttc 240
   agggttactc actggttcag attctggaga cttctacaga aggaggaaaa gcaccaacaa 300
   attctcgatg cctgtcgagc tttggaggtg gtggcgatgg aagtttttgc aatgcatgga 360
   tggcgctcga atgcaagaat tgaagatggc tagtgttttc tatagtcatg gcattttgtt 420
   ttctttgagt tagtttaatt attcaagcaa ttgctctatg gtccaataat cgttatgtaa 480
   tggctgtacg catttgtatg ctaaagccgg aactcttgtt tccataatca aaaaaaaaat 540
   agaaaagaca atacatgctg ctgcgactgt gcatcggaat cgcgggatgt gcaaatatac 600
   taatgctctc gtgtttagtg cagtaaccaa acccttcctt gatttgatat taaggaactg 660
   catctgcatc ggattcccgt tgaaaagtaa cgcggcatta tttattggta tggtcaccta 720
   aaatactatt ttataatata gaccgtatta tttatataat agattttgaa atagataatg 780
   gagataatta agctgttgaa gatagccttt aactagttag taccgcagta cgtgagtggt 840
   gacttgaggg agacctctcg agtcgagtct tgccgacctg gggcctgctg cgcctggatg 900
   aagagctgtg ctgagagaat cattaaagca gcaccgggtc cttgctttgc gcgttgctgt 960
   tcgcataaac aagagatcct agttctactc ccaagca 997
<210> 29
   <211> 997
   <212> DNA
   <213> Zea mays
<400> 29
   tctccgcgtg gagttttata gcgttgttct tttttagcac cgacatgaca tattacatag 60
   aatgaaatga aagttaaatg gaatttatgg gaagttttct gtggagattt aattcgcatg 120
   aatgacttat taagagttga taaatatgcc aagcagagag tttgatagcc ataaagctca 180
   cttcttctct tttctaggat aattgtcacg tacgtcatcc aaaatattta aaactttttg 240
   agtacaaata aaactccaac aagatgagat tagtcttatc acacgttcca ctgacacttg 300
   gaataatata atgtggatgg acgccttatg tcattagtgg gaagaataga caacaaccga 360
   acaacgtgcc attatattgg cgaaagcttg actagcggat tagtttgtat cggtggtcct 420
   gttagtcttt gggcccgcgg gaaagcgagc cgtgccattt ctacgcgcgc actttatttt 480
   atttattgtt tacttcggtc gtcgatgttg ggcagggata agtcttgctt ccggtccacg 540
   gacgcatgct acgttaccat ctctagctag ctagctaggc ggacacttga cgtcgaaaca 600
   agggcagaaa taaaacaacg tgatcattcc atggcagtgc ggcaggccac atacatgcat 660
   gaggatgagc atcgcgagtc gcggcgctcc acgtaaccct tgtaccacca tccggtccgg 720
   gtgtggccag ccgcgtcgta ctcggcggcc gggagacgac tttgacctgc ttgcacggct 780
   ggcccggggc catcgtcagt tcgtcacaac ctgacgtagt gcttgggcac agcgcactga 840
   aaaaaaaaac attaccgtcg cgccgagata aatacgcgct gcggccggac aggctagcta 900
   gaccggacgc gcacaaggca ataaataaaa agcccaacgc tgggctccgc tagctctcgg 960
   tctctctgga gtctggactc gcctccacgg ctccacc 997
<210> 30
   <211> 1000
   <212> DNA
   <213> Zea mays
<400> 30
   cttcgtctag cgctaggtct tctctttcga ctctgatctt tgccgtcgtc actcggttct 60
   ctagctcttg aagcgtgcac ctacaagtcg cccactctcc agctctctcc actcttcggc 120
   aagggtgaga ctgaggcggg tcctgtgtcg tgcatgttgt gcggtgcgcc ctgctggcca 180
   gcccggctgc ccgacctgag ctagcactac gcagctgctt cactgacttt ggtgcttgca 240
   gcttccttgt gccttgtgtg gcccatgacc gtgtgaccat ccttccagcg tagcctaatc 300
   aacctcatac attcactgat tgtgagcact acgacactat atgccacaat aagcaacctc 360
   aacattcatt catgttgtgc tactccgaac ctattagcct attcatcaca caatacaatt 420
   tcattataca aataagtgaa gcggtgaaag cagaggcgcc tggtacagaa acttttttgc 480
   ctttttagac cacgcaccgg cgaggaagca atcaattaga aaccccacga gcgaagtttg 540
   acataggcaa acaccaaaaa gacatctctg tcactgagaa ctgcaagttc aaagtcatgt 600
   aaaaaaccgg tcatacctta aattttactc gtcctccgca actcccaata tattgtacaa 660
   cgggtaatta cggaacaata actaccttac aatattaatg attgaaaaaa aagaaagcat 720
   aaatgataaa attcgtatgt taagcgtcca ttcattttta ctaacgggtc acactattac 780
   tccaatcatc gtacattaat cggcggccgt cacccatata ttcaacattc gtttcgctgt 840
   tgtgttgggc cttactacat ttgtagacgg catctagggc ctcttttgac ctttctagat 900
   gtcatctata aatacgcata cttggcaagc tgatgcatca actcatcacc cgtagtatca 960
   attatcataa cctccgactc cgacgatctg aactgtgaag 1000
<210> 31
   <211> 442
   <212> DNA
   <213> Zea mays
<400> 31
   gttatatttt gtctaaaatt aatcgtccga attaaaaaac taggtggata tttgtttgag 60
   gatgaagtgg tgcatcatga gctcatttct cataaatttg gtgggattct atttatcata 120
   ttaatacaaa ctaattatga ggtgttgata aatcatctta ttgtattcca taaatcaaat 180
   aaaaagaagt aaggagtgag aagataatag actaggttat ttatcaaacc aaatacttta 240
   taactttagt taaaaaagtt aggtcaagta taagcaccaa acaaaagagc cgaggaggat 300
   gtttaacttg ggcctgatca gggcccggcg tgcttcatgt aagctccggc ccgatcgatg 360
   agaggagtca gacggaggca aggccggggt cggcagcaga ggggagcaga gagatctcat 420
   tctccgatcc gcccaggcca cc 442
<210> 32
   <211> 815
   <212> DNA
   <213> Zea mays
<400> 32
   tgggaattac ttaggagctg tgggagtagc ttgcattgtt gcaaaaaagg actataatat 60
   tgtgtgcgtg tctttggtta tgaataaaac aaaatagata atttgccatg tctcttccta 120
   tctcgtttgc attattttgg ggaaaaaaag gaccgtaata atgcgtgtgt ctttggctgt 180
   atgcatgaat acaaaataga caaaataatc tctccagagt ggaacgctaa gaaataatgg 240
   aagagacagg agggacaact aacgactgtt aggttaggta gtatttgaat ctgaagtata 300
   gtgggatgga gcatatctcg gattggatag agtgacttta aaaaggaaaa ttcagttcca 360
   atcatccagc tctctaaaat ctctcggatg aaaccgctgg catggagcta cttcatcccg 420
   atcaccaaac cctacgttac tcaagtgaca agtggtggga ggccaaacgc tagctgggcc 480
   ccaccctagt gcatccgcca agagcacatg catgcccgtg ccgtgcgcgg tgagccccgc 540
   cgccgagttg gctgcagagc tgcggagtct tggcgaagac cccgcgctgg gcgtcgccgc 600
   tgctgagtcg gtcaacgagg cgtaaccagc gagccagcga cgcgcctttg ggttgggatt 660
   ggacccggtc gtcccatccc agccccgcct atgtaaagag cacccgccgc tgcccagctc 720
   tctcattcct gtcggcaagg tgaacgcgag agccgaccgt ctcagtgtgc gcgcgaagaa 780
   gagaaggagg aggaggagga ggaagaagaa gaaga 815
<210> 33
   <211> 630
   <212> DNA
   <213> Zea mays
<400> 33
   gggtgtgtgt tttggtaaaa tatgaacgaa atgaacctcg ctcaagtctc gaacgttggg 60
   ccctagcccc aacctgtttc cgactgctgt tttaactact gcagttataa ttcctgttcc 120
   gactgctgtt ttaactatta cagttataat ttatagagac aaaaataaaa atactatcag 180
   tccgaagctg atacatatta aagtcaagtc ctagctcttg ggtgcgtgtg tgctgctcaa 240
   caccccaacg cgtggaacgg gaaagccgga aaggcccccg cccgcacacg ccgccgccgc 300
   tgccaattcg gtcaacgtac gataccagcc gcgggccggc gagtccaggc ggtcgcagtc 360
   gcacgtcgcg acgcgcctta gcattggacc gagccgcggc cacttgcttt gcaattgcat 420
   ttgccgggat ccatgccagc agctccgtcc tatatgtgaa gcacccggcc gcaaggctct 480
   ctcgctcatt cacggcaaat taagcgagcg ccggccgagg cagacacaac ccgcagcaac 540
   aacttgcatc ggcacggacg accgattgag agcctcggcg ttcgtacgcc acgccaccaa 600
   gagactctgt gcaacaagtg aagggacgaa 630
<210> 34
   <211> 100
   <212> DNA
   <213> Zea mays
<400> 34
   cttggatcat tgacgctgca ctgctgagcg cgttcaggag taatatatat aggaacgaaa 60
   gttaccgaga aactggaaga tcctaaactg ttgccaggac 100
<210> 35
   <211> 89
   <212> DNA
   <213> Zea mays
<400> 35
   gcttatcgga gatgtatctg gcagctatgc cctacgaacc gtacggggtg gcggcggcgc 60
   cagtcgtgtc cttccccgtt gccggagcg 89
<210> 36
   <211> 17
   <212> DNA
   <213> Oligonucleotide
<400> 36
   gatcggctgg gaggcta 17
<210> 37
   <211> 17
   <212> DNA
   <213> Oligonucleotide
<400> 37
   gatctgtatg cggtatc 17
<210> 38
   <211> 17
   <212> DNA
   <213> Oligonucleotide
<400> 38
   gatccagacg gtacata 17
<210> 39
   <211> 17
   <212> DNA
   <213> Oligonucleotide
<400> 39
   gatctttaac tgatatg 17
<210> 40
   <211> 17
   <212> DNA
   <213> Oligonucleotide
<400> 40
   gatctttcaa caacaaa 17
<210> 41
   <211> 17
   <212> DNA
   <213> Oligonucleotide
<400> 41
   gatcatcgcg tacgtac 17
<210> 42
   <211> 17
   <212> DNA
   <213> Oligonucleotide
<400> 42
   gatctggtcg aggacga 17
<210> 43
   <211> 17
   <212> DNA
   <213> Oligonucleotide
<400> 43
   gatctactcg tgcacgt 17
<210> 44
   <211> 126
   <212> PRT
   <213> Lycopersicon
<220>
   <221> UNSURE
   <222> (305)...(305)
   <223> Xaa = any amino acid
<400> 44
   Ser Cys His Phe Ile Met Ser Met His Asp Ser Ile Pro Gly Cys Leu
    1               5                  10                  15
   Thr Cys Trp Cys Pro Cys Ile Thr Phe Gly Arg Val Pro Glu Ile Val
               20                  25                  30
   Asp Xaa Gly Ala Thr Ser Cys Gly Thr Ala Gly Ala Leu Tyr Pro Val
           35                  40                  45
   Leu Ala Tyr Phe Pro Gly Cys Gln Trp Ile Tyr Ser Cys Thr Tyr Arg
       50                  55                  60
   Ala Lys Met Arg Ala Gln Leu Gly Leu Pro Glu Thr Pro Cys Cys Asp
   65                  70                  75                  80
   Cys Leu Val His Phe Cys Cys Glu Pro Cys Ala Leu Cys Gln Gln Tyr
                   85                  90                  95
   Lys Glu Leu Lys Ala Arg Gly Phe Asp Pro Val Leu Gly Trp Asp Arg
               100                 105                 110
   Asn Ala Thr Met Leu Pro Pro Ser Ala Gln Gly Met Gly Arg
           115                 120                 125
<210> 45
   <211> 163
   <212> PRT
   <213> Lycopersicon
<400> 45
   Met Tyr Gln Thr Val Gly Tyr Asn Pro Gly Pro Met Lys Gln Pro Tyr
    1              5                   10                  15
   Val Pro Pro His Tyr Val Ser Ala Pro Gly Thr Thr Thr Ala Arg Trp
               20                  25                  30
   Ser Thr Gly Leu Cys His Cys Phe Asp Asp Pro Ala Asn Cys Leu Val
           35                  40                  45
   Thr Ser Val Cys Pro Cys Ile Thr Phe Gly Gln Ile Ser Glu Ile Leu
       50                  55                  60
   Asn Lys Gly Thr Thr Ser Cys Gly Ser Arg Gly Ala Leu Tyr Cys Leu
   65                  70                  75                  80
   Leu Gly Leu Thr Gly Leu Pro Ser Leu Tyr Ser Cys Phe Tyr Arg Ser
                   85                  90                  95
   Lys Met Arg Gly Gln Tyr Asp Leu Glu Glu Ala Pro Cys Val Asp Cys
               100                 105                 110
   Leu Val His Val Phe Cys Glu Pro Cys Ala Leu Cys Gln Glu Tyr Arg
           115                 120                 125
   Glu Leu Lys Asn Arg Gly Phe Asp Met Gly Ile Gly Trp Gln Ala Asn
       130                 135                 140
   Met Asp Arg Gln Ser Arg Gly Val Thr Met Pro Pro Tyr His Ala Gly
   145                 150                 155                 160
   Met Thr Arg